# EUROPEAN PATENT SPECIFICATION

(11) **EP 2 508 504 B1**
(45) Date of publication and mention of the grant of the patent: **13.04.2016**
(21) Application number: 10834588.5
(22) Date of filing: 25.11.2010
(51) Int. Cl.: C07C 67/30, C07C 69/75, C08F 20/30, C07B 51/00, C07B 61/00

(54) **PROCESS FOR PREPARATION OF CYCLOALKANEDICARBOXYLIC ACID MONOESTERS**
VERFAHREN ZUR HERSTELLUNG VON CYCLOALKANDICARBONSÄUREMONOESTERN
PROCÉDÉ DE PRÉPARATION DE MONOESTERS D'ACIDES CYCLOALCANEDICARBOXYLIQUES

(30) Priority: 01.12.2009 JP 2009273225; 07.04.2010 JP 2010088394
(43) Date of publication of application: 10.10.2012
(73) Proprietor: Sumitomo Chemical Co., Ltd, Chuo-ku, Tokyo 104-8260 (JP)
(72) Inventor: OKAWA, Haruki, Takatsuki-shi Osaka 569-0077 (JP)
(74) Representative: Vossius & Partner Patentanwälte Rechtsanwälte mbB
(86) International application number: PCT/JP2010/071523
(87) International publication number: WO 2011/068138

(56) References cited:
- EP-A1- 0 083 484
- WO-A1-2009/116657
- GB-A- 817 736
- US-A- 6 028 213
- MARINA EDELSON-AVERBUKH ET AL: "Intramolecular benzyl-benzyl interactions in protonated benzyl diethers in the gas phase. Effects of internal hydrogen bonding", JOURNAL OF THE CHEMICAL SOCIETY, PERKIN TRANSACTIONS 2, vol. 2, no. 6, 1 January 1999 (1999-01-01) , pages 1095-1106, XP055129561, ISSN: 0300-9580, DOI: 10.1039/a901622h

## Description

### Technical Field

The present invention relates to a process for preparation of cycloalkanedicarboxylic acid monoesters.

### Background Art

Recently, a compound containing a cycloalkane structure has drawn attention as a liquid material applicable to an optical film such as a polarizing plate and a retardation film used for a flat-panel display device, and as an intermediate for synthesizing the compound, a cycloalkanedicarboxylic acid monoester is preferably used.

JP 62-289545 A discloses a process comprising reacting an alcohol with cyclohexanedicarboxylic chloride and a process comprising reacting an alcohol with cyclohexanedicarboxylic acid in the presence of a condensation agent such as dicyclohexylcarbodiimide as a process for preparation of a cycloalkanedicarboxylic acid monoester.

WO 2009/116657 A1 discloses a method for producing a cycloalkanedicarboxylic acid monoester, including a first step of protecting any one of two carboxyl groups in a cycloalkanedicarboxylic acid with a group e.g. represented by -CH₂Ph, a second step of protecting the remaining carboxyl group of the cycloalkanedicarboxylic acid e.g. with an ethoxymethyl group, and a third step of deprotecting the carboxyl group protected in the first step to obtain the desired cycloalkanedicarboxylic acid monoester.

### Disclosure of the Invention

The present invention provides:
<1> A process for preparation of cycloalkanedicarboxylic acid monoesters which is characterized by comprising: a first step of protecting two carboxyl groups (-COOH) of a compound represented by formula (1-A) with a group represented by Z to obtain a compound represented by formula (2-A), and a second step of deprotecting either of the groups represented by Z of the compound represented by formula (2-A) obtained in the first step with an acid to obtain a compound represented by formula (3-A). 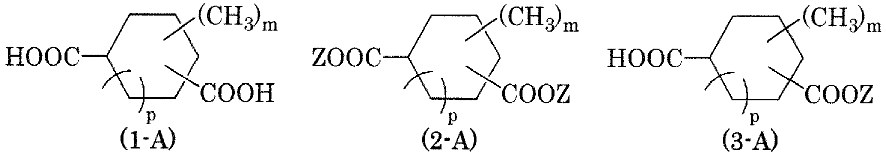 wherein m represents an integer of 0 to 3, p represents 0 or 1, Z represents a methylsulfanylmethyl group (-CH₂-SCH₃), a methoxymethyl group, an ethoxymethyl group, a tert-butoxymethyl group, a (4-pentenyloxy)methyl group, a (2-methoxyethoxy)methyl group, a 1-ethoxyethyl group, a benzyloxymethyl group, a 4-methoxybenzyloxymethyl group, a 2-methoxybenzyloxymethyl group, a 4-nitrobenzyloxymethyl group, a 1-methyl-1-benzyloxy-2-fluoroethyl group, a 1-methyl-1-phenoxyethyl group, a 1-methyl-1-methoxyethyl group, a 1-methyl-1-benzyloxyethyl group, a 2,2,2-trichloroethoxymethyl group, a 1-[2-(trimethylsilyl)ethoxy]ethyl group, a tetrahydropyranyl group, a 3-bromotetrahydropyranyl group, a tetrahydrothiopyranyl group, a tetrahydrofuranyl group, a tetrahydrothiofuranyl group, a tert-butyl group, a trityl group, a benzyl group, a 4-methoxybenzyl group, a 3,4-dimethoxybenzyl group, a 4-nitrobenzyl group, a 1,3-benzodithiolan-2-yl group, a 2,2,2-trichloroethyl group, a 2-phenyl-2-ethanon-1-yl group, a cyclopropylmethyl group, -CH₂-O-Si-R⁵R⁶R⁷ or -SiR⁵R⁶R⁷, and R⁵, R⁶ and R⁷ each independently represent a hydrogen atom, an alkyl group having 1 to 8 carbon atoms, an alkoxy group having 1 to 8 carbon atoms, a phenyl group or a benzyl group;
<2> The process according to <1>, wherein the deprotection in the second step is carried out in a solvent containing an aliphatic hydrocarbon;
<3> The process according to <2>, wherein the content of the aliphatic hydrocarbon in the solvent containing the aliphatic hydrocarbon is a solvent containing in 30% by mass or more and 100% by mass or less;
<4> The process according to <2> or <3>, wherein the aliphatic hydrocarbon is at least one selected from the group consisting of pentane, hexane, cyclohexane, heptane and octane;
<5> The process according to any of <1> to <4>, wherein the acid is a Brønsted acid;
<6> The process according to any of <1> to <5>, wherein the acid is trifluoroacetic acid or trichloroacetic acid;
<7> The process according to any of <1> to <6>, wherein the amount of the acid is 0.1 mole or more and 3 moles or less per 1 mole of the compound represented by formula (2-A);
<8> The process according to any of <1> to <7>, wherein the process further contains a step of recovering the compound represented by formula (1-A) which has been produced as impurities in the second step, and the compound represented by formula (1-A) which has been recovered in the step is reused in the first step;
<9> The process according to any of <1> to <8>, wherein the process further contains a step of recovering the compound represented by formula (2-A) which has been unreacted in the second step, and the compound represented by formula (2-A) which has been recovered in the step is reused in the second step;
<10> The process according to any of <1> to <9>, wherein the first step is a step of reacting the compound represented by formula (1-A) with a compound represented by formula (4-A) or (4-B) to obtain the compound represented by formula (2-A), 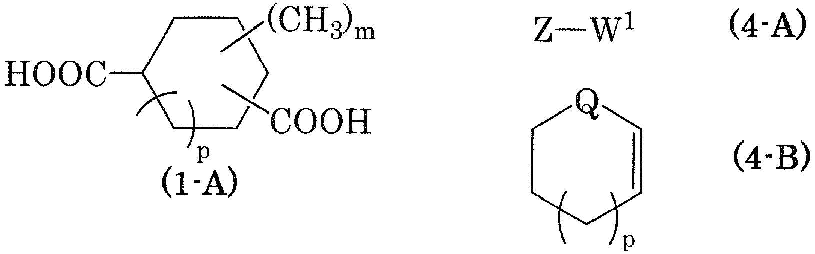 wherein Z, p and m are the same as defined above, W¹ represents a halogen atom, a tosyl group or a mesityl group, Q represents -0- or -S-, and q represents 0 or 1;
<11> The process according to any of <1> to <10>, wherein the group represented by Z is a methylsulfanylmethyl group (-CH₂-SCH₃), a methoxymethyl group, an ethoxymethyl group, a (2-methoxyethoxy)methyl group, a 1-ethoxyethyl group, a tert-butyldimethylsilyl group, a tert-butyldiphenylsilyl group, a triisopropylsilyl group, a tetrahydropyranyl group or a tetrahydrothiopyranyl group;
<12> The process according to any of <1> to <11>, wherein the compound represented by formula (1-A) is a compound represented by formula (1-B), the compound represented by formula (2-A) is a compound represented by formula (2-B), and the compound represented by formula (3-A) is a compound represented by formula (3-B), 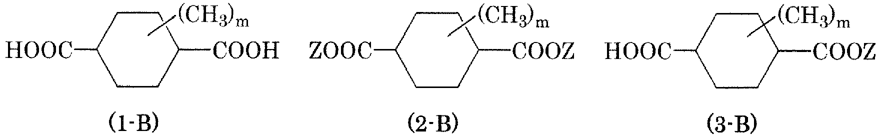 wherein m and Z are the same as defined above;
<13> The process according to any of <1> to <12>, wherein m is 0;
<14> The process according to any of <1> to <13>, wherein the compound represented by formula (3-A) is a trans-1,4-cyclohexanedicarboxylic acid monoester;
<15> A process for producing a polymerizable compound comprising producing the compound represented by formula (3-A) according to the process of any of <1> to <14>, and further comprising a third step of reacting the compound represented by formula (3-A) with a compound represented by formula (5) to obtain a compound represented by formula (6), and a fourth step of deprotecting the group represented by Z of the compound represented by formula (6) which has been obtained in the third step to obtain a polymerizable compound represented by formula (7), 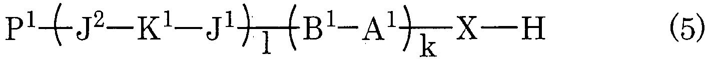 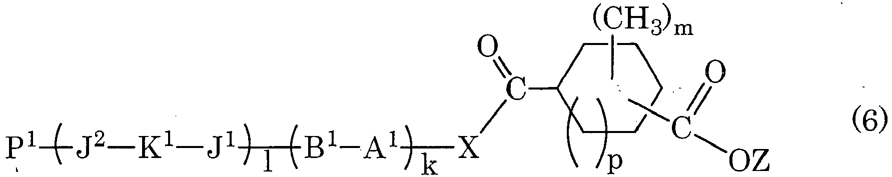 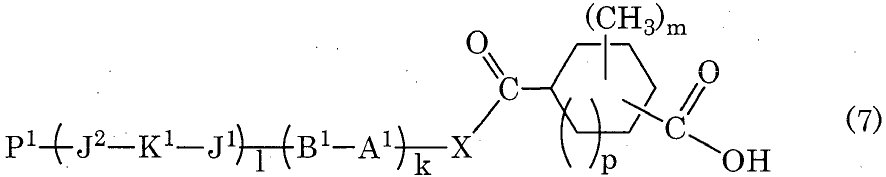 wherein m, Z and p are the same as defined above, X represents -O-, -S- or -N(R¹⁷)-,
   A¹ is independently in each occurrence a divalent alicyclic hydrocarbon group having 3 to 10 carbon atoms or a divalent aromatic hydrocarbon group having 6 to 20 carbon atoms, a hydrogen atom contained in the alicyclic hydrocarbon group or the aromatic hydrocarbon group may be replaced by a halogen atom, an alkyl group having 1 to 4 carbon atoms and optionally substituted with a halogen atom, an alkoxy group having 1 to 4 carbon atoms and optionally substituted with a halogen atom, a cyano group or a nitro group, -CH₂- contained in the alicyclic hydrocarbon group may be replaced by -O-, -S- or -N (R¹⁷)-, -CH (-)-contained in the alicyclic hydrocarbon group may be replaced by -N(-)-,
   B¹ is independently in each occurrence -CR¹⁵R¹⁶-, -CH₂-CH₂-, -O-, -S-, -C(=O)-O-, -O-C(=O)-, -O-C(=O)-O-, -C(=S)-O-, -O-C(=S)-, -O-C(=S)-O-, -C(=O)-N(R¹⁷)-, -N(R¹⁷)-C(=O)-, -OCH₂-, -CH₂O-, -SCH₂-, -CH₂S-, -C(=S)-O-, -O-C(=S)-, -C(=O)-S-, -S-C(=O)-, -(C=S)-S-, -S-C(=S)-, -NH-C(=O)-O-, -O-C(=O)-NH-, -NH-C(=O)-NH-, -N (R¹⁷) -, -S(=O)-, -O-S (=O)₂-O-, -N=N- or a single bond,
   R¹⁵ and R¹⁶ each independently represent a hydrogen atom, a fluorine atom or an alkyl group having 1 to 4 carbon atoms, R¹⁷ represents a hydrogen atom or an alkyl group having 1 to 4 carbon atoms,
   J¹ and J² each independently represent an alkanediyl group having 1 to 18 carbon atoms, and a hydrogen atom contained in the alkanediyl group may be replaced by an alkoxy group having 1 to 5 carbon atoms or a halogen atom,
   K¹ is independently in each occurrence -0-, -C(=O)-O-, -O-C(=O)-, -C(=S)-O-, -O-C(=S)-, -C(=O)-S-, -S-C(=O)-, -(C=S)-S-, -S-C(=S)-, -NH-C(=O)-, -C(=O)-NH-, -NH-C(=O)-O-, -O-C(=O)-NH-, -NH-C(=0)-NH-, -O-C(=O)-O-, -N(R¹⁷)-, -S(=O)-, -O-S(=O)₂-O-, or a single bond,
   k and 1 each independently represent an integer of 1 to 3, and P¹ represents a polymerizable group;
<16> The process according to <15>, wherein P¹ is an acryloyloxy group or a methacryloyloxy group;
<17> The process according to <15> or <16>, wherein the fourth step is a step of deprotecting the group represented by Z of the compound represented by formula (6) which has been obtained in the third step with an acid;
<18> The process according to <17>, wherein the acid is at least one selected from the group consisting of formic acid, acetic acid, propionic acid, hydrochloric acid, hydrobromic acid, hydrogen iodide, nitric acid, sulfuric acid, p-toluenesulfonic acid, pyridinium p-toluenesulfonate, trifluoroacetic acid, trichloroacetic acid and methanesulfonic acid.

### Best Mode for Carrying Out the Present Invention

The process for preparation of a cycloalkanedicarboxylic acid monoester of the present invention comprises a first step of protecting two carboxyl groups (-COOH) of a compound represented by formula (1-A) (hereinafter, simply referred to as compound (1-A)) with a group represented by Z to obtain a compound represented by formula (2-A) (hereinafter, simply referred to as compound (2-A)), and a second step of deprotecting either of the groups represented by Z of compound (2-A) with an acid to obtain a compound represented by formula (3-A) (hereinafter, simply referred to as compound (3-A)).

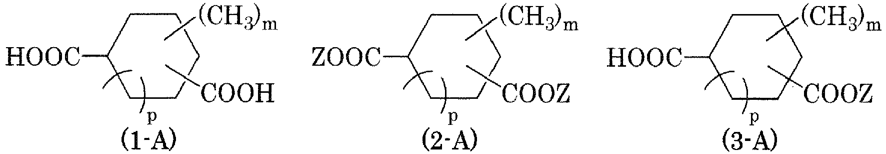

In the formulae (1-A), (2-A) and (3-A), m represents an integer of 0 to 3, and p represents 0 or 1, and m is preferably 0, and p is preferably 1.

Z represents a methylsulfanylmethyl group (-CH₂-SCH₃), a methoxymethyl group, an ethoxymethyl group, a tert-butoxymethyl group, a (4-pentenyloxy)methyl group, a (2-methoxyethoxy)methyl group, a 1-ethoxyethyl group, a benzyloxymethyl group, a 4-methoxybenzyloxymethyl group, a 2-methoxybenzyloxymethyl group, a 4-nitrobenzyloxymethyl group, a 1-methyl-1-benzyloxy-2-fluoroethyl group, a 1-methyl-1-phenoxyethyl group, a 1-methyl-1-methoxyethyl group, a 1-methyl-1-benzyloxyethyl group, a 2,2,2-trichloroethoxymethyl group, a 1-[2-(trimethylsilyi)ethoxy]ethyl group, a tetrahydropyranyl group, a 3-bromotetrahydropyranyl group, a tetrahydrothiopyranyl group, a tetrahydrofuranyl group, a tetrahydrothiofuranyl group, a tert-butyl group, a trityl group, a benzyl group, a 4-methoxybenzyl group, a 3,4-dimethoxybenzyl group, a 4-nitrobenzyl group, a 1,3-benzodithiolan-2-yl group, a 2,2,2-trichloroethyl group, a 2-phenyl-2-ethanon-1-yl group, a cyclopropylmethyl group, -CH₂-O-Si-R⁵R⁶R⁷ or -SiR⁵R⁶R⁷, and R⁵, R⁶ and R⁷ each independently represent a hydrogen atom, an alkyl group having 1 to 8 carbon atoms, an alkoxy group having 1 to 8 carbon atoms, a phenyl group or a benzyl group;

Examples of the alkyl group having 1 to 8 carbon atoms include a methyl group, an ethyl group, an isopropyl group, a tert-butyl group, a hexyl group and an octyl group, and examples of the alkoxy group having 1 to 8 carbon atoms include a methoxy group, an ethoxy group, an isopropoxy group, a tert-butoxy group, a hexyloxy group and an octyloxy group.

Examples of the group represented by -CH₂-O-Si-R⁵R⁶R⁷ include a trimethylsilyloxymethyl group, an isopropyldimethylsilyloxymethyl group, a tert-butyldimethylsilyloxymethyl group, a tert-butyldiphenylsilyloxymethyl group, a tribenzylsilyloxymethyl group, a triisopropylsilyloxymethyl group, and a di-tert-butylmethylsilyloxymethyl group.

Examples of the group represented by -SiR⁵R⁶R⁷ include a trimethylsilyl group, an isopropyldimethylsilyl group, a tert-butyldimethylsilyl group, a tert-butyldiphenylsilyl group, a tribenzylsilyl group, a triisopropylsilyl group, and a di-tert-butylmethylsilyl group.

Z is preferably a methylsulfanylmethyl group, a methoxymethyl group, an ethoxymethyl group, a 2-methoxyethoxymethyl group, a 1-ethoxyethyl group, a tert-butyldimethylsilyl group, a tert-butyldiphenylsilyl group, a triisopropylsilyl group, a tetrahydropyranyl group or a tetrahydrothiopyranyl group. When the group represented by Z is such group, compound (2-A) can be obtained under a mild condition.

Compound (1-A) is preferably a compound represented by formula (1-B). Compound (2-A) is preferably a compound represented by formula (2-B). Compound (3-A) is preferably a compound represented by formula (3-B). 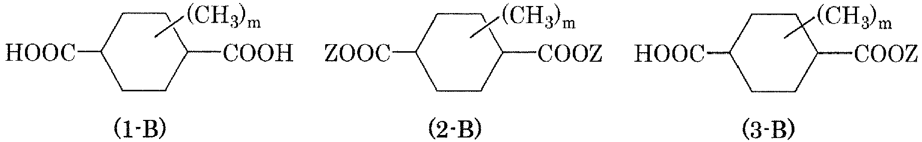 wherein m and Z are the same as defined above.

Compound (1-B) is preferably trans-1,4-cyclohexanedicarboxylic acid, compound (2-B) is preferably a trans-1,4-cyclohexanedicarboxylic acid diester, and compound (3-B) is preferably a trans-1,4-cyclohexanedicarboxylic acid monoester.

When trans-1,4-cyclohexanedicarboxylic acid is used as compound (1-B), a trans-1,4-cyclohexanedicarboxylic acid diester is usually obtained as compound (2-B), and a trans-1,4-cyclohexanedicarboxylic acid monoester is usually obtained as compound (3-B).

The first step will be illustrated below.

The first step is a step of protecting two carboxyl groups of compound (1-A) with the group represented by Z to obtain compound (2-A). 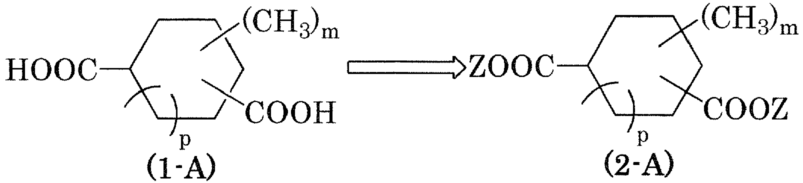 wherein Z, m and p are the same as defined above.

Examples of the specific processes for protecting two carboxyl groups of compound (1-A) with the group represented by Z include the following (Process a), (Process b), (Process c) and (Process d).

(Process a) a process comprising reacting compound (1-A) with a compound represented by formula (4-A) (hereinafter, simply referred to as compound (4-A)) in the presence of a base.

(Process b) a process comprising reacting compound (2-A) with a compound represented by formula (4-B) (hereinafter, simply referred to as compound (4-B)) or a compound represented by formula (4-B') (hereinafter, simply referred to as compound (4-B')).

(Process c) a process comprising reacting compound (2-A) with a compound represented by formula (4-C) (hereinafter, simply referred to as compound (4-C)).

(Process d) a process comprising reacting compound (1-A) with a base to obtain a salt represented by formula (1-A') (hereinafter, simply referred to as salt (1-A')) and reacting salt (1-A') obtained with compound (4-A).

Z-W¹ (4-A)

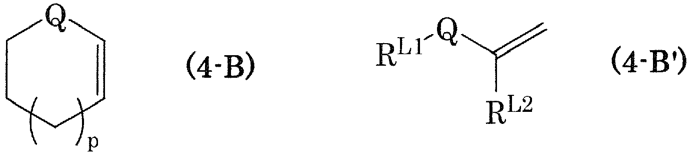

Z-OH (4-C)

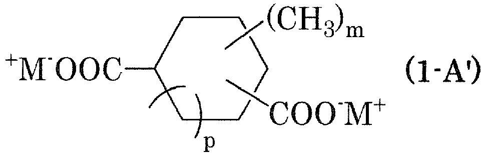

wherein W¹ represents a halogen atom, a tosyl group or a mesityl group, Q represents -0- or -S-, q represents 0 or 1, R^{L1} represents a methyl group, an ethyl group, a phenyl group, a benzyl group or a trimethylsilylethyl group, R^{L2} represents a hydrogen atom, a methyl group or a fluorine atom, M represents an alkali metal atom, and Z, p and m are the same as defined above.

In Process a, the reaction of compound (1-A) and compound (4-A) is usually conducted in an organic solvent. As necessary, a salt generated in the reaction can be removed.

Examples of the base include organic bases such as triethylamine, diisopropylethylamine, pyridine, N-methylmorpholine, dimethylaminopyridine, and dimethylaniline, and inorganic bases such as alkali metal hydroxides such as sodium hydroxide, potassium hydroxide and cesium hydroxide, alkali metal carbonates such as sodium carbonate, potassium carbonate and cesium carbonate, alkali metal hydrogen carbonates such as sodium hydrogen carbonate and potassium hydrogen carbonate, and cesium fluoride. As organic bases, preferred are triethylamine, diisopropylethylamine, pyridine, N-methylmorpholine and dimethylaminopyridine, and more preferred are triethylamine, diisopropylethylamine and pyridine. As inorganic bases, preferred are sodium hydroxide, potassium hydroxide, potassium carbonate and cesium carbonate, and more preferred are potassium hydroxide and potassium carbonate. When an inorganic base is used, a phase transfer catalyst such as crown ethers such as 18-crown-6 and quaternary ammonium salts such as tetrabutylammonium bromide can be used in combination.

The amount of the base to be used is preferably 2 to 5 moles per 1 mole of compound (1-A).

In the formula (4-A), W¹ is preferably a halogen atom, more preferably a chlorine atom, a bromine atom or an iodine atom, and especially preferably a chlorine atom or a bromine atom.

Examples of compound (1-A) include methylsulfanylchloromethane, methylsulfanylbromomethane, methoxychloromethane, methoxybromomethane, chloromethoxyethane, bromomethoxyethane, 2-methoxyethoxymethyl chloride, 2-methoxyethoxymethyl bromide, ethoxyethyl chloride, ethoxyethyl bromide, benzyloxymethyl chloride, benzyloxymethyl bromide, 4-methoxybenzyloxymethyl chloride, 4-nitrobenzyloxymethyl chloride, 2-methoxyphenyloxymethyl chloride, tert-butoxymethyl chloride, tert-butoxymethyl bromide, 4-pentenyloxymethyl chloride, 1-[2-(trimethylsilyl)ethoxy]ethyl chloride, 1-chloromethoxy-2,2,2-trichloroethane, 1-[2-(trimethylsilyl)ethoxy]ethyl chloride, 1-methyl-1-methoxyethyl chloride, 1-methyl-1-benzyloxyethyl chloride, 1-methyl-1-benzyloxy-2-fluoroethyl chloride, 1-methyl-1-phenoxyethyl chloride, tert-butyl chloride, tert-butyl bromide, trityl chloride, trityl bromide, 1,3-benzothiolane-2-chloride, trimethylchlorosilane, trimethylbromosilane, isopropyldimethylchlorosilane, isopropyldimethylbromosilane, tert-butyldimethylchlorosilane, tert-butyldimethylbromosilane, tert-butyldiphenylchlorosilane, tert-butyldiphenylbromosilane, tribenzylchlorosilane, tribenzylbromosilane, triisopropylchlorosilane, triisopropylbromosilane, di-tert-butylmethylchlorosilane, di-tert-butylmethylbromosilane, trimethylsilyloxychloromethane, 2-phenyl-2-ethanon-1-yl chloride, 1,1,1,2-tetrachloroethane and chloromethylcyclopropane. With regard to these, commercially available one can be used.

As the organic solvent, preferred are water-soluble solvents other than alcohol solvents and hydrophobic solvents. Examples of the water-soluble solvents other than alcohol solvents include water-soluble ketone solvents such as acetone, water-soluble ether solvents such as tetrahydrofuran, dioxane and ethylene glycol dimethyl ether, water-soluble amide solvents such as N,N-dimethylformamide, N,N-dimethylacetamide and N-methylpyrrolidone, and water-soluble sulfoxide solvents such as dimethylsulfoxide. Examples of the hydrophobic solvents include aromatic hydrocarbon solvents such as toluene, benzene, xylene and chlorobenzene, hydrophobic ketone solvents such as methyl ethyl ketone, cyclopentanone, cyclohexanone, methyl amyl ketone and methyl isobutyl ketone, aliphatic hydrocarbon solvents such as pentane, hexane and heptane, nitrile solvents such as acetonitrile; ester solvents such as ethyl lactate and ethyl acetate; and halogenated hydrocarbon solvents such as chloroform, dichloromethane, carbon tetrachloride, tetrachloroethane and trichloroethane. Among them, preferred are water-soluble ether solvents, water-soluble amide solvents, water-soluble sulfoxide solvents, aromatic hydrocarbon solvents, hydrophobic ketone solvents and halogenated hydrocarbon solvents, and more preferred are halogenated hydrocarbon solvents. These organic solvents may be used alone, or two or more kinds thereof may be used in combination.

The amount of the organic solvent to be used is preferably 100 to 1500% by mass per total amount of compound (1-A) and compound (4-A), more preferably 100 to 1000% by mass, especially preferably 200 to 600% by mass.

The amount of compound (4-A) to be used is preferably 1.8 to 3 moles per 1 mole of compound (1-A), more preferably 1.97 to 2.7 moles, especially preferably 2 to 2.7 moles.

The reaction temperature is preferably -20 to 140°C, more preferably 0 to 100°C, especially preferably 15 to 45°C.

The reaction time is preferably 1 minute to 72 hours, more preferably 1 to 48 hours, especially preferably 1 to 24 hours.

In Process b, the reaction of compound (1-A) and compound (4-B) or compound (4-B)' is usually carried out in an organic solvent. The reaction is preferably conducted in the presence of an acid catalyst.

Examples of the acid catalyst include p-toluenesulfonic acid, pyridinium p-toluenesulfonate, hydrochloric acid, sulfuric acid and trifluoroacetic acid. The amount of the acid catalyst to be used is preferably 0.005 to 0.2 mole per 1 mole of compound (1-A).

Examples of the organic solvent include ketone solvents such as acetone, methyl ethyl ketone, cyclopentanone, cyclohexanone, methyl amyl ketone and methyl isobutyl ketone; aliphatic hydrocarbon solvents such as pentane, hexane and heptane; aromatic hydrocarbon solvents such as toluene, xylene, benzene and chlorobenzene; nitrile solvents such as acetonitrile; ether solvents such as tetrahydrofuran and dimethoxyethane; ester solvents such as ethyl lactate; and halogenated hydrocarbon solvents such as chloroform and dichloromethane. These organic solvents may be used alone, or two or more kinds thereof may be used in combination. The amount of the organic solvent to be used is preferably 100 to 1200% by mass per total amount of compound (1-A) and compound (4-B) or (4-B'), more preferably 100 to 800% by mass, especially preferably 200 to 600% by mass.

Examples of compound (4-B) include dihydropyran, 3-bromodihydropyran, dihydrothiopyran, dihydrofuran and dihydrothiofuran.

Examples of compound (4-B') include vinyl methyl ether, vinyl ethyl ether, vinyl phenyl ether, vinyl benzyl ether and trimethylsilylethyl vinyl ether.

The amount of compound (4-B) or (4-B') to be used is preferably 1.9 to 5 moles per 1 mole of compound (1-A), more preferably 1.9 to 4 moles, especially preferably 2.2 to 4 moles.

The reaction temperature is preferably 0 to 120°C, more preferably 0 to 60°C, especially preferably 0 to 40°C.

The reaction time is preferably 1 minute to 72 hours, more preferably 1 to 48 hours, especially preferably 1 to 24 hours.

In Process c, the reaction of compound (1-A) and compound (4-C) is usually carried out in an organic solvent. The reaction is preferably conducted in the presence of a condensation agent.

Examples of the condensation agent include carbodiimides such as 1-cyclohexyl-3-(2-morpholinoethyl)carbodiimide metho-p-toluenesulfonate, dicyclohexylcarbodiimide, 1-ethyl-3-(3-dimethylaminopropyl)carbodiimide, 1-ethyl-3-(3-dimethylaminopropyl)carbodiimide hydrochloric acid salt (water-soluble carbodiimide: available as WSC), bis(2,6-diisopropylphenyl)carbodiimide and bis(trimethylsilyl)carbodiimide, 2-methyl-6-nitrobenzoic anhydride, 2,2'-carbonylbis-1H-imidazole, 1,1'-oxalyldimidazole, diphenylphosphoryl azide, 1(4-nitrobenzenesulfonyl)-1H-1,2,4-triazole, 1H-benzotriazol-1-yloxytripyrrolidinophosphpnium hexafluorophosphate, 1H-benzotriazol-1-yloxytris(dimethylamino)phosphonium hexafluorophosphate, N,N,N',N'-tetramethyl-O-(N-succinimidyl)uronium tetrafluoroborate, N-(1,2,2,2-tetrachloroethoxycarbonyloxy)succinimide, N-carbobenzoxysuccinimide, O-(6-chlorobenzotriazol-1-yl)-N,N,N',N'-tetramethyluronium tetrafluoroborate, O-(6-chlorobenzotriazol-1-yl)-N,N,N',N'-tetramethyluronium hexafluorophosphate, 2-bromo-1-ethylpyridinium tetrafluoroborate, 2-chloro-1,3-dimethylimidazolinium chloride, 2-chloro-1,3-dimethylimidazolinium hexafluorophosphate, 2-chloro-1-methylpyridinium iodide, 2-chloro-1-methylpyridinium p-toluenesulfonate, 2-fluoro-1-methylpyridinium p-toluenesulfonate and pentachlorophenyl trichloroacetate.

The amount of the condensation agent to be used is preferably 150 to 250% by mole relative to compound (2-A).

Examples of the organic solvent include the same as those used in Process a. These organic solvents may be used alone, or two or more kinds thereof may be used in combination. The amount of the organic solvent to be used is preferably 100 to 1500% by mass per total amount of compound (1-A) and compound (4-C), more preferably 100 to 800% by mass, especially preferably 100 to 500% by mass.

The amount of compound (4-C) to be used is preferably 1.8 to 3 moles per 1 mole of compound (1-A), more preferably 1.97 to 2.7 moles, especially preferably 2 to 2.7 moles.

The reaction temperature is preferably -20 to 120°C, more preferably 0 to 80°C, especially preferably 0 to 45°C.

The reaction time is preferably 1 minute to 72 hours, more preferably 1 to 48 hours, especially preferably 1 to 24 hours.

In Process d, the reaction of compound (1-A) and the base is usually carried out by mixing the both in a solvent.

As M in formula (1-A'), sodium atom, potassium atom and cesium atom are preferable, and potassium atom is more preferable.

As the solvent used fourthe reaction of compound (1-A) and the base, solvents capable of dissolving the base used such as water, methanol and ethanol are preferable, and methanol and ethanol are preferable.

As the base, inorganic bases are preferable. Examples of the inorganic bases include alkali metal hydroxides such as sodium hydroxide, potassium hydroxide and cesium hydroxide; alkali metal carbonates such as sodium carbonate, potassium carbonate and cesium carbonate; alkali metal hydrogen cabonates such as sodium hydrogen carbonate and potassium hydrogen carbonate; and cesium fluoride. Among them, preferred are sodium hydroxide and potassium hydroxide from the viewpoint of reactivity to the carboxyl group of compound (1-A), and more preferred is potassium hydroxide from the viewpoint of solubility in the solvent. The reaction of compound (1-A) and the base can also be conducted in the presence of a phase transfer catalyst such as crown ethers such as 18-crown-6, and quaternary ammonium salts such as tetrabutylammonium bromide.

The amount of the base to be used is preferably 2 to 3.5 moles per 1 mole of compound (1-A).

The reaction temperature is preferably -20 to 80°C, more preferably -5 to 80°C, especially preferably 0 to 45°C.

The reaction time is preferably 1 minute to 72 hours, more preferably 1 to 48 hours, especially preferably 1 to 24 hours.

After completion of the reaction, salt (1-A') can be isolated by concentrating the reaction mixture obtained. Alternatively, salt (1-A') can also be isolated by mixing a poor solvent for salt (1-A') with the reaction mixture followed by filtrating the mixture obtained. From the viewpoint of easy operation, it is preferred that salt (1-A') is isolated by mixing a poor solvent for salt (1-A') with the reaction mixture followed by filtrating the mixture obtained. Examples of the poor solvent for salt (1-A') include aromatic hydrocarbon solvents such as toluene and xylene; halogenated hydrocarbon solvents such as chloroform and dichloromethane; and ether solvents such as tetrahydrofuran, dioxane and dimethoxyethane, and tetrahydrofuran, dioxane and toluene are preferable.

When salt (1-A') obtained contains water, salt (1-A') obtained may be dried and then salt (1-A') after drying may be reacted with compound (4-A), and salt (1-A') obtained may be reacted with compound (4-A) as it is. It is preferred that salt (1-A') obtained may be reacted with compound (4-A) as it is without drying salt (1-A') obtained.

The reaction of salt (1-A') and compound (4-A) is usually carried out in a solvent. As the solvent, preferred are water-soluble solvents other than alcohol solvents and hydrophobic solvents. Examples of the water-soluble solvents other than alcohol solvents include water-soluble ketone solvents such as acetone; water-soluble ether solvents such as tetrahydrofuran, dioxane and ethylene glycol dimethyl ether; water-soluble amide solvents such as N,N-dimethylformamide, N,N-dimethylacetamide and N-methylpyrrolidone; and water-soluble sulfoxide solvents such as dimethylsulfoxide. Examples of the hydrophobic solvents include aromatic hydrocarbon solvents such as toluene, benzene, xylene and chlorobenzene; hydrophobic ketone solvents such as methyl ethyl ketone, cyclopentanone, cyclohexanone, methyl amyl ketone and methyl isobutyl ketone; aliphatic hydrocarbon solvents such as pentane, hexane and heptane; nitrile solvents such as acetonitrile; ester solvents such as ethyl lactate and ethyl acetate; and halogenated hydrocarbon solvents such as chloroform, dichloromethane, carbon tetrachloride, tetrachloroethane and trichloroethane. Among them, preferred are hydrophobic solvents such as aromatic hydrocarbon solvents, hydrophobic ketone solvents and halogen solvents, and more preferred are toluene, xylene, dichloromethane and chloroform, and especially preferred are toluene and xylene. Even if salt (1-A') containing water is used, compound (2-A) can be obtained in good yield by using these hydrophobic solvents.

These solvents may be used alone, or plural kinds thereof may be used in combination. The amount of the solvent to be used is preferably 100 to 1500% by mass per total amount of salt (1-A') and compound (4-A), more preferably 100 to 1000% by mass, especially preferably 200 to 800% by mass.

The amount of compound (4-A) to be used is preferably 1.8 to 3 moles per 1 mole of salt (1-A'), more preferably 1.97 to 2.7 moles, especially preferably 2 to 2.3 moles.

The reaction temperature is preferably -20 to 140°C, more preferably 0 to 100°C, especially preferably 0 to 45°C.

The reaction time is preferably 1 minute to 72 hours, more preferably 1 to 48 hours, especially preferably 1 to 24 hours.

The reaction of salt (1-A') and compound (4-A) can also be conducted in the presence of a phase transfer catalyst such as crown ethers such as 18-crown-6, and quaternary ammonium salts such as tetrabutylammonium bromide.

When salt (1-A') containing water is used, the reaction of salt (1-A') and compound (4-A) may be conducted in the presence of a dehydrating agent such as anhydrous sodium sulfate, anhydrous magnesium sulfate and molecular sieves in order to inhibit the progress of side reactions caused by water such as the reaction of compound (4-A) and water.

The reaction of salt (1-A') and compound (4-A) may be conducted in the presence of a base. Although an acid is generated by the reaction of compound (4-A) and water when salt (1-A') containing water is used, the acid generated is neutralized with the base by conducting the reaction of salt (1-A') and compound (4-A) in the presence of a base, and therefore, the decomposition of compound (2-A) by the acid can be inhibited and compound (2-A) can be obtained in a good yield. Examples of the base include organic bases such as triethylamine, diisopropylethylamine, pyridine, N-methylmorpholine, dimethylaminopyridine and dimethylaniline; and inorganic bases such as alkali metal hydroxides such as sodium hydroxide, potassium hydroxide and cesium hydroxide, alkali metal carbonates such as sodium carbonate, potassium carbonate and cesium carbonate, alkali metal hydrogen carbonates such as sodium hydrogen carbonate and potassium hydrogen carbonate, and cesium fluoride, and inorganic bases are preferable. As the organic base, preferred are triethylamine, diisopropylethylamine, pyridine, N-methylmorpholine and dimethylaminopyridine, and more preferred are triethylamine, diisopropylethylamine and pyridine. As inorganic base, preferred are potassium carbonate and cesium carbonate, and more preferred is potassium carbonate. When the inorganic base is used, a phase transfer catalyst such as crown ethers such as 18-crown-6, and quaternary ammonium salts such as tetrabutylammonium bromide can also be used in combination. The amount of the base to be used is preferably 0.5 to 2 moles per 1 mole of salt (1-A').

From the viewpoint of mild reaction condition, Process a, Process b and Process d are preferable.

As the group represented by Z, preferred are a methylsulfanylmethyl group, a methoxymethyl group, an ethoxymethyl group, a 2-methoxyethoxymethyl group, a 1-ethoxyethyl group, a tert-butyldimethylsilyl group, a tert-butyldiphenylsilyl group, a triisopropylsilyl group, a tetrahydropyranyl group and a tetrahydrothiopyranyl group, which are groups capable of being introduced by any of Process a, b or d.

Alternatively, preferred are a methylsulfanylmethyl group, a methoxymethyl group, an ethoxymethyl group, a 2-methoxyethoxymethyl group, a tert-butyldimethylsilyl group, a tert-butyldiphenylsilyl group and a triisopropylsilyl group, which are groups capable of being introduced by Process a or d from the viewpoint of stability of compound (2-A).

Examples of preferable compound (2-A) include compound s represented by the following formulae (2-A-1) to (2-A-38), and compounds represented by the formulae (2-A-1) to (2-A-5) are preferable.

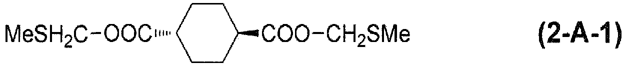

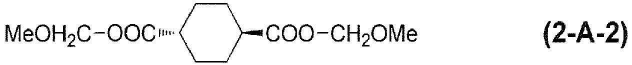

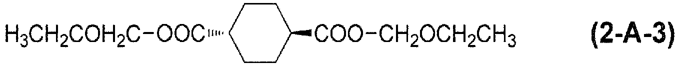

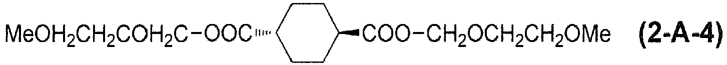

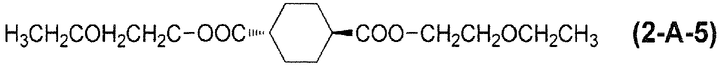

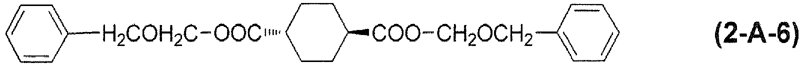

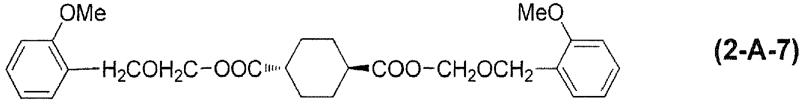

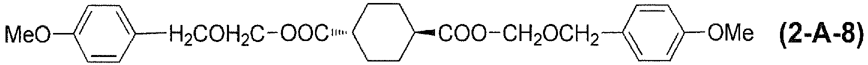

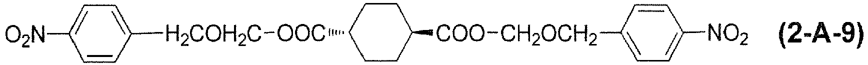

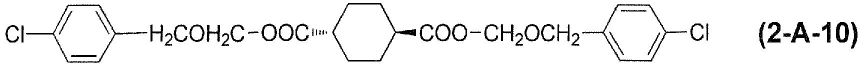

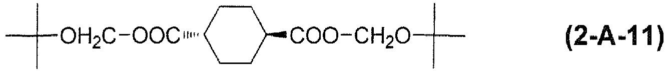

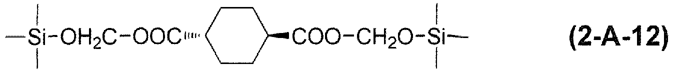

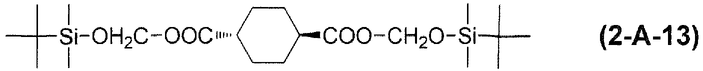

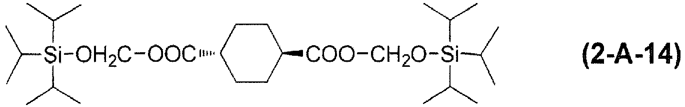

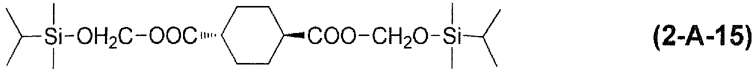

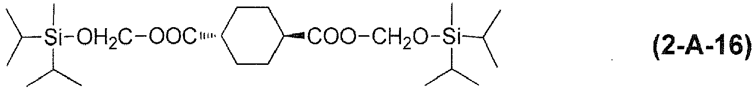

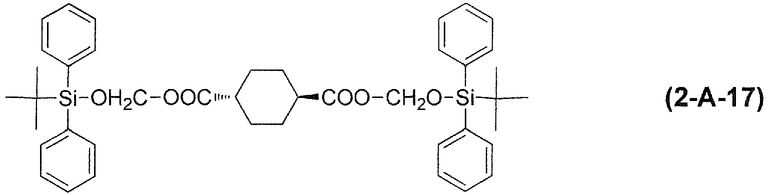

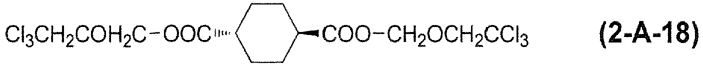

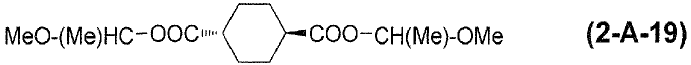

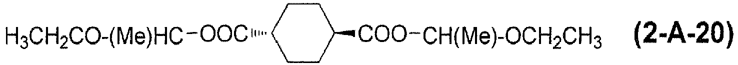

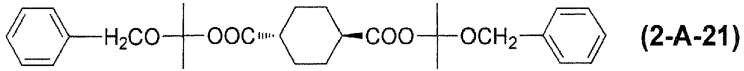

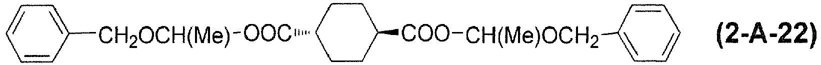

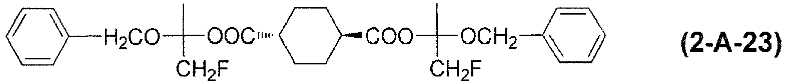

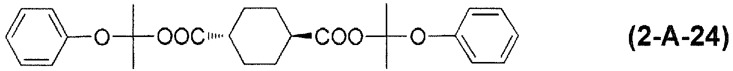

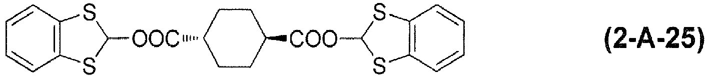

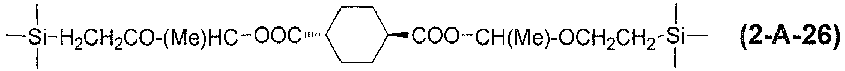

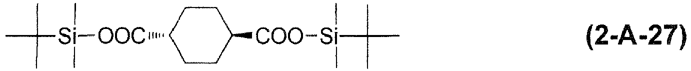

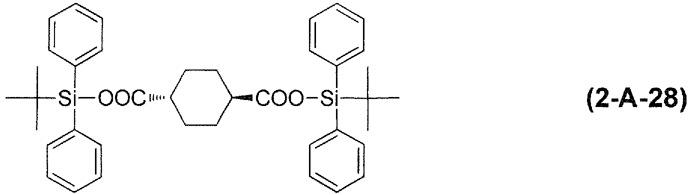

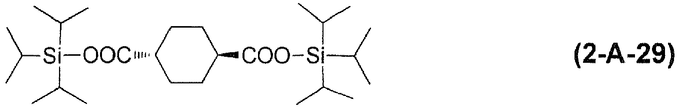

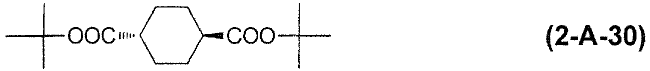

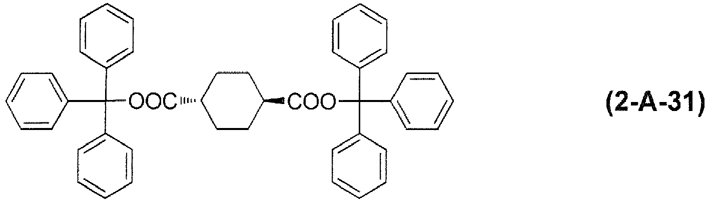

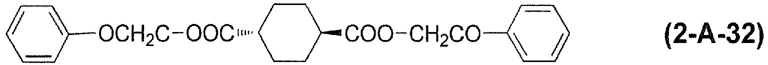

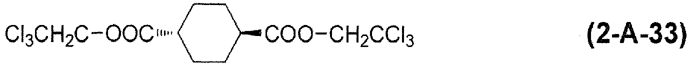

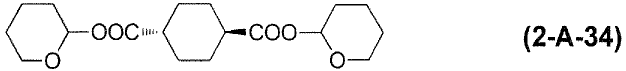

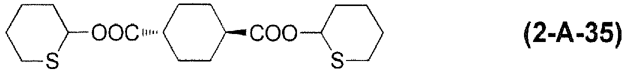

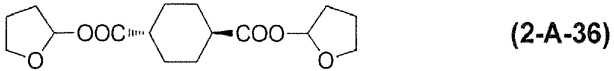

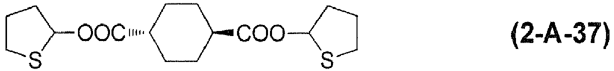

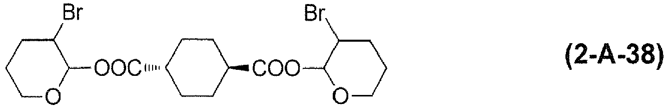

Next, the second step will be illustrated.

The second step is a step of deprotecting either of the groups represented by Z of compound (2-A) obtained in the first step with an acid to obtain compound (3-A). 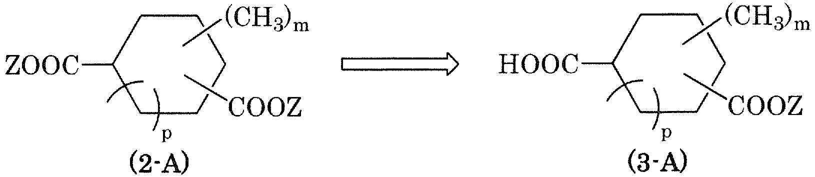 wherein Z, m and p are the same as defined above.

Examples of the acid used for deprotecting include Brsansted acids and Lewis acids.

Examples of the Bronsted acids include inorganic Brønsted acids such as hydrochloric acid, hydrobromic acid, hydroiodic acid, nitric acid and sulfuric acid, and organic Brønsted acids such as formic acid, acetic acid, propionic acid, p-toluenesulfonic acid, pyridinium p-toluenesulfonate, trifluoroacetic acid, trichloroacetic acid, methanesulfonic acid and trifluoromethanesulfonic acid.

Examples of the Lewis acids include phosphorus pentafluoride, boron trifluoride, boron tribromide, aluminum chloride, titanium chloride, tin chloride, antimony chloride, iron trichloride, zinc bromide and boron trifluoride diethyl ether complex. From the viewpoint of easy handling, aluminum chloride, titanium chloride, tin chloride and boron trifluoride diethyl ether complex are preferable, and from the viewpoint of inexpensiveness, aluminum chloride and boron trifluoride diethyl ether complex are more preferable.

As the acid, preferred are Brønsted acids, and more preferred are organic Brønsted acids from the viewpoint of solubility in an organic solvent and enablement of the deprotection reaction in a homogeneouos system in which an organic solvent is used. Among them, more preferred are trifluoroacetic acid, trichloroacetic acid, methanesulfonic acid and trifluoromethanesulfonic acid from the viewpoint of high solubility in an organic solvent and having high acidity, and especially preferred are trifluoroacetic acid, trichloroacetic acid and methanesulfonic acid. From the viewpoint of easy handling, inexpensiveness and easy removal from the reaction mixture, especially preferred are trifluoroacetic acid, trichloroacetic acid and methanesulfonic acid.

The deprotection reaction of compound (2-A) can be carried out, for example, according to the method described in "Protective Groups in Organic Synthesis-THIRD EDITION" (author GreeneWuts, WILEY-INTERSCIENCE).

The amount of the acid to be used is preferably 0.1 mole or more and 3 moles or less per 1 mole of compound (2-A), more preferably 0.1 mole or more and 2.0 moles or less, especially preferably 0.1 mole or more and 1.8 moles or less.

In order to accelerate the deprotection reaction of compound (2-A), additives such as thioanisole, triethylsilane and anisole may be used in combination.

The deprotection reaction of compound (2-A) is usually conducted in a solvent, and as the solvent, a solvent containing an aliphatic hydrocarbon is preferable. Herein, "aliphatic hydrocarbon" means a linear, branched chain and cyclic aliphatic hydrocarbon. Examples of the aliphatic hydrocarbon include pentane, hexane, heptane, octane, isooctane, cyclopentane, cyclohexane, cycloheptane and methylcyclohexane, and saturated aliphatic hydrocarbons are preferable, pentane hexane, heptane, octane and cyclohexane are more preferable, hexane, heptanem octane and cyclohexane are especially preferable. From the viewpoint of easy handling, preferred are heptane, octane and cyclohexane, and from the viewpoint of inexpensiveness, preferred is heptane.

Form the viewpoint of tendency that the solubility of compound (3-A) in the reaction mixture becomes lower and easiness of its purification, the solvent preferably contains an aliphatic hydrocarbon.

Examples of the solvents other than aliphatic hydrocarbons include ketone solvents such as acetone, methyl ethyl ketone, cyclopentanone, cyclohexanone, methyl amyl ketone and methyl isobutyl ketone; aromatic hydrocarbon solvents such as benzene, toluene, xylene and chlorobenzene; ether solvents such as tetrahydrofuran, dimethoxyethane and dioxane; ester solvents such as ethyl lactate; and halogenated hydrocarbon solvents such as chloroform. These solvents may be used alone, or two or more kinds thereof may be used in combination.

The solvents other than aliphatic hydrocarbon solvents are preferably mixed with the aliphatic hydrocarbon solvents to be used. As the solvents other than aliphatic hydrocarbon solvents, aromatic hydrocarbon solvents such as benzene, toluene, xylene and chlorobenzene are preferable. The solvent wherein the content of the aliphatic hydrocarbon solvents is 30% by mass or more and 100% by mass or less is preferably used, and the solvent wherein the content of the aliphatic hydrocarbon solvents is 50% by mass or more and 100% by mass or less is more preferably used, and the solvent wherein the content of the aliphatic hydrocarbon solvents is 75% by mass or more and 100% by mass or less is especially preferably used.

The amount of the solvent to be used is preferably 100 to 1200% by mass relative to compound (2-A), more preferably 100 to 1000% by mass, especially preferably 200 to 800% by mass.

The reaction temperature is preferably -80 to 160°C, more preferably -15 to 100°C, especially preferably 0 to 60°C.

The reaction time is preferably 1 minute to 72 hours, more preferably 1 to 48 hours, especially preferably 3 to 48 hours.

Examples of preferable compound (3-A) include the compounds represented by the following formulae (3-A-1) to

(3-A-38).

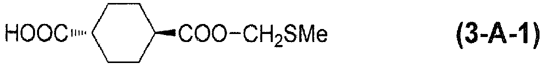

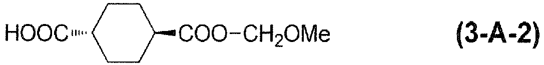

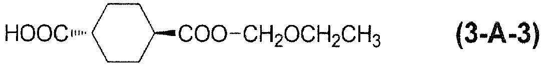

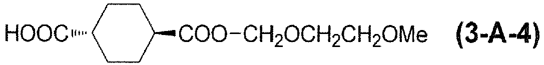

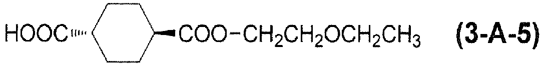

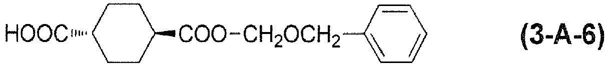

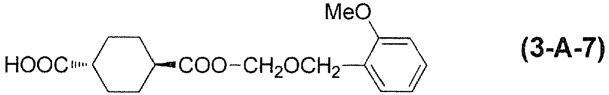

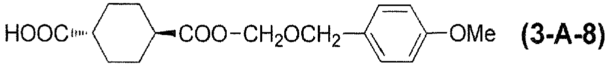

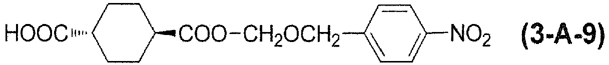

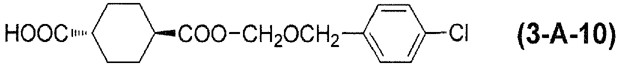

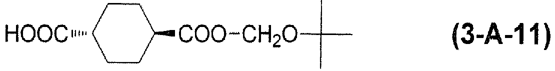

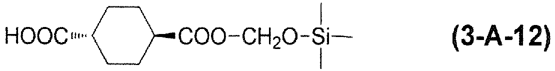

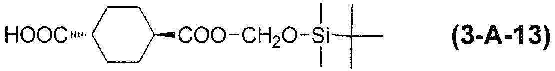

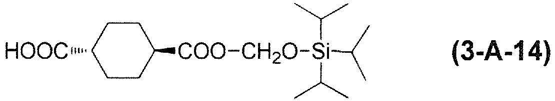

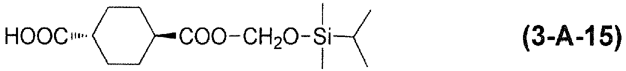

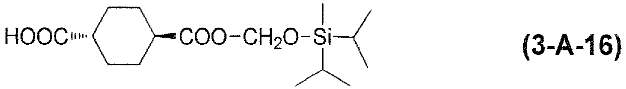

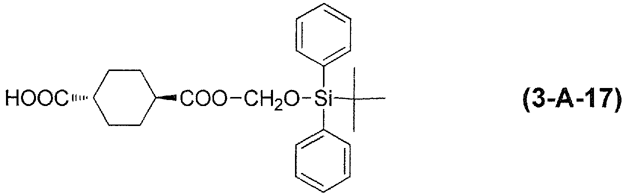

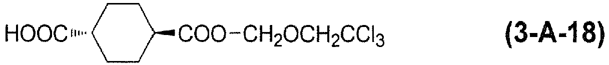

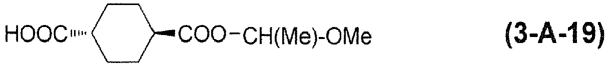

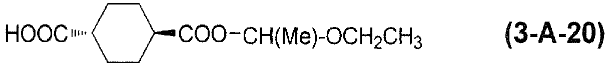

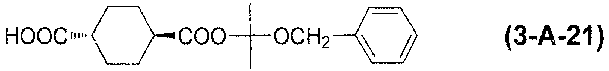

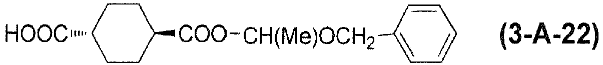

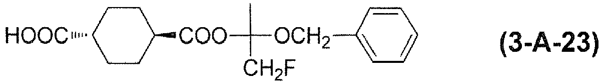

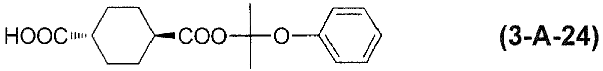

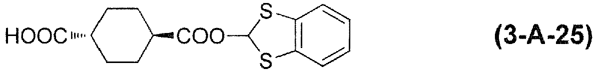

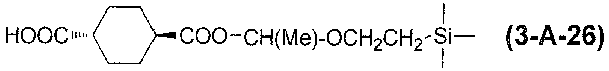

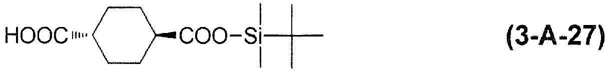

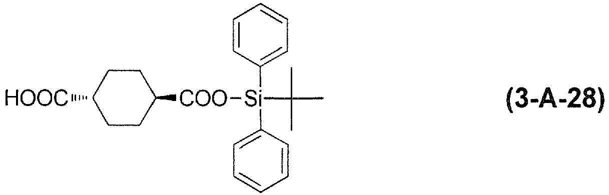

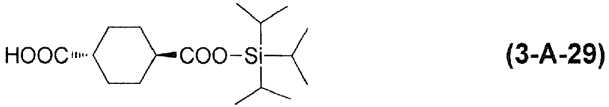

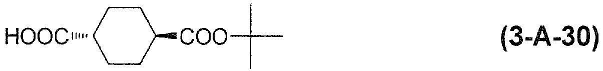

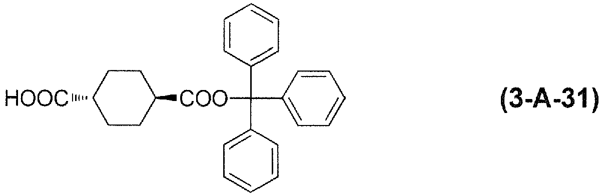

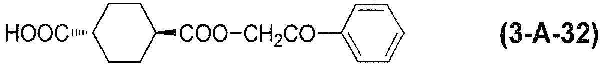

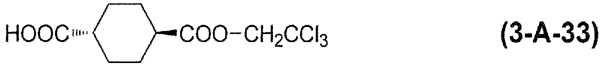

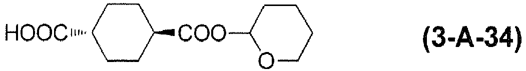

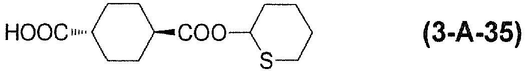

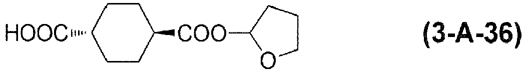

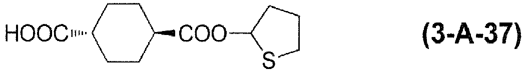

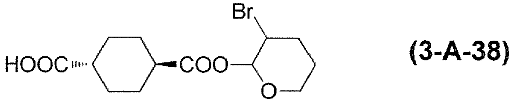

As described below, compound (3-A) is useful as an intermediate for synthesizing a polymerizable compound.

The reaction mixture obtained by reacting compound (2-A) with an acid usually contains compound (1-A) wherein two groups represented by Z of compound (1-A) are deprotected, the desired compound (3-A), and the unreacted compound (2-A). The residue containing compound (3-A) is obtained, for example, by concentrating the reaction mixture obtained. The residue is subjected to methods such as purification with silica gel chromatography, reprecipitation purification using an aliphatic hydrocarbon solvent such as hexane and heptane, washing with water-soluble organic solvents such as methanol, N,N-dimethylformamide, dimethylsulfoxide, N,N-dimethylacetamide, tetrahydrofuran, acetone and N-methylpyrrolidone, or a mixed solvent of the water-soluble organic solvents and water, and extraction with halogenated hydrocarbon solvents such as chloroform, and aromatic hydrocarbon solvents such as toluene, thereby being able to obtain the purified compound (3-A). Two or more kinds of the above-mentioned methods may be combined.

Compound (1-A) contained in the reaction mixture can be recovered and then, compound (1-A) recovered can be reused in the first step. Alternatively, compound (2-A) contained in the reaction mixture can be recovered and then, compound (2-A) recovered can be reused in the second step.

Examples of the process for recovering compound (2-A) contained in the reaction mixture include a process comprising cooling the reaction mixture obtained, separating solid matters containing compound (3-A) and compound (1-A) precipitated by filtration, and concentrating the filtrate obtained. When a solvent having high solubility of compound (3-A) is used in the second step, compound (2-A) can be recovered by replacing the solvent in the reaction mixture to an aliphatic hydrocarbon solvent such as hexane and heptane by concentration or the like, and then, separating solid matters containing compound (3-A) and compound (1-A) precipitated by filtration, and concentrating the filtrate obtained.

Examples of the process for recovering compound (1-A) contained in the reaction mixture include a process comprising mixing the reaction mixture with a non-polar organic solvent to precipitate compound (1-A) as solid, and then, isolating compound (1-A) precipitated by filtration or the like. Examples of the non-polar organic solvent include halogenated hydrocarbon solvents such as chloroform and aromatic hydrocarbon solvents such as toluene. The solubility of compound (1-A) in these non-polar organic solvent is low, and the dissolving of compound (1-A) in the filtrate can be inhibited, and compound (1-A) can be recovered in a high recovery rate.

When compound (1-A) is filtrated, filtrating aids such as Celite and silica gel can be used in combination. Although a mixture of the filtrating aids and compound (1-A) is obtained when the filtrating aids is used, compound (1-A) can be obtained by extracting compound (1-A) with a polar solvent such as acetone from the mixture obtained, and then, concentrating the extract obtained.

Compound (1-A) may be recovered after recovering compound (2-A) from the reaction mixture, and compound (2-A) may be recovered after recovering compound (1-A) from the reaction mixture.

Examples of the process for recovering compound (1-A) and obtaining compound (3-A) after recovering compound (2-A) containing the reaction mixture include the following processes.

When the solubility of compound (3-A) in the reaction mixture is high, a filtrate containing solid matters containing compound (1-A) and compound (3-A), and compound (2-A) can be obtained by replacing the solvent in the reaction mixture to an aliphatic hydrocarbon solvent such as heptane and hexane, and then, filtrating the mixture obtained. Compound (2-A) can be renovated by concentrating the filtrate containing compound (2-A). Compound (1-A) can be isolated as solid by mixing the obtained solid matters containing compound (1-A) and compound (3-A) with halogenated hydrocarbon solvents such as chloroform or aromatic hydrocarbon solvents such as toluene, followed by filtrating the mixture obtained, and compound (3-A) can be recovered by concentrating the filtrate obtained.

Examples of the process for recovering compound (2-A) and obtaining compound (3-A) after recovering compound (1-A) containing the reaction mixture include the following processes.

The reaction mixture is concentrated, and the concentrated residue obtained is mixed with a halogenated hydrocarbon solvent such as chloroform or an aromatic hydrocarbon solvent such as toluene. Solid compound (1-A) can be recovered by filtrating the mixture obtained. The filtrate obtained by filtration is concentrated and the concentrated residue obtained is mixed with an aliphatic hydrocarbon solvent such as heptane or hexane. Solid compound (3-A) can be isolated by filtrating the mixture obtained, and compound (2-A) can be recovered b concentrating the filtrate obtained. Aliphatic hydrocarbon solvents has not only tendency of low solubility of compound (3-A) but also tendency of high solubility of compound (2-A), and therefore, it is preferable solvent for separating compound (2-A) and compound (3-A).

Next, the process for producing a poymerizable compound of the present invention will be illustrated.

The process for producing a polymerizable compound of the present invention comprises a third step of reacting compound (3-A) which has been produced according to the process comprising the above-mentioned first and second steps with a compound represented by formula (5) (hereinafter, simply referred to as compound (5)) to obtain a compound represented by formula (6) (hereinafter, simply referred to as compound (6)), and a fourth step of deprotecting the group represented by Z of compound (6) which has been obtained in the third step to obtain a polymerizable compound represented by formula (7) (hereinafter, simply referred to as polymerizable compound (7)),

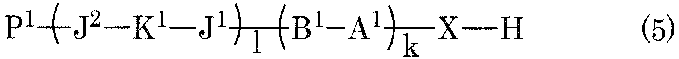

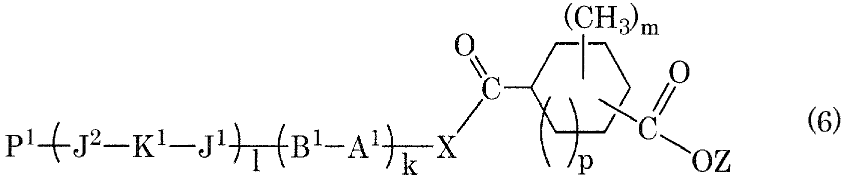

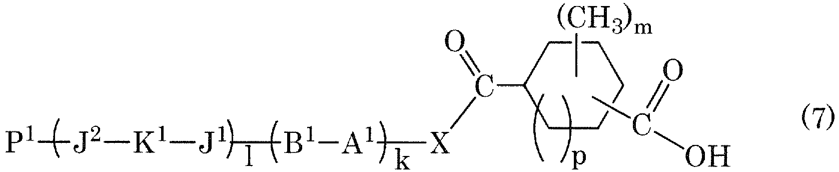

In the formulae (5), (6) and (7), m, Z and p are the same as defined above, k and 1 each independently represent an integer of 1 to 3, k is preferably 1 or 2, more preferably 1, and l is preferably 1 or 2, more preferably 1.

P¹ represents a polymerizable group.

X represents -0-, -S- or -N(R¹⁷)-, and is preferably -0-.

A¹ is independently in each occurrence a divalent alicyclic hydrocarbon group having 3 to 10 carbon atoms or a divalent aromatic hydrocarbon group having 6 to 20 carbon atoms, a hydrogen atom contained in the alicyclic hydrocarbon group or the aromatic hydrocarbon group may be replaced by a halogen atom, an alkyl group having 1 to 4 carbon atoms and optionally substituted with a halogen atom, an alkoxy group having 1 to 4 carbon atoms and optionally substituted with a halogen atom, a cyano group or a nitro group, -CH₂- contained in the alicyclic hydrocarbon group may be replaced by -O-, -S- or -N (R¹⁷)-, -CH(-)-contained in the alicyclic hydrocarbon group may be replaced by -N(-)-, B¹ is independently in each occurrence -CR¹⁵R¹⁶-, CH₂-CH₂-, -O-, -S-, -C(=O)-O-, -O-C(=O)-, -O-C(=O)-O-, -C(=S)-O-, -O-C(=S)-, -O-C(=S)-O-, -C(=O)-N(R¹⁷) -, -N(R¹⁷)-C(=O)-, -OCH₂, -CH₂O-, -SCH₂-, -CH₂S-, -C(=S)-O-, -O-C(=S)-, -C(=O)-S-, -S-C(=O)-, -(C=S)-S-, -S-C(=S)-, -NH-C(=O)-O-, -O-C(=O)-NH-, -NH-C(=O)-NH-, -N(R¹⁷)-, -S(=O)-, -O-S(=O)₂-O-, -N=N- or a single bond.

R¹⁵ and R¹⁶ each independently represent a hydrogen atom, a fluorine atom or an alkyl group having 1 to 4 carbon atoms, R¹⁷ represents a hydrogen atom or an alkyl group having 1 to 4 carbon atoms, J¹ and J² each independently represent an alkanediyl group having 1 to 18 carbon atoms, and a hydrogen atom contained in the alkanediyl group may be replaced by an alkoxy group having 1 to 5 carbon atoms or a halogen atom.

K¹ is independently in each occurrence -0-, -C(=O)-O-, -O-C(=O)-, -C(=S)-O-, -O-C(=S)-, -C(=O)-S-, -S-C(=O)-, -(C=S)-S-, -S-C(=S)-, -NH-C(=O)-, -C(=O)-NH-, -NH-C(=O)-O-, -O-C(=O)-NH-, -NH-C(=O)-NH-, -O-C(=O)-O-, -N(R¹⁷)-, -S(=O)-, -O-S(=O)₂-O-, or a single bond.

Examples of the divalent alicyclic hydrocarbon group having 3 to 10 carbon atoms include the groups represented by formulae (g-1) to (g-4). Examples of the divalent alicyclic hydrocarbon group wherein -CH₂- contained in the alicyclic hydrocarbon group is replaced by -O-, -S- or -N (R¹⁷)- include the groups represented by formulae (g-5) to (g-8). Examples of the divalent alicyclic hydrocarbon group wherein -CH(-)- contained in the alicyclic hydrocarbon group is replaced by -N (-) - include the groups represented by formulae (g-9) to (g-10). A five- or six-membered alicyclic hydrocarbon group is preferable.

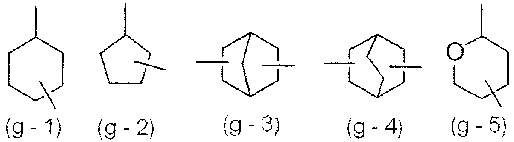

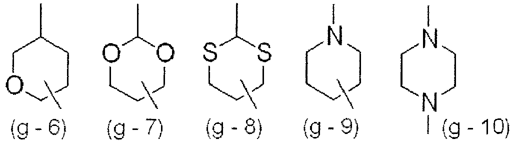

A hydrogen atom contained in the divalent alicyclic hydrocarbon group may be replaced by a halogen atom such as a fluorine atom, a chlorine atom, a bromine atom and an iodine atom; an alkyl group having 1 to 4 carbon atoms such as a methyl group, an ethyl group, an isopropyl group and a tert-butyl group; an alkoxy group having 1 to 4 carbon atoms such as a methoxy group and an ethoxy group; a fluoroalkyl group having 1 to 4 carbon atoms such as a trifluoromethyl group; a fluoroalkoxy group having 1 to 4 carbon atoms such as a trifluoromethoxy group; a cyano group; or a nitro group.

It is preferred that the divalent alicyclic hydrocarbon group is the group represented by formula (g-1), it is more preferred that it is a 1,4-cyclohexanediyl group, and it is especially preferred that it is a trans-1,4-cyclohexanediyl group.

Examples of the divalent aromatic hydrocarbon group having 6 to 20 carbon atoms include the groups represented by formulae (a-1) to (a-8).

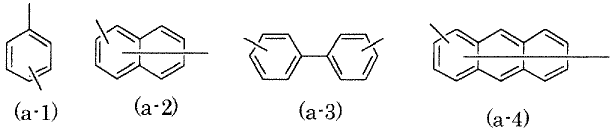

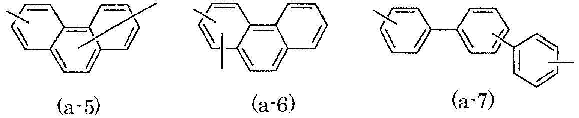

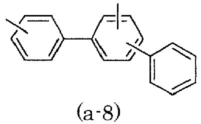

A hydrogen atom contained in the divalent aromatic hydrocarbon group may be replaced by a halogen atom such as a fluorine atom, a chlorine atom, a bromine atom and an iodine atom; an alkyl group having 1 to 4 carbon atoms such as a methyl group, an ethyl group, an isopropyl group and a tert-butyl group; an alkoxy group having 1 to 4 carbon atoms such as a methoxy group and an ethoxy group; a trifluoromethyl group; a trifluoromethyloxy group; a cyano group; or a nitro group.

It is preferred that the divalent aromatic hydrocarbon group is a 1,4-phenylene group.

B¹ is preferably-CH₂-CH₂-, -C(=O)-O-, -O-C(=O)-, -C(=O)-NH-, -NH-C(=O)-, -O-CH₂-, -CH₂-O- or a single bond, and -C(=O)-O- or -O-C(=O)- is more preferable.

Examples of the alkyl group having 1 to 4 carbon atoms in R¹⁵, R¹⁶ and R¹⁷ include those as same as the above.

Examples of the alkanediyl group having 1 to 18 carbon atoms represented by J¹ and J² include a methylene group, an ethanediyl group, a propanediyl group, a butanediyl group, a pentanediyl group, a hexanediyl group, an octanediyl group and a decanediyl group. A hydrogen atom contained in the alkanediyl group may be replaced by an alkoxy group having 1 to 5 carbon atoms such as a methoxy group and an ethoxy group, or a halogen atom suc has a fluorine atom, a chlorine atom, a bromine atom and an iodine atom.

It is preferred that J¹ and J² each independently an alkanediyl group having 1 to 8 carbon atoms.

K¹ is preferably -0-, -C(=O)-O-, -O-C(=O)- or a single bond.

P¹ is a polymerizable group. The polymerizable group is a group containing a group capable of being involved in the polymerization reaction. Examples of the group capable of being involved in the polymerization reaction include a vinyl group, a p-(2-phenylethenyl)phenyl group, an acryloyl group, an acryloyloxy group, a methacryloyl group, a methacryloyloxy group, a carboxyl group, a methylcarbonyl group, a hydroxyl group, a carbamoyl group, an alkylamino group having 1 to 4 carbon atoms, an amino group, a formyl group, -N=C=O, -N=C=S, an oxiranyl group and an oxetanyl group. Examples of the polymerizable group include the group capable of being involved in the polymerization reaction and a group formed by combining the group capable of being involved in the polymerization reaction with a group represented by B¹ or with a group represented by K¹. Among them, preferred are a radical polymerizable group and a cationic polymerizable group from the viewpoint of suitability for photopolymerization, preferred are an acryloyl group, an acryloyloxy group, a methacryloyl group, and a methacryloyloxy group, from the viewpoint of easy handling and easy production, and more preferred are an acryloyl group and an acryloyloxy group from the viewpoint of high polymerizing ability.

The third step is a step of reacting compound (3-A) which has been produced according to the process comprising the above-mentioned first and second steps with compound (5) to obtain compound (6). 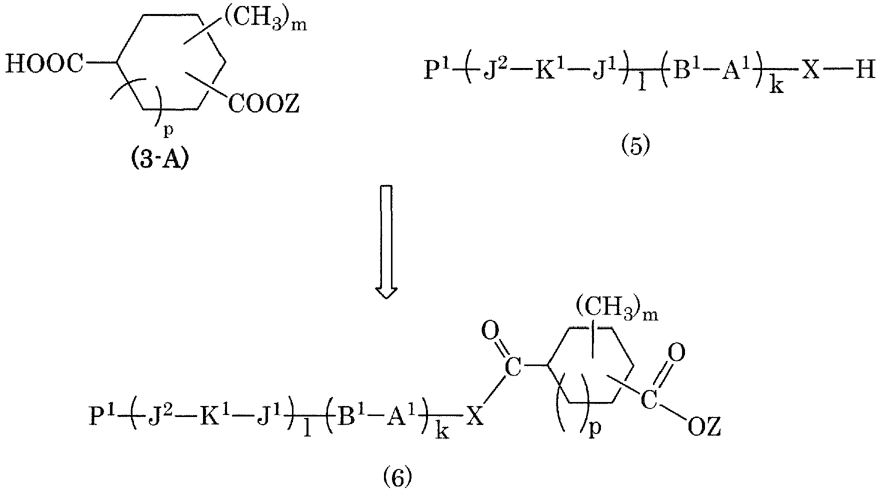 wherein Z, m, p, X, A¹, B¹, J¹, J², K¹, k and l are the same as defined above.

The reaction of compound (3-A) and compound (5) is preferably carried out in the presence of a condensation agent.

Examples of the condensation agent include carbodiimides such as 1-cyclohexyl-3-(2-morpholinoethyl)carbodiimide metho-p-toluenesulfonate, dicyclohexylcarbodiimide, 1-ethyl-3-(3-dimethylaminopropyl)carbodiimide, 1-ethyl-3-(3-dimethylaminopropyl)carbodiimide hydrochloric acid salt (water-soluble carbodiimide: available as WSC), bis(2,6-diisopropylphenyl)carbodiimide and bis(trimethylsilyl)carbodiimide, 2-methyl-6-nitrobenzoic anhydride, 2,2'-carbonylbis-1H-imidazole, 1,1'-oxalyldimidazole, diphenylphosphoryl azide, 1(4-nitrobenzenesulfonyl)-1H-1,2,4-triazole, 1H-benzotriazol-1-yloxytripyrrolidinophosphpnium hexafluorophosphate, 1H-benzotriazol-1-yloxytris(dimethylamino)phosphonium hexafluorophosphate, N,N,N',N'-tetramethyl-O-(N-succinimidyl)uronium tetrafluoroborate, N-(l,2,2,2-tetrachloroethoxycarbonyloxy)succinimide, N-carbobenzoxysuccinimide, O-(6-chlorobenzotriazol-1-yl)-N,N,N',N'-tetramethyluronium tetrafluoroborate, O-(6-chlorobenzotriazol-1-yl)-N,N,N',N'-tetramethyluronium hexafluorophosphate, 2-bromo-1-ethylpyridinium tetrafluoroborate, 2-chloro-1,3-dimethylimidazolinium chloride, 2-chloro-1,3-dimethylimidazolinium hexafluorophosphate, 2-chloro-1-methylpyridinium iodide, 2-chloro-1-methylpyridinium p-toluenesulfonate, 2-fluoro-1-methylpyridinium p-toluenesulfonate and pentachlorophenyl trichloroacetate. Among them, preferred are 1-cyclohexyl-3-(2-morpholinoethyl)carbodiimide metho-p-toluenesulfonate, dicyclohexylcarbodiimide, 1-ethyl-3-(3-dimethylaminopropyl)carbodiimide, 1-ethyl-3-(3-dimethylaminopropyl)carbodiimide hydrochloric acid salt (water-soluble carbodiimide: available as WSC), bis(2,6-diisopropylphenyl)carbodiimide, 2,2'-carbonylbis-1H-imidazole, 1,1'-oxalyldimidazole, diphenylphosphoryl azide, 1H-benzotriazol-1-yloxytripyrrolidinophosphpnium hexafluorophosphate, 1H-benzotriazol-1-yloxytris(dimethylamino)phosphonium hexafluorophosphate, N,N,N',N'-tetramethyl-O-(N-succinimidyl)uronium tetrafluoroborate, N-(1,2,2,2-tetrachloroethoxycarbonyloxy)succinimide, O-(6-chlorobenzotriazol-1-yl)-N,N,N',N'-tetramethyluronium hexafluorophosphate, 2-chloro-1,3-dimethylimidazolinium chloride, 2-chloro-1,3-dimethylimidazolinium hexafluorophosphate, 2-chloro-1-methylpyridinium iodide and 2-chioro-1-methylpyridinium p-toluenesulfonate, and more preferred are dicyclohexylcarbodiimide, 1-ethyl-3-(3-dimethylaminopropyl)carbodiimide, 1-ethyl-3-(3-dimethylaminopropyl)carbodiimide hydrochloric acid salt (water-soluble carbodiimide: available as WSC), bis(2,6-diisopropylphenyl)carbodiimide, 2,2'-carbonylbis-1H-imidazole, 1H-benzotriazol-1-yloxytripyrrolidinophosphpnium hexafluorophosphate, 1H-benzotriazol-1-yloxytris(dimethylamino)phosphonium hexafluorophosphate, N,N,N',N'-tetramethyl-O-(N-succinimidyl)uronium tetrafluoroborate, 0-(6-chlorobenzotriazol-1-yl)-N,N,N',N'-tetramethyluronium hexafluorophosphate, 2-chloro-2,3-dimethylimidazolinium chloride and 2-chloro-1-methylpyridinium iodide, and especially preferred are dicyclohexylcarbodiimide, 1-ethyl-3-(3-dimethylaminopropyl)carbodiimide, 1-ethyl-3-(3-dimethylaminopropyl)carbodiimide hydrochloric acid salt (water-soluble carbodiimide: available as WSC), bis(2,6-diisopropylphenyl)carbodiimide, 2,2'-carbonylbis-1H-imidazole and 2-chloro-1-methylpyridinium iodide.

The amount of the condensation agent to be used is preferably 1 to 3 moles per 1 mole of compound (3-A).

Additives such as N-hydroxysuccinimide, benzotriazole and p-nitrophenol may be used together with the condensation agent.

The amount of the additives to be used is preferably 0.03 to 1.2 moles per 1 mole of the condensation agent.

The reaction of compound (3-A) and compound (5) may be conducted in the presence of a catalyst such as N,N-dimethylaminopyridine, N,N-dimethylaniline and dimethylammonium pentafluorobenzenesulfonate. Among them, preferred are N,N-dimethylaminopyridine and N,N-dimethylaniline, and more preferred is N,N-dimethylaminopyridine.

The amount of the catalyst to be used is preferably 0.01 to 0.5 mole per 1 mole of compound (3-A).

The reaction of compound (3-A) and compound (5) is usually conducted in the presence of a solvent. Examples of the solvent include ketone solvents such as acetone, methyl ethyl ketone, cyclopentanone, cyclohexanone, methyl amyl ketone and methyl isobutyl ketone; aliphatic hydrocarbon solvents such as pentane, hexane and heptane; aromatic hydrocarbon solvents such as toluene, xylene, benzene and chlorobenzene; nitrile solvents such as acetonitrile; ether solvents such as tetrahydrofuran and dimethoxyethane; ester solvents such as ethyl lactate; and halogenated hydrocarbon solvents such as chloroform and dichloromethane. These organic solvents may be used alone, or two or more kinds thereof may be used in combination. The amount of the solvent to be used is preferably 100 to 1400% by mass per total amount of compound (3-A) and compound (5), more preferably 100 to 900% by mass, especially preferably 100 to 500% by mass.

The amount of compound (5) to be used is preferably 0.8 to 1.5 moles per 1 mole of compound (3-A), more preferably 0.9 to 1.2 moles, especially preferably 0.98 to 1.1 moles.

The reaction temperature is preferably -20 to 120°C, more preferably 0 to 80°C, especially preferably 15 to 45°C.

The reaction time is preferably 1 minute to 72 hours, more preferably 1 to 48 hours, especially preferably 1 to 24 hours.

Examples of compound (5) include the compounds represented by formulae (5-1-a) to (5-36-e).

| | |
|---|---|
| 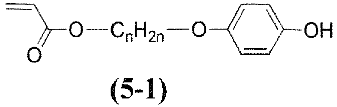 | **(5-1-a) n=4** |
| | **(5-1-b) n=6** |
| | **(5-1-c) n=8** |
| | **(5-1-d) n=11** |
| | **(5-1-e) n=12** |
| | |
| 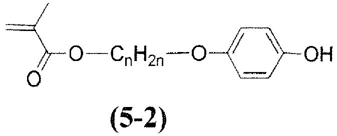 | **(5-2-a) n=4** |
| | **(5-2-b) n=6** |
| | **(5-2-c) n=8** |
| | **(5-2-d) n=11** |
| | **(5-2-e) n=12** |
| | |
| 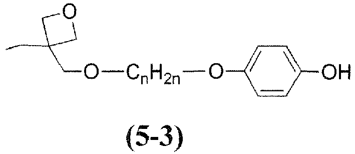 | **(5-3-a) n=4** |
| | **(5-3-b) n=6** |
| | **(5-3-c) n=8** |
| | **(5-3-d) n=11** |
| | **(5-3-e) n=12** |
| | |
| 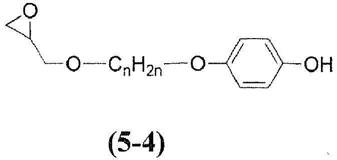 | **(5-4-a) n=4** |
| | **(5-4-b) n=6** |
| | **(5-4-c) n=8** |
| | **(5-4-d) n=11** |
| | **(5-4-e) n=12** |
| | |
| 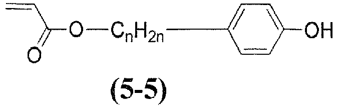 | **(5-5-a) n=4** |
| | **(5-5-b) n=6** |
| | **(5-5-c) n=8** |
| | **(5-5-d) n=11** |
| | **(5-5-e) n=12** |
| 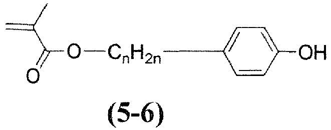 | **(5-6-a) n=4** |
| | **(5-6-b) n=6** |
| | **(5-6-c) n=8** |
| | **(5-6-d) n=11** |
| | **(5-6-e) n=12** |
| | |
| 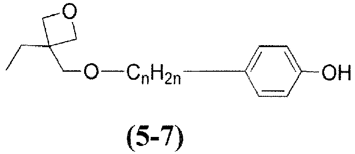 | **(5-7-a) n=4** |
| | **(5-7-b) n=6** |
| | **(5-7-c) n=8** |
| | **(5-7-d) n=11** |
| | **(5-7-e) n=12** |
| | |
| 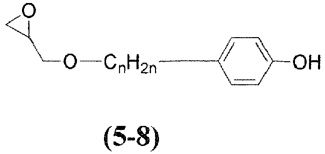 | **(5-8-a) n=4** |
| | **(5-8-b) n=6** |
| | **(5-8-c) n=8** |
| | **(5-8-d) n=11** |
| | **(5-8-e) n=12** |
| | |
| 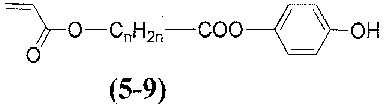 | **(5-9-a) n=3** |
| | **(5-9-b) n=5** |
| | **(5-9-c) n=7** |
| | **(5-9-d) n=10** |
| | **(5-9-e) n=11** |
| | |
| 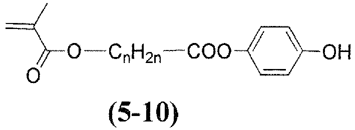 | **(5-10-a) n=3** |
| | **(5-10-b) n=5** |
| | **(5-10-c) n=7** |
| | **(5-10-d) n=10** |
| | **(5-10-e) n=11** |
| 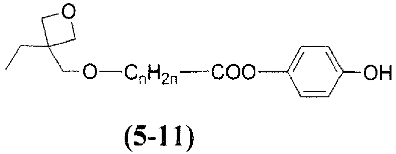 | **(5-11-a) n=3** |
| | **(5-11-b) n=5** |
| | **(5-11-c) n=7** |
| | **(5-11-d) n=10** |
| | **(5-11-e) n=11** |
| | |
| 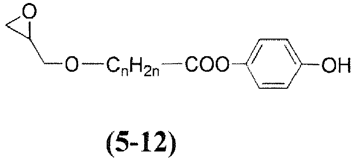 | **(5-12-a) n=3** |
| | **(5-12-b) n=5** |
| | **(5-12-c) n=7** |
| | **(5-12-d) n=10** |
| | **(5-12-e) n=11** |
| | |
| 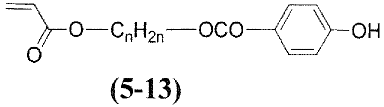 | **(5-13-a) n=3** |
| | **(5-13-b) n=5** |
| | **(5-13-c) n=7** |
| | **(5-13-d) n=10** |
| | **(5-13-e) n=11** |
| | |
| 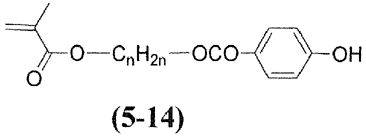 | **(5-14-a) n=3** |
| | **(5-14-b) n=5** |
| | **(5-14-c) n=7** |
| | **(5-14-d) n=10** |
| | **(5-14-e) n=11** |
| | |
| 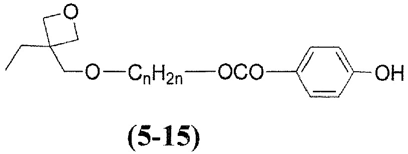 | **(5-15-a) n=3** |
| | **(5-15-b) n=5** |
| | **(5-15-c) n=7** |
| | **(5-15-d) n=10** |
| | **(5-15-e) n=11** |
| 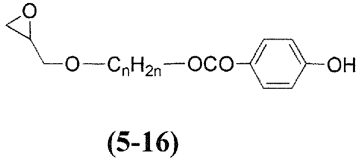 | **(5-16-a) n=3** |
| | **(5-16-b) n=5** |
| | **(5-16-c) n=7** |
| | **(5-16-d) n=10** |
| | **(5-16-e) n=11** |
| | |
| 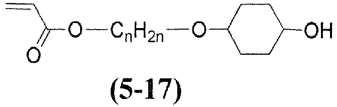 | **(5-17-a) n=4** |
| | **(5-17-b) n=6** |
| | **(5-17-c) n=8** |
| | **(5-17-d) n=11** |
| | **(5-17-e) n=12** |
| | |
| 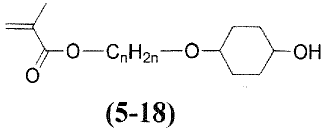 | **(5-18-a) n=4** |
| | **(5-18-b) n=6** |
| | **(5-18-c) n=8** |
| | **(5-18-d) n=11** |
| | **(5-18-e) n=12** |
| | |
| 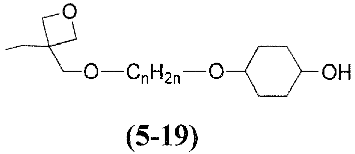 | **(5-19-a) n=4** |
| | **(5-19-b) n=6** |
| | **(5-19-c) n=8** |
| | **(5-19-d) n=11** |
| | **(5-19-e) n=12** |
| | |
| 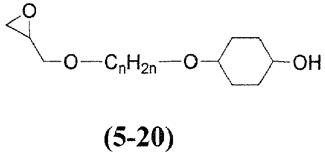 | **(5-20-a) n=4** |
| | **(5-20-b) n=6** |
| | **(5-20-c) n=8** |
| | **(5-20-d) n=11** |
| | **(5-20-e) n=12** |
| 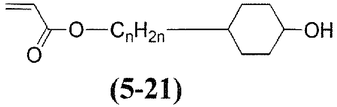 | **(5-21-a) n=4** |
| | **(5-21-b) n=6** |
| | **(5-21-c) n=8** |
| | **(5-21-d) n=11** |
| | **(5-21-e) n=12** |
| | |
| 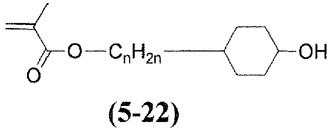 | **(5-22-a) n=4** |
| | **(5-22-b) n=6** |
| | **(5-22-c) n=8** |
| | **(5-22-d) n=11** |
| | **(5-22-e) n=12** |
| | |
| 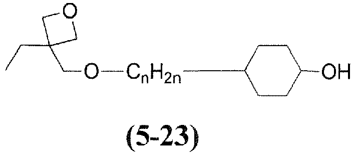 | **(5-23-a) n=4** |
| | **(5-23-b) n=6** |
| | **(5-23-c) n=8** |
| | **(5-23-d) n=11** |
| | **(5-23-e) n=12** |
| | |
| 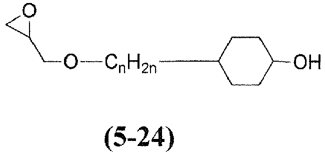 | **(5-24-a) n=4** |
| | **(5-24-b) n=6** |
| | **(5-24-c) n=8** |
| | **(5-24-d) n=11** |
| | **(5-24-e) n=12** |
| | |
| 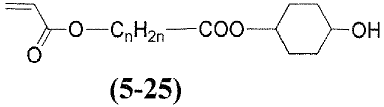 | **(5-25-a) n=3** |
| | **(5-25-b) n=5** |
| | **(5-25-c) n=7** |
| | **(5-25-d) n=10** |
| | **(5-25-e) n=11** |
| 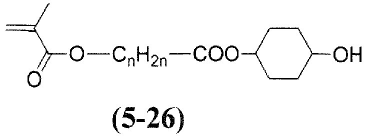 | **(5-26-a) n=3** |
| | **(5-26-b) n=5** |
| | **(5-26-c) n=7** |
| | **(5-26-d) n=10** |
| | **(5-26-e) n=11** |
| | |
| 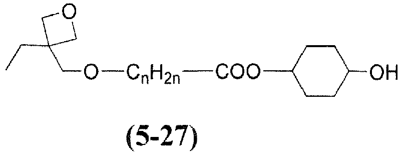 | **(5-27-a) n=3** |
| | **(5-27-b) n=5** |
| | **(5-27-c) n=7** |
| | **(5-27-d) n=10** |
| | **(5-27-e) n=11** |
| | |
| 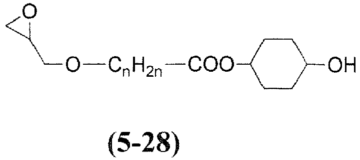 | **(5-28-a) n=3** |
| | **(5-28-b) n=5** |
| | **(5-28-c) n=7** |
| | **(5-28-d) n=10** |
| | **(5-28-e) n=11** |
| | |
| 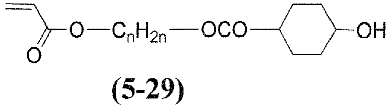 | **(5-29-a) n=3** |
| | **(5-29-b) n=5** |
| | **(5-29-c) n=7** |
| | **(5-29-d) n=10** |
| | **(5-29-e) n=11** |
| | |
| 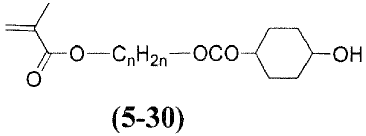 | **(5-30-a) n=3** |
| | **(5-30-b) n=5** |
| | **(5-30-c) n=7** |
| | **(5-30-d) n=10** |
| | **(5-30-e) n=11** |
| 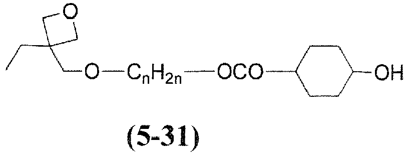 | **(5-31-a) n=3** |
| | **(5-31-b) n=5** |
| | **(5-31-c) n=7** |
| | **(5-31-d) n=10** |
| | **(5-31-e) n=11** |
| | |
| 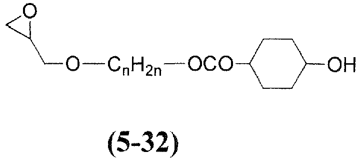 | **(5-32-a) n=3** |
| | **(5-32-b) n=5** |
| | **(5-32-c) n=7** |
| | **(5-32-d) n=10** |
| | **(5-32-e) n=11** |
| | |
| 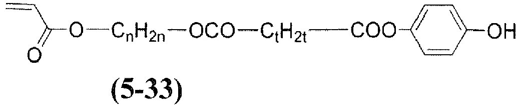 | **(5-33-a) n=2 t=2** |
| | **(5-33-b) n=2 t=4** |
| | **(5-33-c) n=2 t=5** |
| | **(5-33-d) n=4 t=2** |
| | **(5-33-e) n=4 t=4** |
| | |
| 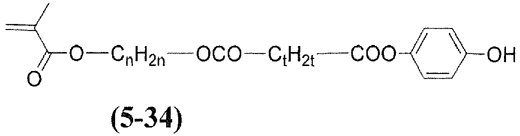 | **(5-34-a) n=2 t=2** |
| | **(5-34-b) n=2 t=4** |
| | **(5-34-c) n=2 t=5** |
| | **(5-34-d) n=4 t=2** |
| | **(5-34-e) n=4 t=4** |
| | |
| 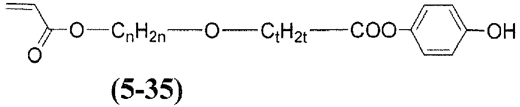 | **(5-35-a) n=2 t=2** |
| | **(5-35-b) n=2 t=4** |
| | **(5-35-c) n=2 t=5** |
| | **(5-35-d) n=4 t=2** |
| | **(5-35-e) n=4 t=4** |
| 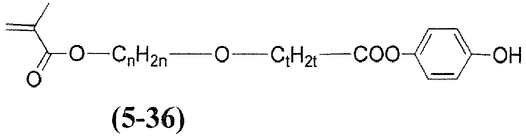 | **(5-36-a) n=2 t=2** |
| | **(5-36-b) n=2 t=4** |
| | **(5-36-c) n=2 t=5** |
| | **(5-36-d) n=4 t=2** |
| | **(5-36-e) n=4 t=4** |

Examples of compound (6) include the compounds represented by formulae (6-1-a) to (6-20-e).

| | |
|---|---|
| 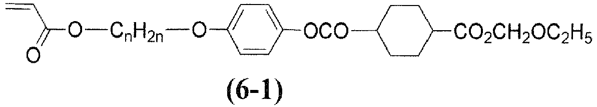 | **(6-1-a) n=4** |
| | **(6-1-b) n=6** |
| | **(6-1-c) n=8** |
| | **(6-1-d) n=11** |
| | **(6-1-e) n=12** |
| | |
| 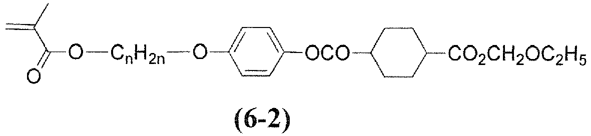 | **(6-2-a) n=4** |
| | **(6-2-b) n=6** |
| | **(6-2-c) n=8** |
| | **(6-2-d) n=11** |
| | **(6-2-e) n=12** |
| | |
| 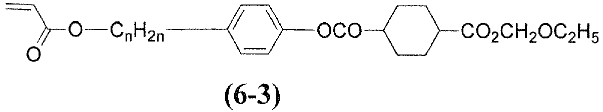 | **(6-3-a) n=4** |
| | **(6-3-b) n=6** |
| | **(6-3-c) n=8** |
| | **(6-3-d) n=11** |
| | **(6-3-e) n=12** |
| 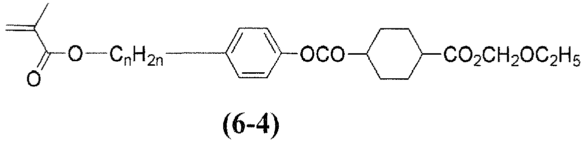 | **(6-4-a) n=4** |
| | **(6-4-b) n=6** |
| | **(6-4-c) n=8** |
| | **(6-4-d) n=11** |
| | **(6-4-e) n=12** |
| | |
| 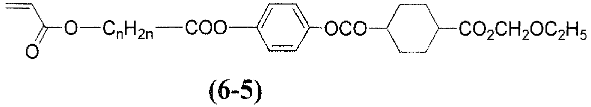 | **(6-5-a) n=3** |
| | **(6-5-b) n=5** |
| | **(6-5-c) n=7** |
| | **(6-5-d) n=10** |
| | **(6-5-e) n=11** |
| | |
| 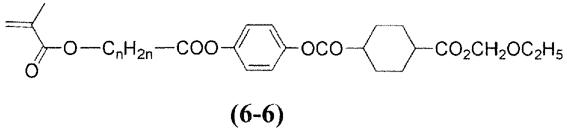 | **(6-6-a) n=3** |
| | **(6-6-b) n=5** |
| | **(6-6-c) n=7** |
| | **(6-6-d) n=10** |
| | **(6-6-e) n=11** |
| | |
| 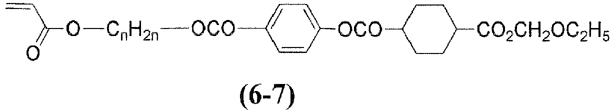 | **(6-7-a) n=3** |
| | **(6-7-b) n=5** |
| | **(6-7-c) n=7** |
| | **(6-7-d) n=10** |
| | **(6-7-e) n=11** |
| | |
| 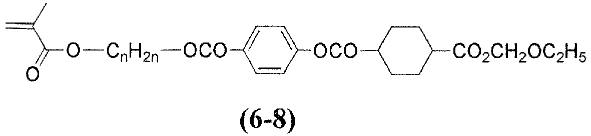 | **(6-8-a) n=3** |
| | **(6-8-b) n=5** |
| | **(6-8-c) n=7** |
| | **(6-8-d) n=10** |
| | **(6-8-e) n=11** |
| 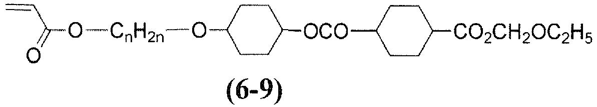 | **(6-9-a) n=4** |
| | **(6-9-b) n=6** |
| | **(6-9-c) n=8** |
| | **(6-9-d) n=11** |
| | **(6-9-e) n=12** |
| | |
| 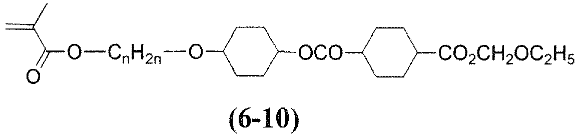 | **(6-10-a) n=4** |
| | **(6-10-b) n=6** |
| | **(6-10-c) n=8** |
| | **(6-10-d) n=11** |
| | **(6-10-e) n=12** |
| | |
| 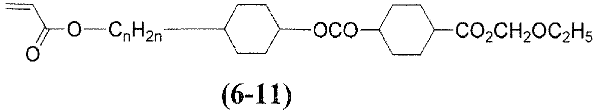 | (**6**-**11**-**a**) **n=4** |
| | **(6-11-b) n=6** |
| | **(6-11-c) n=8** |
| | **(6-11-d) n=11** |
| | **(6-11-e) n=12** |
| | |
| 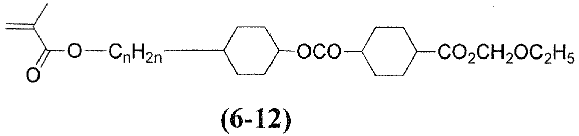 | **(6-12-a) n=4** |
| | **(6-12-b) n=6** |
| | **(6-12-c) n=8** |
| | **(6-12-d) n=11** |
| | **(6-12-e) n=12** |
| | |
| 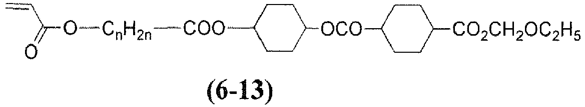 | **(6-13-a) n=3** |
| | **(6-13-b) n=5** |
| | **(6-13-c) n=7** |
| | **(6-13-d) n=10** |
| | **(6-13-e) n=11** |
| 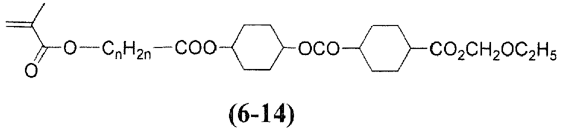 | **(6-14-a) n=3** |
| | **(6-14-b) n=5** |
| | **(6-14-c) n=7** |
| | **(6-14-d) n=10** |
| | **(6-14-e) n=11** |
| 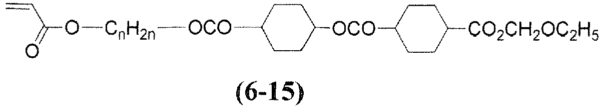 | **(6-15-a) n=3** |
| | **(6-15-b) n=5** |
| | **(6-15-c) n=7** |
| | **(6-15-d) n=10** |
| | **(6-15-e) n=11** |
| | |
| 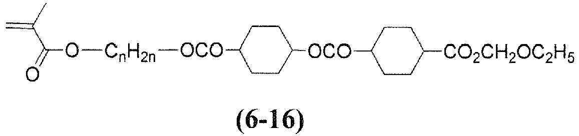 | (**6**-**16**-**a**) **n=3** |
| | (6-16-b) **n=5** |
| | (6-16-c) **n=7** |
| | (6-16-d) **n=10** |
| | (6-16-e) **n=11** |
| | |
| 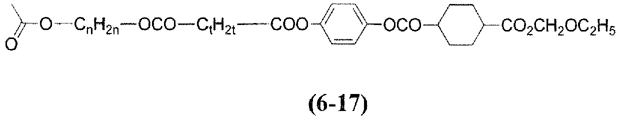 | (6-17-a) **n=2** t=2 |
| | (6-17-b) **n=2** t=4 |
| | (6-17-c) **n=2** t=5 |
| | (6-17-d) **n=4** t=2 |
| | (6-17-e) **n=4** t=4 |
| | |
| 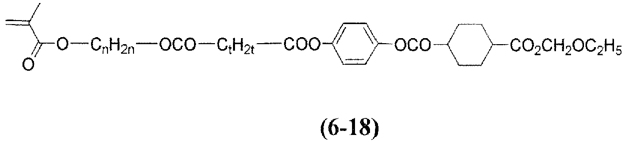 | **(6-18-a) n=2 t=2** |
| | **(6-18-b) n=2 t=4** |
| | **(6-18-c) n=2 t=5** |
| | **(6-18-d) n=4 t=2** |
| | **(6-18-e) n=4 t=4** |
| 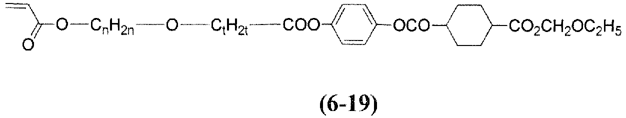 | **(6-19-a) n=2 t=2** |
| | **(6-19-b) n=2 t=4** |
| | **(6-19-c) n=2 t=5** |
| | **(6-19-d) n=4 t=2** |
| | **(6-19-e) n=4 t=4** |
| | |
| 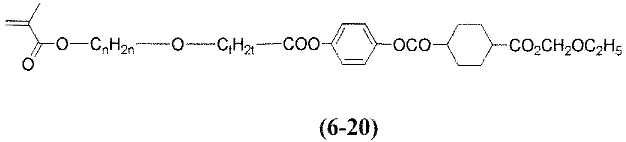 | **(6-20-a) n=2 t=2** |
| | **(6-20-b) n=2 t=4** |
| | **(6-20-c) n=2 t=5** |
| | **(6-20-d) n=4 t=2** |
| | **(6-20-e) n=4 t=4** |

Next, the fourth step will be illustrated.

The fourth step is a step of deprotecting the group represented by Z of compound (6) to obtain polymerizable compound (7).

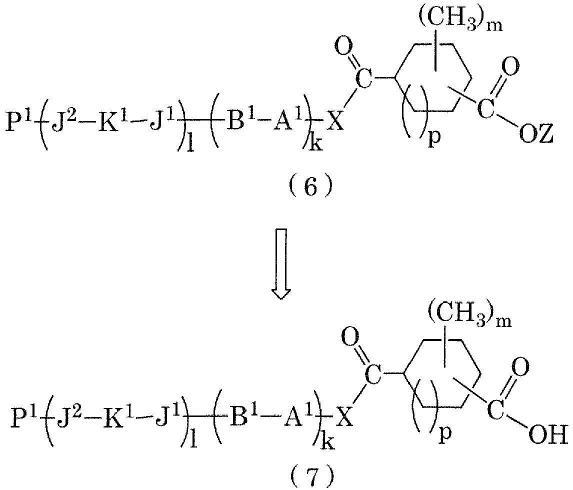

Examples of the deprotection processes of the group represented by Z include a process for deprotecting with an acid as same as those in the second step, and can be conducted under the same conditions as those in the second step. As the acid, preferred are Brønsted acids, and more preferred is at least one selected from the group consisting of formic acid, acetic acid, propionic acid, hydrochloric acid, hydrobromic acid, hydrogen iodide, nitric acid, sulfuric acid, p-toluenesulfonic acid, pyridinium p-toluenesulfonate, trifluoroacetic acid, trichloroacetic acid and methanesulfonic acid.

Examples of compound (7) include the compounds represented by formulae (7-1-a) to (7-36-e).

| | |
|---|---|
| 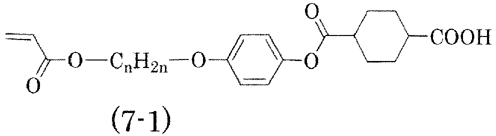 | **(7-1-a) n=4** |
| | **(7-1-b) n=6** |
| | **(7-1-c) n=8** |
| | **(7-1-d) n=11** |
| | **(7-1-e) n=12** |
| | |
| 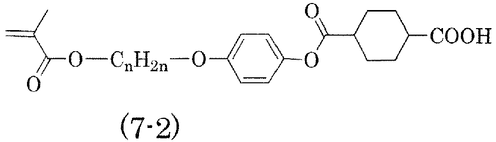 | **(7-2-a) n=4** |
| | **(7-2-b) n=6** |
| | **(7-2-c) n=8** |
| | **(7-2-d) n=11** |
| | **(7-2-e) n=12** |
| | |
| 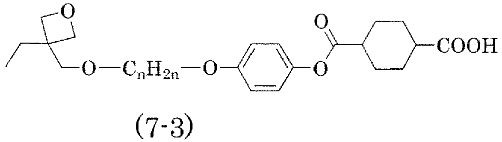 | **(7-3-a) n=4** |
| | **(7-3-b) n=6** |
| | **(7-3-c) n=8** |
| | **(7-3-d) n=11** |
| | **(7-3-e) n=12** |
| | |
| 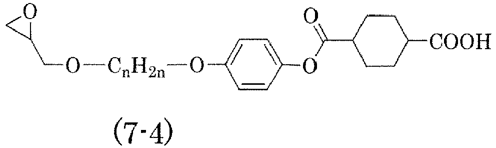 | **(7-4-a) n=4** |
| | **(7-4-b) n=6** |
| | **(7-4-c) n=8** |
| | **(7-4-d) n=11** |
| | **(7-4-e) n=12** |
| 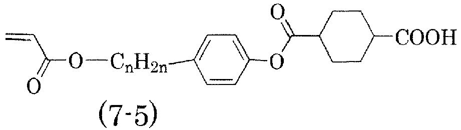 | **(7-5-a) n=4** |
| | **(7-5-b) n=6** |
| | **(7-5-c) n=8** |
| | **(7-5-d) n=11** |
| | **(7-5-e) n=12** |
| | |
| 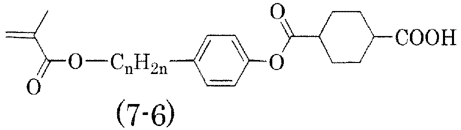 | **(7-6-a) n=4** |
| | **(7-6-b) n=6** |
| | **(7-6-c) n=8** |
| | **(7-6-d) n=11** |
| | **(7-6-e) n=12** |
| | |
| 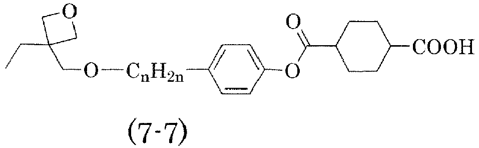 | **(7-7-a) n=4** |
| | **(7-7-b) n=6** |
| | **(7-7-c) n=8** |
| | **(7-7-d) n=11** |
| | **(7-7-e) n=12** |
| | |
| 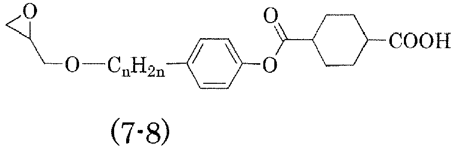 | **(7-8-a) n=4** |
| | **(7-8-b) n=6** |
| | **(7-8-c) n=8** |
| | **(7-8-d) n=11** |
| | **(7-8-e) n=12** |
| | |
| 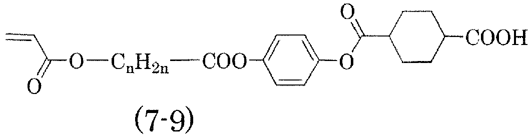 | **(7-9-a) n=3** |
| | **(7-9-b) n=5** |
| | **(7-9-c) n=7** |
| | **(7-9-d) n=10** |
| | **(7-9-e) n=11** |
| 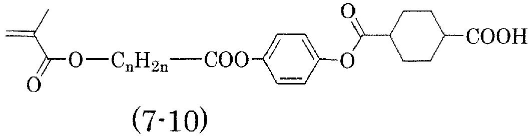 | **(7-10-a) n=3** |
| | **(7-10-b) n=5** |
| | **(7-10-c) n=7** |
| | **(7-10-d) n=10** |
| | **(7-10-e) n=11** |
| | |
| 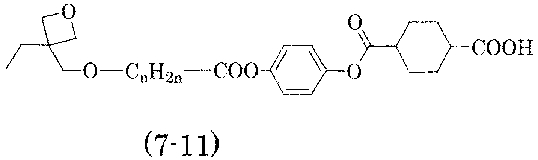 | **(7-11-a) n=3** |
| | **(7-11-b) n=5** |
| | **(7-11-c) n=7** |
| | **(7-11-d) n=10** |
| | **(7-11-e) n=11** |
| | |
| 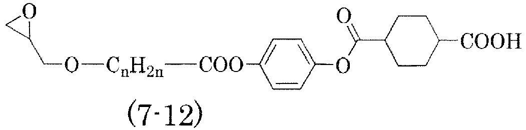 | **(7-12-a) n=3** |
| | **(7-12-b) n=5** |
| | **(7-12-c) n=7** |
| | **(7-12-d) n=10** |
| | **(7-12-e) n=11** |
| 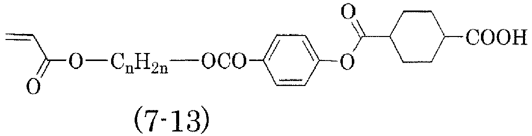 | |
| | **(7-13-a) n=3** |
| | **(7-13-b) n=5** |
| | **(7-13-c) n=7** |
| | **(7-13-d) n=10** |
| | **(7-13-e) n=11** |
| 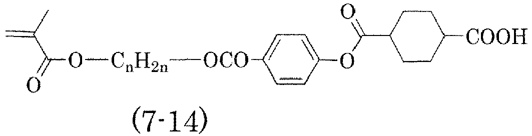 | |
| | **(7-14-a) n=3** |
| | **(7-14-b) n=5** |
| | **(7-14-c) n=7** |
| | **(7-14-d) n=10** |
| | **(7-14-e) n=11** |
| 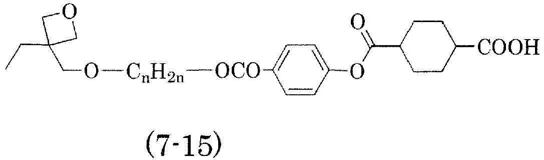 | **(7-15-a) n=3** |
| | **(7-15-b) n=5** |
| | **(7-15-c) n=7** |
| | **(7-15-d) n=10** |
| | **(7-15-e) n=11** |
| | |
| 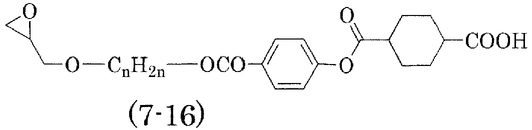 | **(7-16-a) n=3** |
| | **(7-16-b) n=5** |
| | **(7-16-c) n=7** |
| | **(7-16-d) n=10** |
| | **(7-16-e) n=11** |
| | |
| 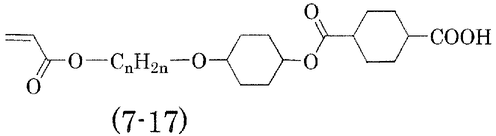 | **(7-17-a) n=4** |
| | **(7-17-b) n=6** |
| | **(7-17-c) n=8** |
| | **(7-17-d) n=11** |
| | **(7-17-e) n=12** |
| 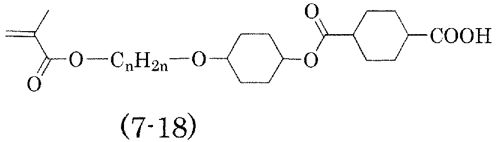 | |
| | **(7-18-a) n=4** |
| | **(7-18-b) n=6** |
| | **(7-18-c) n=8** |
| | **(7-18-d) n=11** |
| | **(7-18-e) n=12** |
| | |
| 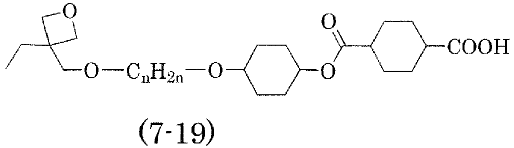 | **(7-19-a) n=4** |
| | **(7-19-b) n=6** |
| | **(7-19-c) n=8** |
| | **(7-19-d) n=11** |
| | **(7-19-e) n=12** |
| 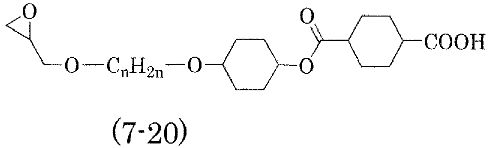 | **(7-20-a) n=4** |
| | **(7-20-b) n=6** |
| | **(7-20-c) n=8** |
| | **(7-20-d) n=11** |
| | **(7-20-e) n=12** |
| | |
| 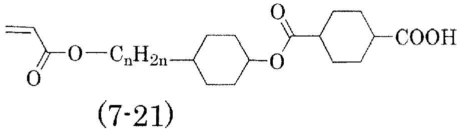 | **(7-21-a) n=4** |
| | **(7-21-b) n=6** |
| | **(7-21-c) n=8** |
| | **(7-21-d) n=11** |
| | **(7-21-e) n=12** |
| | |
| 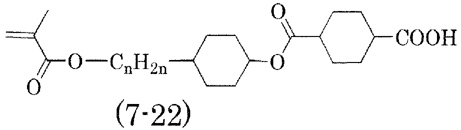 | **(7-22-a) n=4** |
| | **(7-22-b) n=6** |
| | **(7-22-c) n=8** |
| | **(7-22-d) n=11** |
| | **(7-22-e) n=12** |
| | |
| 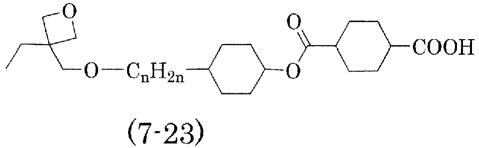 | **(7-23-a) n=4** |
| | **(7-23-b) n=6** |
| | **(7-23-c) n=8** |
| | **(7-23-d) n=11** |
| | **(7-23-e) n=12** |
| | |
| 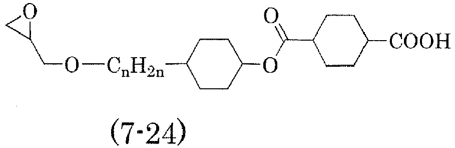 | **(7-24-a) n=4** |
| | **(7-24-b) n=6** |
| | **(7-24-c) n=8** |
| | **(7-24-d) n=11** |
| | **(7-24-e) n=12** |
| 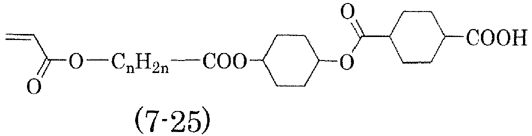 | **(7-25-a) n=3** |
| | **(7-25-b) n=5** |
| | **(7-25-c) n=7** |
| | **(7-25-d) n=10** |
| | **(7-25-e) n=11** |
| | |
| 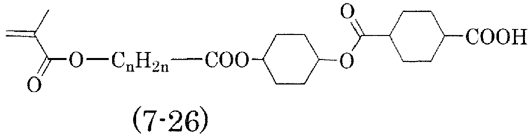 | **(7-26-a) n=3** |
| | **(7-26-b) n=5** |
| | **(7-26-c) n=7** |
| | **(7-26-d) n=10** |
| | **(7-26-e) n=11** |
| | |
| 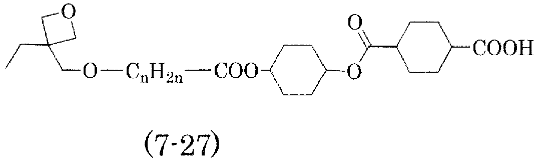 | **(7-27-a) n=3** |
| | **(7-27-b) n=5** |
| | **(7-27-c) n=7** |
| | **(7-27-d) n=10** |
| | **(7-27-e) n=11** |
| | |
| 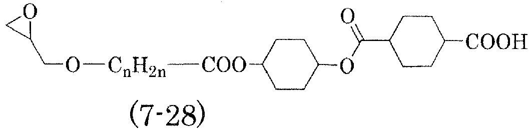 | **(7-28-a) n=3** |
| | **(7-28-b) n=5** |
| | **(7-28-c) n=7** |
| | **(7-28-d) n=10** |
| | **(7-28-e) n=11** |
| | |
| 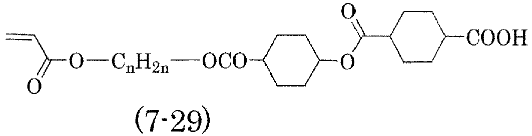 | **(7-29-a) n=3** |
| | **(7-29-b) n=5** |
| | **(7-29-c) n=7** |
| | **(7-29-d) n=10** |
| | **(7-29-e) n=11** |
| 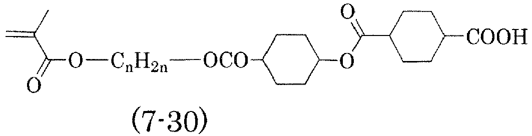 | **(7-30-a) n=3** |
| | **(7-30-b) n=5** |
| | **(7-30-c) n=7** |
| | **(7-30-d) n=10** |
| | **(7-30-e) n=11** |
| | |
| 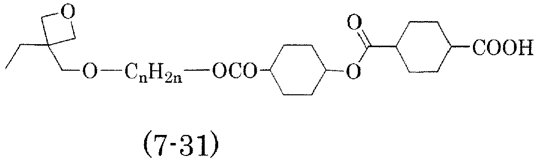 | **(7-31-a) n=3** |
| | **(7-31-b) n=5** |
| | **(7-31-c) n=7** |
| | **(7-31-d) n=10** |
| | **(7-31-e) n=11** |
| | |
| 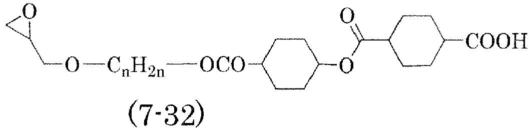 | **(7-32-a) n=3** |
| | **(7-32-b) n=5** |
| | **(7-32-c) n=7** |
| | **(7-32-d) n=10** |
| | **(7-32-e) n=11** |
| | |
| 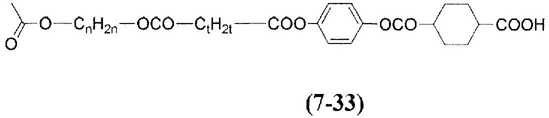 | **(7-33-a) n=2 t=2** |
| | **(7-33-b) n=2 t=4** |
| | **(7-33-c) n=2 t=5** |
| | **(7-33-d) n=4 t=2** |
| | **(7-33-e) n=4 t=4** |
| | |
| 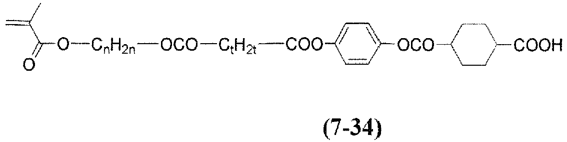 | **(7-34-a) n=2 t=2** |
| | **(7-34-b) n=2 t=4** |
| | **(7-34-c) n=2 t=5** |
| | **(7-34-d) n=4 t=2** |
| | **(7-34-e) n=4 t=4** |
| 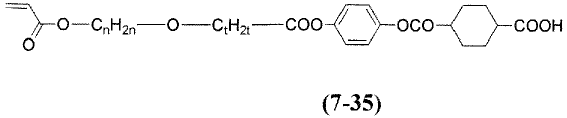 | **(7-35-a) n=2 t=2** |
| | **(7-35-b) n=2 t=4** |
| | **(7-35-c) n=2 t=5** |
| | **(7-35-d) n=4 t=2** |
| | **(7-35-e) n=4 t=4** |
| | |
| 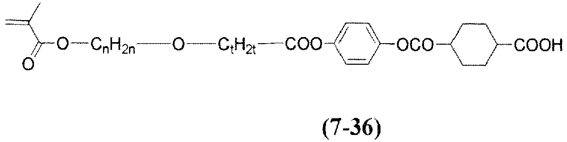 | **(7-36-a) n=2 t=2** |
| | **(7-36-b) n=2 t=4** |
| | **(7-36-c) n=2 t=5** |
| | **(7-36-d) n=4 t=2** |
| | **(7-36-e) n=4 t=4** |

### Examples

The present invention will be illustrated in more detail by Examples below.

The "%" and "part(s)" in the following Examples are % by weight and part(s) by weight unless otherwise specifically noted.

### Example 1

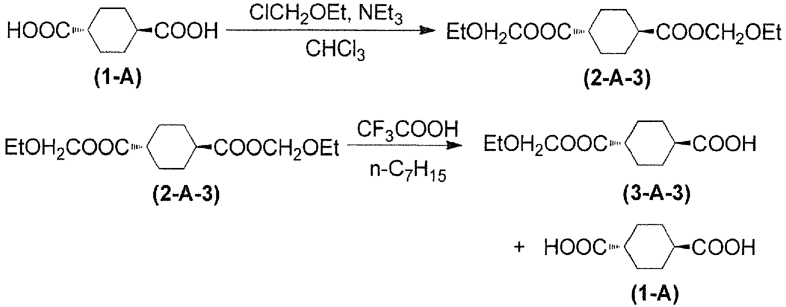

### <First step>

Two hundred (200) grams of a compound represented by formula (1-A) (trans-1,4-cyclohexanedicarboxylic acid) was mixed with 1000 mL of chloroform. To the mixture obtained, 176.31 g of triethylamine was added under an atmosphere of nitrogen with stirring and cooling with ice to obtain a homogeneous solution. To the homogeneous solution obtained, 230.61 g of ethoxychloromethane and 176.31 g of triethylamine were respectively added dropwise under ice-cooling with maintaining pH 7 or more. The solution obtained was stirred for 5 hours under ice-cooling, and then, white precipitation precipitated was removed by filtration. The filtrate obtained was washed twice with 500 mL of pure water. The organic layer obtained was concentrated under reduced pressure with an evaporator. To the residue obtained, heptane was added, and the mixture obtained was dried under reduced pressure to obtain 304. 37 g of a compound represented by formula (2-A-3). Yield: 91% (based on the compound represented by formula (1-A)).
¹H-NMR of the compound represented by formula (2-A-3) (CDCl₃): δ (ppm) 1.20-1.26 (t, 6H), 1. 41-1. 57 (m, 4H), 2.08-2.11 (d, 4H), 2.32 (br, 2H), 3.64-3.72 (q, 4H), 5.29 (s, 4H)

### <Second step>

Two hundred eight (208) grams of the compound represented by formula (2-A-3) was mixed with 1200 mL of heptane. The mixture obtained was stirred at 40°C under an atmosphere of nitrogen to obtain a solution of the compound represented by formula (2-A-3) . A solution prepared by mixing 82.25 g of trifluoroacetic acid with 370 mL of heptane was added dropwise to the solution of the compound represented by formula (2-A-3) at 40°C with stirring. The reaction mixture was cooled down to room temperature at the time the reaction mixture during the addition dropwise thereof had begun to become cloudy, and then, the addition was further continued. The total time of addition of the heptane solution of trifluoroacrtic acid was 4 hours. The reaction mixture obtained was concentrated under reduced pressure. To the residue obtained, 800 mL of chloroform and 43 g of silica gel were added. The mixture obtained was filtrated through Celite to obtain a solid matter containing a compound represented by formula (1-A) which was a by-product, and a filtrate containing a compound represented by formula (3-A-3).

The solid matter obtained was mixed with 400 mL of acetone, and the mixture obtained was stirred for 1 hour, and then, filtrated to obtain a filtrate. The filtrate obtained was concentrated, and the residue obtained was dried in a vacuum to obtain 30 g of a compound represented by formula (1-A). Yield: 24.2% (based on the compound represented by formula (2-A-3)).

The filtrate containing the compound represented by formula (3-A-3) which has been obtained in the above was concentrated under reduced pressure. To the residue obtained, 400 mL of heptane was added, and then, the mixture obtained was stirred for 15 minutes. The mixture obtained was filtrated to obtain a solid matter and a filtrate. The operation wherein the solid matter obtained was mixed with heptane and the mixture obtained was filtrated to obtain a solid matter and a filtrate was repeated three times in total. The filtrates obtained were mixed to concentrate under reduced pressure, and the residue obtained was dried in a vacuum to obtain 89.5 g of white powder of a compound represented by formula (3-A-3).
Yield of a compound represented by formula (3-A-3): 53. 9% (based on the compound represented by formula (2-A-3)).
Recovery rate of the unreacted compound represented by formula (2-A-3): 19.0%
¹H-NMR of the compound represented by formula (3-A-3) (CDCl₃): δ (ppm) 1.20-1.26 (t, 3H), 1.45-1.56 (dt, 4H), 2. 09-2. 12 (br, d, 4H), 2.32 (br, 2H), 3.64-3.72 (q, 2H), 5.29 (s, 2H)

The utilization ratio of cyclohexane in the above-mentioned second step was 97.1%. Hereinafter, "utilization ratio of cyclohexane in the second step" means sum of the yield of the compound represented by formula (3-A-3) in the second step, the yield of the compound represented by formula (1-A) which was a by-product, and the recovery rate of the unreacted compound represented by formula (2-A-3).

Total utilization ratio of cyclohexane was 88.4%. Hereinafter, "Total utilization ratio of cyclohexane" means a product of the utilization ratio of cyclohexane in the second step and the yield of the compound represented by formula (2-A-3) in the first step.

Additionally, the yield of the compound represented by formula (3-A-3) based on the compound represented by formula (1-A) was 49%.

Further, as described in the above, the compound represented by formula (1-A) which was a starting material in the first step and the compound represented by formula (2-A-3) which was a starting material in the second step could be recovered from the reaction mixture by easy operations such as concentration (removal of solvents), crystallization by cooling, crystallization by addition of a poor solvent, and filtration.

### Example 2

### <Reuse 1 of the compound represented by formula (2-A-3) which has been recovered for the second step>

One hundred fifty two point four two (152.42) grams of the compound represented by formula (2-A-3) which has been recovered in the above-mentioned <Second step> of Example 1 was mixed with 900 mL of heptane. The mixture obtained was stirred at 40°C under an atmosphere of nitrogen to obtain a solution of the compound represented by formula (2-A-3). A solution prepared by mixing 48.22 g of trifluoroacetic acid with 250 mL of heptane was added dropwise to the solution of the compound represented by formula (2-A-3) at 40°C with stirring. The reaction mixture was cooled down to room temperature at the time the reaction mixture during the addition dropwise thereof had begun to become cloudy, and then, the addition was further continued. The total time of addition of the heptane solution of trifluoroacrtic acid was 4 hours. The reaction mixture obtained was concentrated under reduced pressure.

To the residue obtained, 400 mL of heptane was added. The mixture obtained was stirred for 15 minutes, and then, filtrated to obtain a solid matter and a filtrate. The operation wherein the solid matter obtained was mixed with heptane and the mixture obtained was filtrated to obtain a solid matter and a filtrate was repeated three times in total. The filtrates obtained were mixed and then, concentrated under reduced pressure, and the residue obtained was dried in a vacuum to obtain 43.5 g of the unreacted compound represented by formula (2-A-3).

To the solid matter obtained, 500 mL of chloroform and 36 g of silica gel were added. The mixture obtained was filtrated through Celite to obtain a solid matter containing a compound represented by formula (1-A) which was a by-product, and a filtrate containing a compound represented by formula (3-A-3). The solid matter was mixed with 400 mL of acetone, and the mixture obtained was stirred for 1 hour, and then, filtrated to obtain a filtrate. The filtrate obtained was concentrated under reduced pressure, and the residue obtained was dried in a vacuum to obtain 1.8 g of a compound represented by formula (1-A).

The filtrate containing the compound represented by formula (3-A-3) which has been obtained was concentrated under reduced pressure. The residue obtained was dried in a vacuum to obtain 81.8 g of white powder of a compound represented by formula (3-A-3).
Recovery rate of the unreacted compound represented by formula (2-A-3): 28.5%
Yield of a compound represented by formula (1-A): 1.9% (based on the compound represented by formula (2-A-3)).
Yield of a compound represented by formula (3-A-3) : 67.2% (based on the compound represented by formula (2-A-3)).

The utilization ratio of cyclohexane in the second step: 97.6%.

Total utilization ratio of cyclohexane: 88.8%.

Additionally, the yield of the compound represented by formula (3-A-3) based on the compound represented by formula (1-A) which has been used in Example 1 was 61%.

Further, as described in the above, the compound represented by formula (1-A) which was a starting material in the first step and the compound represented by formula (2-A-3) which was a starting material in the second step could be recovered from the reaction mixture by easy operations such as concentration (removal of solvents), crystallization by cooling, crystallization by addition of a poor solvent, and filtration.

### Example 3

### <Reuse 2 of the compound represented by formula (2-A-3) which has been recovered for the second step>

Seventy eight point six four (78.64) grams of the compound represented by formula (2-A-3) was mixed with 43.5 g of the compound represented by formula (2-A-3) which has been recovered in the above-mentioned Example 2 and 720 mL of heptane. The mixture obtained was stirred at 40°C under an atmosphere of nitrogen to obtain a solution of the compound represented by formula (2-A-3). A solution prepared by mixing 48.3 g of trifluoroacetic acid with 230 mL of heptane was added dropwise to the solution of the compound represented by formula (2-A-3) at 40°C with stirring. The reaction mixture was cooled down to room temperature at the time the reaction mixture during the addition dropwise thereof had begun to become cloudy, and then, the addition was further continued. The total time of addition of the heptane solution of trifluoroacrtic acid was 4 hours. The reaction mixture obtained was concentrated under reduced pressure.

To the residue obtained, 400 mL of heptane was added. The mixture obtained was stirred for 15 minutes, and then, filtrated to obtain a solid matter and a filtrate. The operation wherein the solid matter obtained was mixed with heptane and the mixture obtained was filtrated to obtain a solid matter and a filtrate was repeated three times in total. The filtrates obtained were mixed and then, concentrated under reduced pressure, and the residue obtained was dried in a vacuum to obtain 24.9 g of the unreacted compound represented by formula (2-A-3).

To the solid matter obtained, 500 mL of chloroform and 36 g of silica gel were added. The mixture obtained was filtrated through Celite to obtain a solid matter containing a compound represented by formula (1-A) which was a by-product, and a filtrate containing a compound represented by formula (3-A-3). The solid matter was mixed with 400 mL of acetone, and the mixture obtained was stirred for 1 hour, and then, filtrated to obtain a filtrate. The filtrate obtained was concentrated under reduced pressure, and the residue obtained was dried in a vacuum to obtain 7.8 g of a compound represented by formula (1-A).

The filtrate containing the compound represented by formula (3-A-3) which has been obtained was concentrated under reduced pressure. The residue obtained was dried in a vacuum to obtain 64.0 g of white powder of a compound represented by formula (3-A-3).
Recovery rate of the unreacted compound represented by formula (2-A-3): 20.4%
Yield of a compound represented by formula (1-A): 10.7% (based on the compound represented by formula (2-A-3)).
Yield of a compound represented by formula (3-A-3) : 65. 6% (based on the compound represented by formula (2-A-3)).

The utilization ratio of cyclohexane in the second step: 96.4%.

Total utilization ratio of cyclohexane: 88%.

Additionally, the yield of the compound represented by formula (3-A-3) based on the compound represented by formula (1-A) which has been used in Example 1 was 60%.

Further, as described in the above, the compound represented by formula (1-A) which was a starting material in the first step and the compound represented by formula (2-A-3) which was a starting material in the second step could be recovered from the reaction mixture by easy operations such as concentration (removal of solvents), crystallization by cooling, crystallization by addition of a poor solvent, and filtration.

### Example 4

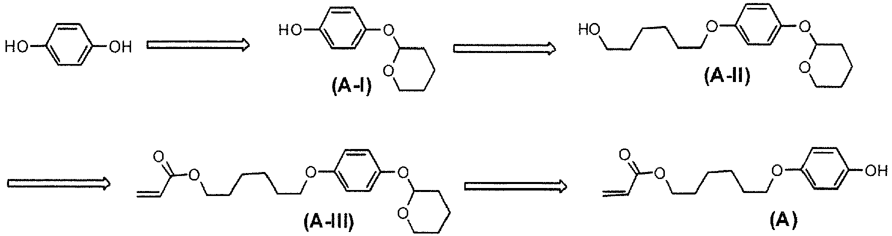

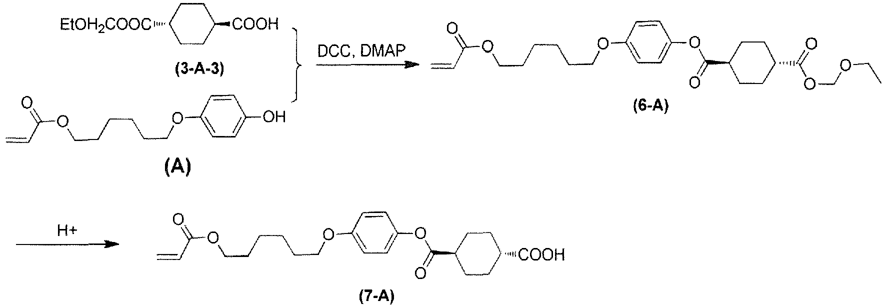

According to the method described in JP 2004-262884 A, the compound represented by formula (A-I) was produced by reacting dihydroquinone with dihydropyran in the presence of an acid catalyst.

One hundred point one (100.1) grams of the compound represented by formula (A-I) was mixed with 97.1 g of potassium carbonate, 64 g of 6-chlorohexanol and N,N-dimethylacetamide, and the mixture obtained was stirred at 90°C under an atmosphere of nitrogen, and further stirred at 100°C. The solution obtained was cooled down to room temperature, and then, pure water and methyl isobutyl ketone were added thereto. The organic layer was separated from the mixture obtained, and then, the organic layer was washed with an aqueous sodium hydroxide solution and pure water. The organic layer was filtrated, and then, concentrated under reduced pressure. To the residue obtained, methanol was added, and the precipitate generated was isolated by filtration. The precipitate isolated was dried in a vacuum to obtain 126 g of a compound represented by formula (A-II). Yield: 91% (based on 6-chlorohexanol).

One hundred twenty six (126) grams of the compound represented by formula (A-II) was mixed with 1.40 g of 3,5-di-tert-butyl-4-hydroxytoluene, 116.7 g of N,N-dimethylaniline, 1.00 g of 1,3-dimethyl-2-imidazolidinone and chloroform. Under an atmosphere of nitrogen, the mixture obtained was cooled with ice, and then, 58.1 1 g of acryloyl chloride was added dropwise thereto. Pure water was further added thereto, and the solution obtained was stirred, and then, an organic layer was separated. The organic layer obtained was washed with hydrochloric acid, saturated aqueous sodium carbonate solution and pure water. The organic layer was dried, and then, 1 g of 3,5-di-tert-butyl-4-hydroxytoluene was added thereto, and the concentration under reduced pressure was conducted to obtain a compound represented by formula (A-III).

To the compound represented by formula (A-III), 200 mL of tetrahydrofuran was added. The solution obtained was concentrated, and then, 200 mL of tetrahydrofuran was added thereto. To the solution obtained, hydrochloric acid and concentrated hydrochloric acid were added to stir at 60°C under an atmosphere of nitrogen. The reaction solution obtained was washed with 500 mL of saturated aqueous sodium chloride solution, and then, an organic layer was dried and concentrated under reduced pressure. To the concentrated residue, hexane was added to stir under ice-cooling. The powder precipitated was filtrated, and dried in a vacuum to obtain 90 g of a compound represented by formula (A). Yield: 79% (based on the compound represented by formula (A-II)).

Fifty six point eight (56.8) grams of the compound represented by formula (A) was mixed with 2.65 g of dimethylaminopyridine, 50 g of a compound represented by formula (3-A-3) and 300 mL of chloroform. A solution obtained by dissolving 48.79 g of dicyclohexylcarbodiimide in 50 mL of chloroform was added dropwise to the mixture obtained with ice-cooling and stirring under an atmosphere of nitrogen. After completion of addition, the mixture obtained was stirred at room temperature for 4 hours to effect reaction. To the reaction solution obtained, 200 mL of chloroform and 200 mL of heptane were added, and precipitation was removed by filtration. The filtrate obtained was washed with 2N hydrochloric acid, and then, insoluble matter was removed by filtration. The filtrate was dried over anhydrous sodium sulfate. After removing sodium sulfate by filtration, concentration was conducted, and the residue obtained was dried in a vacuum to obtain 100 g of a compound represented by formula (6-A).

One hundred (100) grams of the compound represented by formula (6-A) was mixed with 3.64 g of pure water, 3.84 g of p-toluenesulfonic acid monohydrate and 200 mL of tetrahydrofuran. Under an atmosphere of nitrogen, the mixture obtained was stirred at 50°C for 3 hours to effect reaction. The reaction mixture obtained was cooled down to room temperature, and then, concentrated. To the residue obtained, 200 mL of heptane was added. The precipitation precipitated was isolated by filtration, and washed with pure water, and then, dried in a vacuum. The powder obtained was mixed with chloroform, and the mixture obtained was filtrated through silica gel. The filtrate was mixed with 400 mL of chloroform to concentrate. The residue was dissolved in toluene, and the solution obtained was concentrated under reduced pressure. To the concentrated residue, heptane was added to conduct crystallization. The powder obtained was filtrated, and dried in vacuum to obtain 64. 1 g of a compound represented by formula (7-A). Yield: 76% (based on the compound represented by formula (A)). According to the result of high performance liquid chromatography analysis, the purity was 92%, and impurities were not detected.

### Example 5

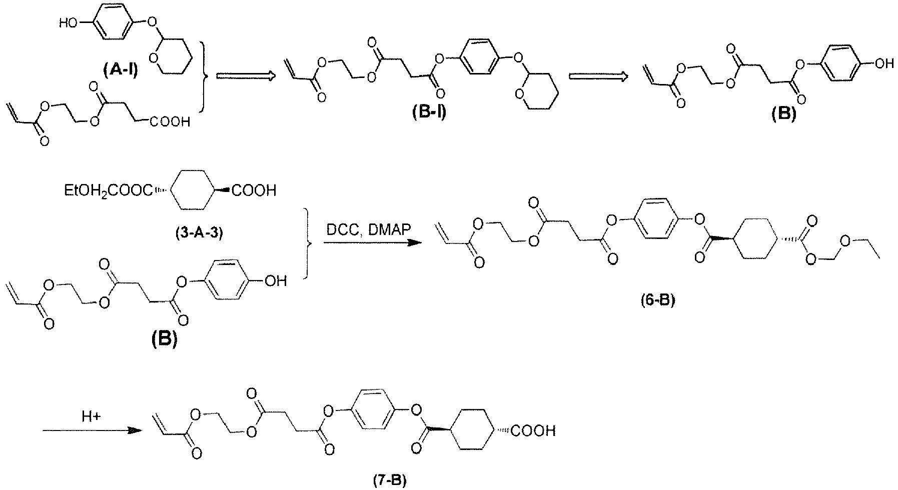

Sixteen point nine (16.9) grams of a compound represented by formula (A-I) was mixed with 0.85 g of dimethylaminopyridine, 15 g of 2-acryloyloxyethyl succinate, 80 mg of 3,5-di-tert-butyl-4-hydroxytoluene and 100 mL of chloroform. The solution obtained was cooled with ice under an atmosphere of nitrogen, and then, a solution obtained by dissolving 19.68 g of dicyclohexylcarbodiimide in 20 mL of chloroform was added dropwise thereto. After completion of addition, the mixture obtained was stirred. To the reaction mixture obtained, 200 mL of chloroform and 200 mL of heptane were added to filtrate precipitation. The filtrate was washed with 2N hydrochloric acid, and then, insoluble matter was removed by filtration. The filtrate was dried over anhydrous sodium sulfate. After removing sodium sulfate by filtration, the filtrate was concentrated. The residue obtained was dried in a vacuum to obtain 21 g of a compound represented by formula (B-1).

Twenty one (21) grams of the compound represented by formula (B-1) was mixed with 1.16 g of pure water, 1.02 g of p-toluenesulfonic acid monohydrate, 100 mg of 3,5-di-tert-butyl-4-hydroxytoluene and 200 mL of tetrahydrofuran. Under an atmosphere of nitrogen, the mixture obtained was stirred at 30°C. The reaction mixture was cooled down to room temperature, and then, 200 ml of toluene was added thereto. The mixture obtained was washed with pure water, and then, 200 mL of heptane was added thereto. The insoluble matter was removed by filtration, and then, the filtrate obtained was concentrated under reduced pressure. To the concentrated residue, chloroform was added, and the mixture obtained was filtrated through silica gel. The filtrate was concentrated under reduced pressure, and the residue obtained was dried in a vacuum to obtain 16 g of a compound represented by formula (B) . Yield: 75% (based on the compound represented by formula (A-I)).

Ten point zero five (10.05) grams of the compound represented by formula (B) was mixed with 0.40 g of dimethylaminopyridine, 7.5 g of a compound represented by formula (3-A-3), 20 mg of 3,5-di-tert-butyl-4-hydroxytoluene and 300 mL of chloroform. The mixture obtained was cooled with ice, and then, a solution obtained by dissolving 7.39 g of dicyclohexylcarbodiimide in 20 mL of chloroform was added dropwise thereto. After completion of addition, the mixture obtained was stirred at room temperature for 3 hours to effect reaction. To the reaction solution, 200 mL of chloroform and 200 mL of heptane were added, and precipitation was removed by filtration. The filtrate obtained was washed with hydrochloric acid, and then, insoluble matter was removed by filtration. The filtrate was dried over anhydrous sodium sulfate. Sodium sulfate was removed by filtration, and the filtrate was concentrated. The residue obtained was dried in a vacuum to obtain 14.2 g of a compound represented by formula (6-B).

Fourteen point two (14.2) grams of the compound represented by formula (6-B) was mixed with 0.59 g of pure water, 0.62 g of p-toluenesulfonic acid monohydrate, 40 mg of 3,5-di-tert-butyl-4-hydroxytoluene and 80 mL of tetrahydrofuran. Under an atmosphere of nitrogen, the mixture obtained was stirred at 50°C for 3 hours to effect reaction. The reaction mixture was cooled down to room temperature, and then, concentrated to remove tetrahydrofuran. To the residue, 200 mL of heptane was added. The precipitation precipitated was isolated by filtration, and washed with pure water, and then, dried in a vacuum. The powder obtained was mixed with chloroform, and the mixture obtained was filtrated through silica gel. The filtrate was dissolved in 400 mL of chloroform, and the solution obtained was concentrated. To the concentrated residue, toluene was added to concentrate under reduced pressure. To the residue, 500 mL of heptane was added to conduct crystallization. The powder obtained was isolated by filtration, and dried in vacuum to obtain 10. 1 g of a compound represented by formula (7-B) . Yield: 67% (based on the compound represented by formula (B)). According to the result of high performance liquid chromatography analysis, impurities were not detected.

### Example 6

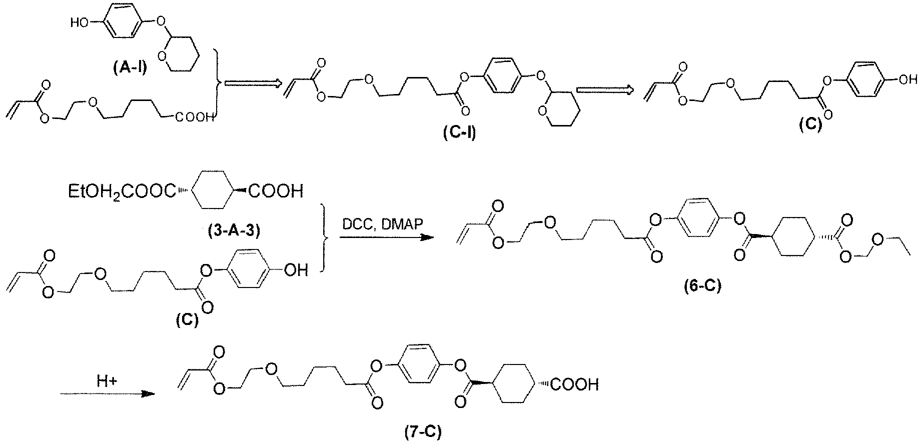

Ten point zero (10.0) grams of a compound represented by formula (A-I) was mixed with 5.14 g of dimethylaminopyridine, 10.5 g of 6-(2-acryloyloxyethoxy) caproate, 0.1 g of 3,5-di-tert-butyl-4-hydroxytoluene and 100 mL of chloroform. The solution obtained was cooled with ice under an atmosphere of nitrogen, and then, a solution obtained by dissolving 10.72 g of dicyclohexylcarbodiimide in 50 mL of chloroform was added dropwise thereto. After completion of addition, the mixture obtained was stirred at room temperature for 2 hours to effect reaction. The reaction mixture obtained was filtrated. To the filtrate obtained, 45 g of silica gel was added, and then, the mixture obtained was stirred at room temperature for 1 hour. The mixture was filtrated, and the filtrate obtained was concentrated under reduced pressure with a evaporator. The concentrated residue was mixed with tetrahydrofuran, and the mixture obtained was filtrated to remove insoluble matter. The filtrate obtained was concentrated under reduced pressure to obtain 17.55 g of a compound represented by formula (C-1) as a viscous liquid. Yield: 94% (based on the compound represented by formula (A-I)).

Fourteen point zero (14.0) grams of the compound represented by formula (C-1) was mixed with 0.70 g of pure water, 0.367 g of p-toluenesulfonic acid monohydrate, 0.1 g of 3,5-di-tert-butyl-4-hydroxytoluene and 100 mL of tetrahydrofuran. Under an atmosphere of nitrogen, the mixture obtained was stirred at 30°C, and then, further stirred at 60°C for 30 minutes. The reaction mixture was concentrated under reduced pressure below 30°C until the amount thereof became a third. The concentrated solution was mixed with 800 g of ice, and the white powder precipitated was isolated by filtration. The powder isolated was washed twice with 200 mL of pure water, and then, dried in a vacuum to obtain 9.11 g of white powder of a compound represented by formula (C) . Yield: 85% (based on the compound represented by formula (C-I)).

Eight point zero zero (8.00) grams of the compound represented by formula (C) was mixed with 0.245 g of dimethylaminopyridine, 6.62 g of a compound represented by formula (3-A-3), 0.1 g of 3,5-di-tert-butyl-4-hydroxytoluene and 80 mL of chloroform. Under an atmosphere of nitrogen, a solution obtained by dissolving 6.05 g of dicyclohexylcarbodiimide in 30 mL of chloroform was added dropwise to the mixture obtained under ice-cooling. After completion of addition, the mixture obtained was stirred at room temperature for 3 hours to effect reaction. The reaction mixture was filtrated. To the filtrate, 10 g of silica gel was added, and then, the mixture obtained was stirred at room temperature for 1 hour. The mixture was filtrated, and the filtrate obtained was concentrated under reduced pressure. The concentrated residue was mixed with 60 mL of tetrahydrofuran, and the mixture obtained was filtrated to remove insoluble matter. The filtrate obtained was concentrated under reduced pressure. The residue obtained was dried in a vacuum to obtain 13.56 g of a compound represented by formula (6-C) as a viscous liquid. Yield: 96% (based on the compound represented by formula (C)).

Thirteen point five six (13.56) grams of the compound represented by formula (6-C) was mixed with 0. 512 g of pure water, 0.631 g of p-toluenesulfonic acid monohydrate and 0.1 g of 3,5-di-tert-butyl-4-hydroxytoluene. Under an atmosphere of nitrogen, the mixture obtained was stirred at 50°C for 2 hours to effect reaction. The reaction mixture was concentrated below 30°C under reduced pressure. To the concentrated residue, 400 g of ice was added. The white crystal precipitated was isolated by filtration. The crystal isolated was washed twice with 300 mL of pure water, and then, further washed three times with a mixed solution of 100 mL of pure water and 50 mL of methanol. The crystal was dried in a vacuum to obtain 9.98 g of a compound represented by formula (7-C) . Yield: 84% (based on the compound represented by formula (C)).

### Example 7

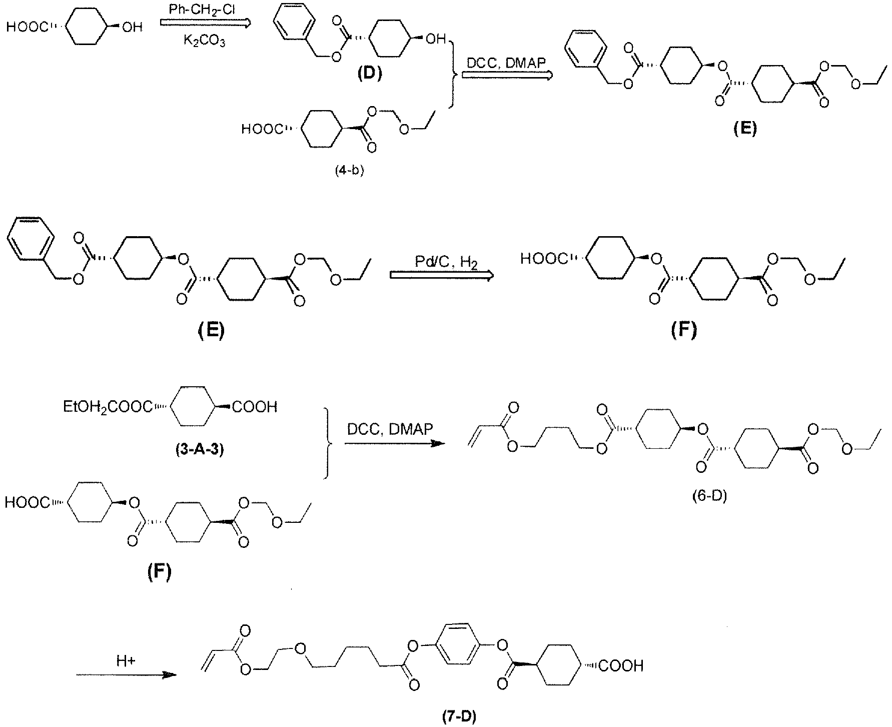

One hundred twenty five (125) grams of trans-4-hydroxycyclohexanecarboxylic acid was mixed with 143. 8 g of potassium carbonate, 140.87 g of benzyl bromide and 700 mL of N,N-dimethylacetamide. The mixture obtained was stirred at 80°C under an atmosphere of nitrogen. The reaction mixture obtained was cooled down to room temperature, and then, was poured into a mixture of 1000g of water and 500 g of methyl isobutyl ketone/heptane (weight ratio=3/2). The mixture obtained was stirred, and then, separated to an organic layer and an aqueous layer. The organic layer was washed with pure water, and then, dried over anhydrous sodium sulfate. Sodium sulfate was removed by filtration, and the filtrate obtained was concentrated. To the residue, added was heptane. The crystal precipitated was isolated by filtration, and dried in a vacuum to obtain 150 g of a compound represented by formula (D) . Yield: 75% (based on trans-4-hydroxycyclohexanecarboxylic acid).

Thirty point five (30.5) grams of the compound represented by formula (D) was mixed with 1.59 g of dimethylaminopyridine, 30 g of a compound represented by formula (4-b) and 200 mL of chloroform. The mixture obtained was cooled with ice under an atmosphere of nitrogen, and then, 29.57 g of dicyclohexylcarbodiimide was added dropwise thereto over 1 hour. After completion of addition, the mixture obtained was stirred. To the reaction mixture obtained, 200 mL of chloroform and 200 mL of heptane were added, and the precipitation was filtrated. The filtrate was washed three times with pure water, and then, dried over anhydrous sodium sulfate. Sodium sulfate was removed by filtration, and the filtrate was concentrated. To the residue, methanol was added to stir, and the powder obtained was isolated by filtration. To the powder, methanol was added to stir followed by filtration. The powder was dried in a vacuum to obtain 42 g of a compound represented by formula (E). Yield: 90% (base on the compound represented by formula (4-b)).

Twenty three (23) grams of the compound represented by formula (E) was mixed with 150 mL of tetrahydrofuran. Under an atmosphere of nitrogen, 1.2 g of 10% palladium-carbon (containing 50% water) was added thereto. The mixture obtained was stirred to effect reaction at room temperature at a normal pressure under an atmosphere of hydrogen. The reaction mixture was filtrated to remove the catalyst. The filtrate was concentrated, and the residue obtained was mixed with toluene. The mixture obtained was filtrated to remove an insoluble matter. The filtrate obtained was concentrated. The residue obtained was washed with water/methanol solution (volume ratio=1/1), and further washed with water. The crystal obtained was dried in a vacuum to obtain 17.8 g of a compound represented by formula (F). Yield: 97% (base on the compound represented by formula (E)).

Sixteen (16) grams of the compound represented by formula (F) was mixed with 0.55 g of dimethylaminopyridine, 6.47 g of 4-hydroxybutyl acrylate and 100 mL of chloroform. The mixture obtained was cooled with ice under an atmosphere of nitrogen, and a solution obtained by dissolving 10.19 g of 1-ethyl-3-(3-dimethylaminopropyl)carbodiimide hydrochloric acid salt in 50 mL of chloroform was added dropwise thereto. After completion of addition, the mixture obtained was stirred at room temperature for 2 hours to effect reaction. To the reaction mixture obtained, 200 mL of toluene was added, and the precipitation was filtrated. The filtrate was concentrated under reduced pressure to remove chloroform to obtain a toluene solution. The toluene solution obtained was washed three times with 1 N hydrochloric acid, and then, dried over anhydrous sodium sulfate. After filtration, the filtrate was concentrated, and the concentrated residue obtained was dried in a vacuum to obtain 18.5 g of a compound represented by formula (6-D).

Eighteen point five (18.5) grams of the compound represented by formula (6-D) was mixed with 0.97 g of pure water, 0.85 g of p-toluenesolfonic acid monohydrate and 100 mL of tetrahydrofuran. The mixture obtained was stirred at 50°C for 3 hours under an atmosphere of nitrogen to effect reaction. The reaction mixture was cooled down to room temperature, and then, concentrated. To the residue obtained, 200 mL of heptane, and the precipitation precipitated was isolated by filtration. The precipitation was washed with pure water, and then, dried in a vacuum to obtain 15. 4 g of a compound represented by formula (7-D) . Yield: 81% (base on the compound represented by formula (F)).

### Comparative Example 1

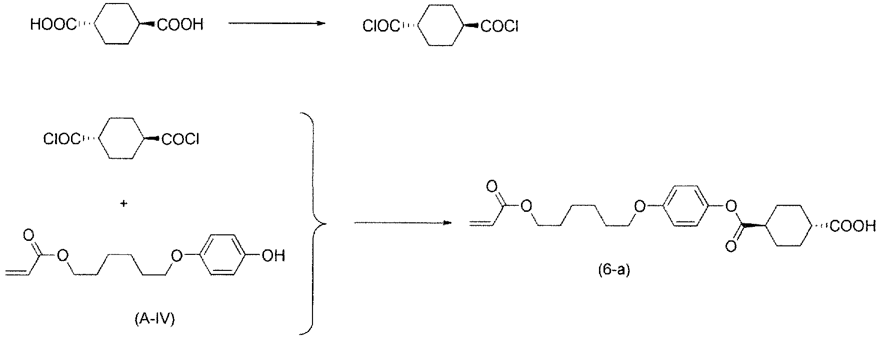

Twenty four point six eight (24.68) grams of trans-1,4-cyclohexanedicarboxylic acid was mixed with toluene. To the solution obtained, 74.61 g of oxalyl dichloride and 0.5 mL of N,N-dimethylformamide were added. The solution obtained was stirred under an atmosphere of nitrogen to effect reaction. The reaction mixture obtained was concentrated under reduced pressure to remove toluene and unreacted oxalyl dichloride. The solution obtained was mixed with chloroform to obtain a solution containing trans-1,4-cyclohexanedicarboxylic dichloride.

Twelve (12) grams of a compound represented by formula (A) was mixed with chloroform. The solution obtained and 12.6 g of pyridine were added dropwise under ice-cooling to the solution containing trans-1,4-cyclohexanedicarboxylic dichloride which was previously obtained. The mixture obtained was stirred under an atmosphere of nitrogen. The precipitation was removed by filtration, and the filtrate obtained was concentrated under reduced pressure. The concentrated liquid was added dropwise to water/methanol solution (volume ratio=1/1). The precipitation generated was crushed, and then, filtrated. The precipitation was washed with pure water. The precipitation was filtrated and dried in a vacuum. The powder obtained was crushed, and then, heptane was added thereto. The mixture obtained was stirred, and then, the precipitation was isolated. The precipitation was mixed with toluene, and an insoluble matter was removed by filtration. The filtrate was concentrated under reduced pressure, and heptane was added to the concentrated liquid obtained. The precipitation was isolated by filtration, and dried in a vacuum to obtain 7.8 g of powder containing a compound represented by formula (7-A). Yield: 40% (based on the compound represented by formula (A)). Its purity was 70%.

According to the results of high performance liquid chromatography and mass spectroscopy analysis, it was found that the powder obtained contained impurities represented by the following formulae (III) to (X) in addition to the compound represented by formula (7-A).

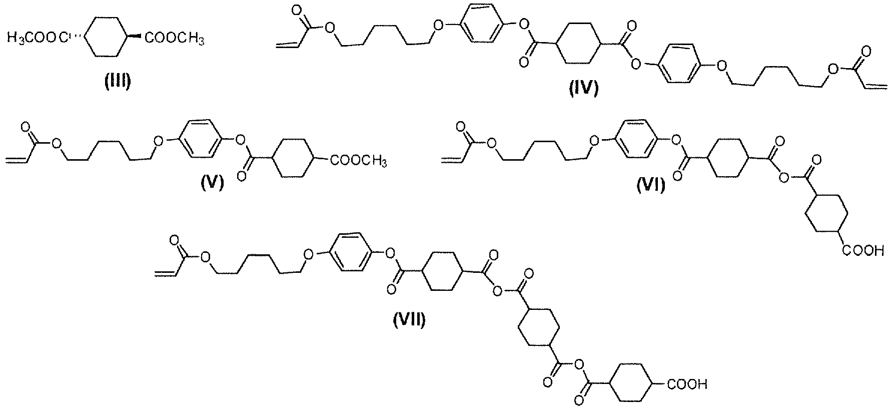

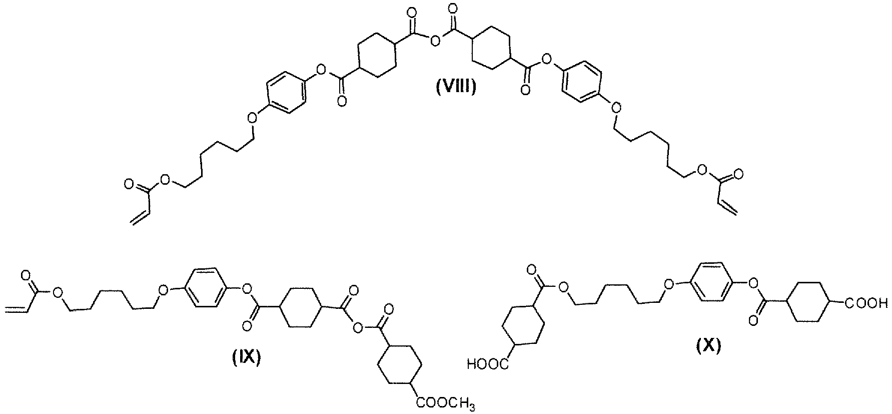

### Comparative Example 2

Twenty four point six eight (24.68) grams of trans-1,4-cyclohexanedicarboxylic acid was mixed with 74.91 g of oxalyl dichloride and 0.5 mL of N,N-dimethylformamide. The solution obtained was stirred under an atmosphere of nitrogen to effect reaction. The reaction mixture obtained was concentrated under reduced pressure to remove toluene and unreacted oxalyl dichloride. The solution obtained was mixed with chloroform to obtain a solution containing trans-1,4-cyclohexanedicarboxylic dichloride.

Twelve (12) grams of a compound represented by formula (A) was mixed with chloroform. The solution obtained and 12.6 g of pyridine were added dropwise under ice-cooling to the solution containing trans-1,4-cyclohexanedicarboxylic dichloride which was previously obtained. The solution obtained was stirred under an atmosphere of nitrogen. The precipitation was removed by filtration, and the filtrate was concentrated under reduced pressure. The concentrated liquid was added dropwise to water. The precipitation generated was crushed, and then, filtrated. The precipitation was mixed with pure water to filtrate. The precipitation was dried in a vacuum. The powder obtained was crushed, and then, added to water/methanol solution (volume ratio=1/1). The precipitation was crushed, and then, filtrated. To the precipitation, heptane was added. The mixture obtained was stirred, and then, the insoluble matter was isolated by filtration. The insoluble matter was mixed with toluene to filtrate. The filtrate obtained was concentrated under reduced pressure, and heptane was added to the concentrated liquid obtained. The precipitation was isolated by filtration, and dried in a vacuum to obtain 2.1 g of a compound represented by formula (7-A). Yield: 12% (based on the compound represented by formula (A)). Its purity was 85%.

### Example 8

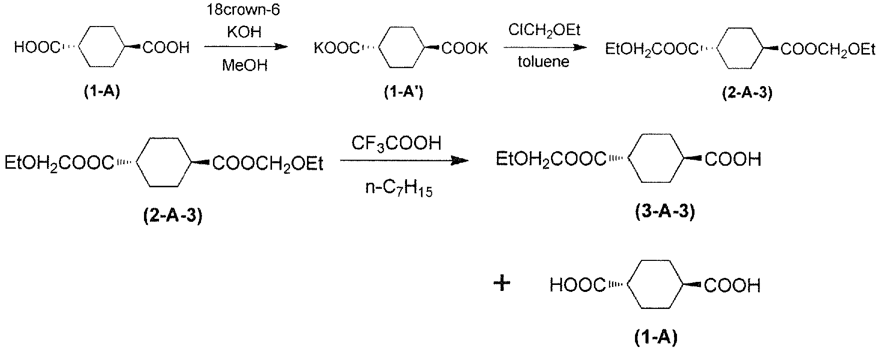

### <First step>

Nine point seven eight (9.78) grams of potassium carbonate was mixed with 0.46 g of 18-crown-6 and 30 g of methanol. The mixture obtained was cooled with ice with stirring under an atmosphere of nitrogen, and 10 g of a compound represented by formula (1-A) (trans-1,4-cyclohexanedicarboxylix acid) was added thereto. The mixture obtained was stirred at room temperature for 8 hours. To the reaction mixture obtained, 30 g of toluene was added, and the precipitation was isolated by filtration. The precipitation was washed with toluene to obtain a salt represented by formula (1-A'). The obtained salt represented by formula (1-A') was mixed with 50 g of toluene, and 16.06 g of potassium carbonate was further added thereto. To the mixture obtained, 10.98 g of ethoxychoromethane was added dropwise with checking pH of the reaction mixture was maintained 7 or more. The reaction mixture obtained was stirred at room temperature for 3 hours, and then, the white precipitation precipitated was removed by filtration. The filtrate obtained was concentrated under reduced pressure, and 20 g of ice and 80 mL of pure water were added to the residue. The white crystal precipitated was isolated by filtration. The crystal was further washed twice with pure water, and then, dried in a vacuum to obtain 14.7 g of a compound represented by formula (2-A-3). Yield: 88% (based on the compound represented by formula (1-A)).

### <Second step>

A compound represented by formula (3-A-3) can be obtained according to the same manner as those of the above-mentioned <Second step> of Example 1 using the compound represented by formula (2-A-3) obtained in the above.

### Example 9

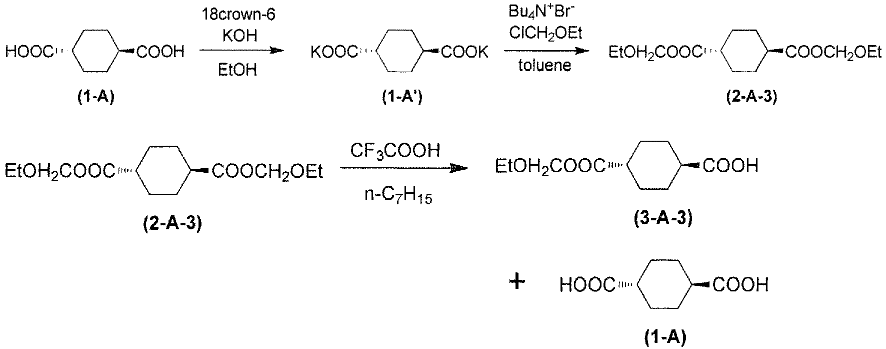

### <First step>

Eight point one five (8.15) grams of potassium carbonate was mixed with 0.46 g of 18-crown-6 and 50 g of ethanol. The mixture obtained was cooled with ice with stirring under an atmosphere of nitrogen, and 10 g of a compound represented by formula (1-A) (trans-1,4-cyclohexanedicarboxylix acid) was added thereto. The mixture obtained was stirred at room temperature for 8 hours. To the reaction mixture obtained, 50 g of tetrahydrofuran was added, and the precipitation was isolated by filtration. The precipitation was washed with toluene and tetrahydrofuran to obtain a salt represented by formula (1-A'). The obtained salt represented by formula (1-A') was mixed with 50 g of toluene, and 16.06 g of potassium carbonate and 1.0 g of tetrabutylammonium bromide were further added thereto. To the mixture obtained, 10.98 g of ethoxychoromethane was added dropwise with checking pH of the reaction mixture was maintained 7 or more. The reaction mixture obtained was stirred at room temperature for 3 hours, and then, the white precipitation precipitated was removed by filtration. The filtrate obtained was concentrated under reduced pressure, and 20 g of ice and 80 mL of pure water were added to the residue. The white crystal precipitated was isolated by filtration. The crystal was further washed twice with pure water, and then, dried in a vacuum to obtain 15.6 g of a compound represented by formula (2-A-3). Yield: 93% (based on the compound represented by formula (1-A)).

### <Second step>

A compound represented by formula (3-A-3) can be obtained according to the same manner as those of the above-mentioned <Second step> of Example 1 using the compound represented by formula (2-A-3) obtained in the above.

### Example 10

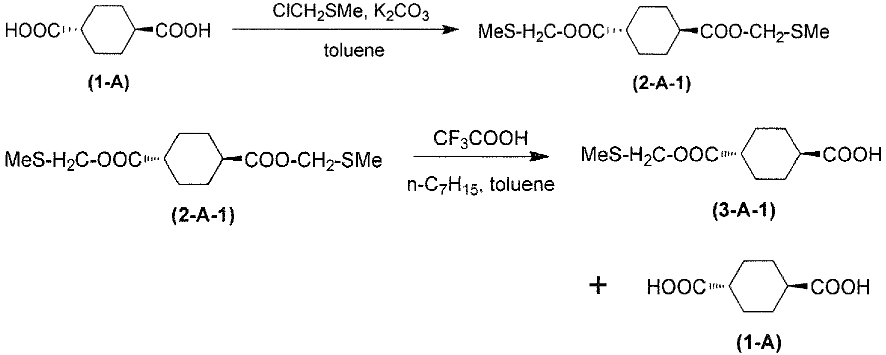

### <First step>

Ten (10) grams of a compound represented by formula (1-A) (trans-1,4-cyclohexanedicarboxylix acid) was mixed with 16.05 g of potassium carbonate, 2.70 g of potassium iodide, 4.3 g of 18-crown-6 and 50 g of toluene. The mixture obtained was heated up to 60°C with stirring under an atmosphere of nitrogen, and 13. 46 g of methylthiomethyl chloride was added dropwise thereto. The mixture obtained was stirred at 60°C for 7 hours. The reaction mixture obtained was cooled down to room temperature, and then, 300 mL of pure water was added to separate to an organic layer and an aqueous layer. The organic layer was washed twice with 100 mL of pure water, and then, dried over anhydrous sodium sulfate. Sodium sulfate was filtrated, and then, the filtrate obtained was concentrated under reduced pressure. The concentrated residue was cooled at 0°C, and then, 40 mL of heptane was added thereto. The mixture obtained was stirred at 0°C for 15 minutes, and then, the white crystal precipitated was isolated by filtration. The white crystal isolated was dried in a vacuum to obtain 13.5 g of a compound represented by formula (2-A-1). Yield: 79% (based on the compound represented by formula (1-A)).
¹H-NMR of the compound represented by formula (2-A-1) (CDCl₃): δ (ppm) 1.41-1.58 (m, 4H), 2.04-2.14 (br, d, 4H), 2.24 (s, 6H), 2.27-2.38 (m, 2H), 5.13 (s, 4H)

### <Second step>

A compound represented by formula (3-A-1) and a compound represented by formula (1-A-1) were obtained in yields of 48% (based on the compound represented by formula (2-A-1)) and of 25% (based on the compound represented by formula (2-A-1)), respectively, according to the same manner as those of <Second step> of Example 1 except that the compound represented by formula (2-A-1) obtained in the above was used in place of the compound represented by formula (2-A-3), the reaction temperature was changed to 60°C, and a mixed solvent of heptane and toluene (heptane/toluene weight ratio=80/20) was used as a solvent. Additionally, the unreacted compound represented by formula (2-A-1) was recovered at a recovery rate of 27%.
¹H-NMR of the compound represented by formula (3-A-1) (CDCl₃): δ (ppm) 1.40-1.59 (m, 4H), 2.03-2.14 (br, d, 4H), 2.25 (s, 3H), 2.25-2.36 (m, 2H), 5.12 (s, 2H)

### Example 11

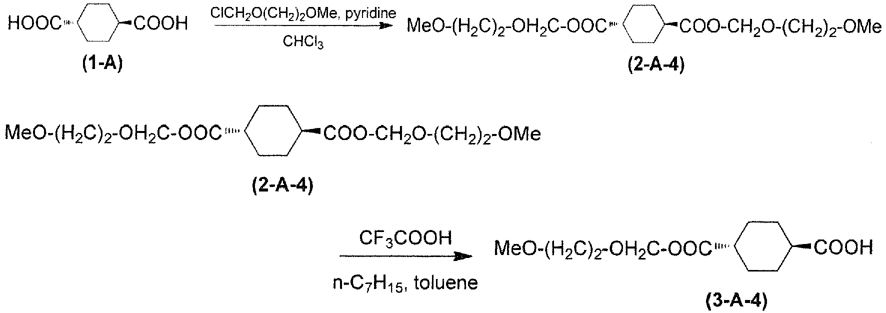

### <First step>

Seven (7) grams of a compound represented by formula (1-A) (trans-1,4-cyclohexanedicarboxylix acid) was mixed with 35 mL of chloroform. The mixture obtained was cooled with ice with stirring under an atmosphere of nitrogen, and then, 12.34 g of triethylamine was added thereto. The mixture obtained was stirred to obtain a homogeneous solution. Twelve point one five (12.15) grams of methoxyethoxychloromethane and 4.08 g of triethylamine were added dropwise to the solution with stirring and maintaining pH 7 or more. The solution obtained was stirred for 5 hours under ice-cooling. The white precipitation precipitated was removed by filtration. The filtrate obtained was washed twice with 500 mL of pure water, and then, concentrated unde reduced pressure with an evaporator. To the concentrated residue, heptane was added, and the mixture obtained was concentrated under reduced pressure to obtain 12.6 g of a compound represented by formula (2-A-4). Yield: 89% (based on the compound represented by formula (1-A)).
¹H-NMR of the compound represented by formula (2-A-4) (CDCl₃): δ (ppm) 1.41-1.56 (m, 4H), 2.08-2.11 (d, 4H), 2.33 (br, 2H), 3.39 (s, 6H), 3.54-3.58 (m, 4H), 3.76-3.80 (m, 4H), 5.33 (s, 4H)

### <Second step>

A compound represented by formula (3-A-4) and a compound represented by formula (1-A-1) were obtained in yields of 42% (based on the compound represented by formula (2-A-4)) and of 35% (based on the compound represented by formula (2-A-4)), respectively, according to the same manner as those of <Second step> of Example 1 except that the compound represented by formula (2-A-4) obtained in the above was used in place of the compound represented by formula (2-A-3), and a mixed solvent of heptane and toluene (heptane/toluene weight ratio=80/20) was used as a solvent. Additionally, the unreacted compound represented by formula (2-A-1) was recovered at a recovery rate of 23%.
¹H-NMR of the compound represented by formula (3-A-4) (CDCl₃): δ (ppm) 1.41-1.56 (dt, 4H), 2.08-2.11 (d, 4H), 2.28 (br, 2H), 3.38 (s, 3H), 3.56-3.58 (m, 2H), 3.76-3.81 (m, 2H), 5.35 (s, 2H)

### Industrial Applicability

According to the present invention, cycloalkanedicarboxylic acid monoesters can be obtained in a good yield.

## Claims

1. A process for preparation of cycloalkanedicarboxylic acid monoesters which is **characterized by** comprising: a first step of protecting two carboxyl groups (-COOH) of a compound represented by formula (1-A) with a group represented by Z to obtain a compound represented by formula (2-A), and a second step of deprotecting either of the groups represented by Z of the compound represented by formula (2-A) obtained in the first step with an acid to obtain a compound represented by formula (3-A). 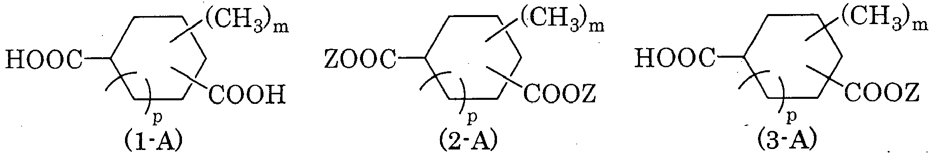 wherein m represents an integer of 0 to 3, p represents 0 or 1, Z represents a methylsulfanylmethyl group (-CH₂-SCH₃) a methoxymethyl group, an ethoxymethyl group, a tert-butoxymethyl group, a (4-pentenyloxy)methyl group, a (2-methoxyethoxy)methyl group, a 1-ethoxyethyl group, a benzyloxymethyl group, a 4-methoxybenzyloxymethyl group, a 2-methoxybenzyloxymethyl group, a 4-nitrobenzyloxymethyl group, a 1-methyl-1-benzyloxy-2-fluoroethyl group, a 1-methyl-1-phenoxyethyl group, a 1-methyl-1-methoxyethyl group, a 1-methyl-1-benzyloxyethyl group, a 2,2,2-trichloroethoxymethyl group, a 1-[2-(trimethylsilyl)ethoxy]ethyl group, a tetrahydropyranyl group, a 3-bromotetrahydropyranyl group, a tetrahydrothiopyranyl group, a tetrahydrofuranyl group, a tetrahydrothiofuranyl group, a tert-butyl group, a trityl group, a benzyl group, a 4-methoxybenzyl group, a 3,4-dimethoxybenzyl group, a 4-nitrobenzyl group, a 1,3-benzodithiolan-2-yl group, a 2,2,2-trichloroethyl group, a 2-phenyl-2-ethanon-1-yl group, a cyclopropylmethyl group, -CH₂-O-Si-R⁵R⁶R⁷ or -SiR⁵R⁶R⁷, and R⁵, R⁶ and R⁷ each independently represent a hydrogen atom, an alkyl group having 1 to 8 carbon atoms, an alkoxy group having 1 to 8 carbon atoms, a phenyl group or a benzyl group.

2. The process according to claim 1, wherein the deprotection in the second step is carried out in a solvent containing an aliphatic hydrocarbon.

3. The process according to claim 2, wherein the content of the aliphatic hydrocarbon in the solvent containing the aliphatic hydrocarbon is a solvent containing in 30% by mass or more and 100% by mass or less.

4. The process according to claim 2, wherein the aliphatic hydrocarbon is at least one selected from the group consisting of pentane, hexane, cyclohexane, heptane and octane.

5. The process according to claim 1, wherein the acid is a Brønsted acid.

6. The process according to claim 1, wherein the acid is trifluoroacetic acid or trichloroacetic acid.

7. The process according to claim 1, wherein the amount of the acid is 0.1 mole or more and 3 moles or less per 1 mole of the compound represented by formula (2-A).

8. The process according to claim 1, wherein the process further contains a step of recovering the compound represented by formula (1-A) which has been produced as impurities in the second step, and the compound represented by formula (1-A) which has been recovered in the step is reused in the first step.

9. The process according to claim 1, wherein the process further contains a step of recovering the compound represented by formula (2-A) which has been unreacted in the second step, and the compound represented by formula (2-A) which has been recovered in the step is reused in the second step.

10. The process according to claim 1, wherein the first step is a step of reacting the compound represented by formula (1-A) with a compound represented by formula (4-A) or (4-B) to obtain the compound represented by formula (2-A), 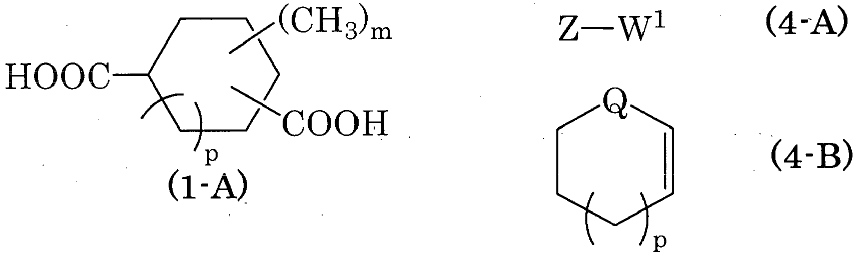 wherein Z, p and m are the same as defined above, W¹ represents a halogen atom, a tosyl group or a mesityl group, Q represents -0- or -S-, and q represents 0 or 1.

11. The process according to claim 1, wherein the group represented by Z is a methylsulfanylmethyl group (-CH₂-SCH₃), a methoxymethyl group, an ethoxymethyl group, a (2-methoxyethoxy)methyl group, a 1-ethoxyethyl group, a tertbutyldimethylsilyl group, a tert-butyldiphenylsilyl group, a triisopropylsilyl group, a tetrahydropyranyl group or a tetrahydrothiopyranyl group.

12. The process according to claim 1, wherein the compound represented by formula (1-A) is a compound represented by formula (1-B), the compound represented by formula (2-A) is a compound represented by formula (2-B), and the compound represented by formula (3-A) is a compound represented by formula (3-B), 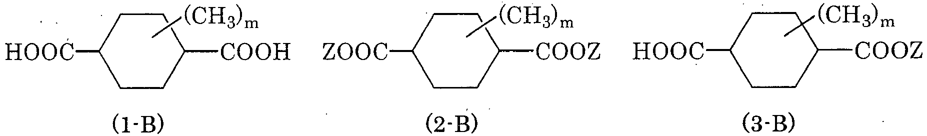 wherein m and Z are the same as defined above.

13. The process according to claim 1, wherein m is 0.

14. The process according to claim 1, wherein the compound represented by formula (3-A) is a trans-1,4-cyclohexanedicarboxylic acid monoester.

15. A process for producing a polymerizable compound comprising producing the compound represented by formula (3-A) according to the process of claim 1, and further comprising a third step of reacting the compound represented by formula (3-A) with a compound represented by formula- (5) to obtain a compound represented by formula (6), and a fourth step of deprotecting the group represented by Z of the compound represented by formula (6) which has been obtained in the third step to obtain a polymerizable compound represented by formula (7), 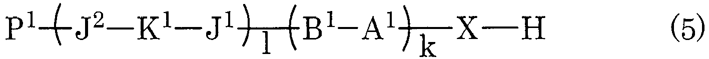 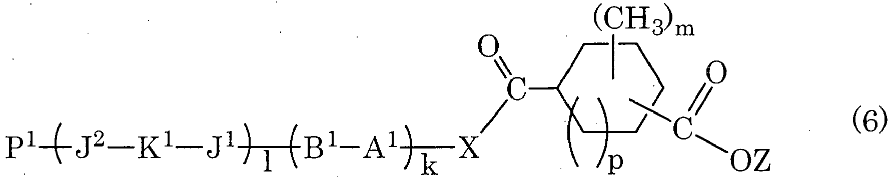 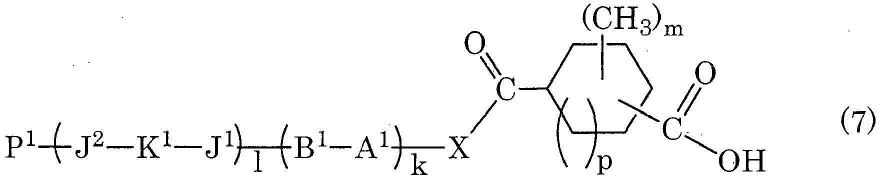 wherein m, Z and p are the same as defined above, X represents -O-, -S- or -N(R¹⁷)-,
A¹ is independently in each occurrence a divalent alicyclic hydrocarbon group having 3 to 10 carbon atoms or a divalent aromatic hydrocarbon group having 6 to 20 carbon atoms, a hydrogen atom contained in the alicyclic hydrocarbon group or the aromatic hydrocarbon group may be replaced by a halogen atom, an alkyl group having 1 to 4 carbon atoms and optionally substituted with a halogen atom, an alkoxy group having 1 to 4 carbon atoms and optionally substituted with a halogen atom, a cyano group or a nitro group, -CH₂- contained in the alicyclic hydrocarbon group may be replaced by -0-, -S- or -N(R¹⁷)-, -CH(-)- contained in the alicyclic hydrocarbon group may be replaced by -N(-)-,
B¹ is independently in each occurrence -CR¹⁵R¹⁶-, -CH₂-CH₂-, -O-, -S-, -C(=O)-O-, -O-C(=O)-, -O-C(=O)-O-, -C(=S)-O-, -O-C(=S)-, -O-C(=S)-O-, -C(=O)-N(R¹⁷)-, -N(R¹⁷)-C(=O)-, -OCH₂-, -CH₂O-, -SCH₂-, -CH₂S-, -C(=S)-O-, -O-C(=S)-, -C(=O)-S-, -S-C(=O)-, -(C=S)-S-, -S-C(=S)-, -NH-C(=O)-O-, =O-C(=O)-NH-, -NH-C(=O)-NH-, -N(R¹⁷)-, -S(=O)*-,* -O-S(=O)₂-O-, -N=N- or a single bond,
R¹⁵ and R¹⁶ each independently represent a hydrogen atom, a fluorine atom or an alkyl group having 1 to 4 carbon atoms, R¹⁷ represents a hydrogen atom or an alkyl group having 1 to 4 carbon atoms,
J¹ and J² each independently represent an alkanediyl group having 1 to 18 carbon atoms, and a hydrogen atom contained in the alkanediyl group may be replaced by an alkoxy group having 1 to 5 carbon atoms or a halogen atom,
K¹ is independently in each occurrence -0-, -C(=O)-O-, -0-C(=O)-, -C(=S)-O-, -O-C(=S)-, -C(=O)-S-, -S-C(=O)-, -(C=S)-S-, -S-C(=S)-, -NH-C(=O)-, -C(=O)-NH-, -NH-C(=O)-O-, -O-C(=O)-NH-, -NH-C(=O)-NH-, -O-C(=O)-O-, -N(R¹⁷)-, -S (=O) -, -O-S(=O)₂-O-, or a single bond,
k and 1 each independently represent an integer of 1 to 3, and P¹ represents a polymerizable group.

16. The process according to claim 15, wherein P¹ is an acryloyloxy group or a methacryloyloxy group.

17. The process according to claim 15, wherein the fourth step is a step of deprotecting the group represented by Z of the compound represented by formula (6) which has been obtained in the third step with an acid.

18. The process according to claim 17, wherein the acid is at least one selected from the group consisting of formic acid, acetic acid, propionic acid, hydrochloric acid, hydrobromic acid, hydrogen iodide, nitric acid, sulfuric acid, p-toluenesulfonic acid, pyridinium p-toluenesulfonate, trifluoroacetic acid, trichloroacetic acid and methanesulfonic acid.

## Patentansprüche

1. Ein Verfahren zur Herstellung von Cycloalkandicarbonsäuremonoestern, welches **dadurch gekennzeichnet ist, dass** es umfasst: einen ersten Schritt des Schützens von zwei Carboxylgruppen (-COOH) einer Verbindung dargestellt durch Formel (1-A) mit einer durch Z dargestellten Gruppe, um eine Verbindung dargestellt durch Formel (2-A) zu erhalten, und einen zweiten Schritt des Entfernens einer durch Z dargestellten Schutzgruppe von der in dem ersten Schritt erhaltenen Verbindung dargestellt durch Formel (2-A) mit einer Säure, um eine Verbindung, dargestellt durch Formel (3-A), zu erhalten, 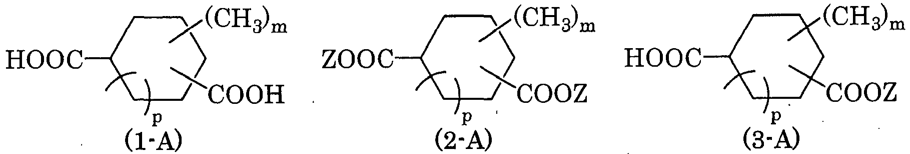 wobei m eine ganze Zahl von 0 bis 3 bedeutet, p 0 oder 1 bedeutet, Z eine Methylsulfanylmethylgruppe (-CH₂-SCH₃), eine Methoxymethylgruppe, eine Ethoxymethylgruppe, eine tert-Butoxymethylgruppe, eine (4-Pentenyloxy)methylgruppe, eine (2-Methoxyethoxy)methylgruppe, eine 1-Ethoxyethylgruppe, eine Benzyloxymethylgruppe, eine 4-Methoxybenzyloxymethylgruppe, eine 2-Methoxybenzyloxymethylgruppe, eine 4-Nitrobenzyloxymethylgruppe, eine 1-Methyl-1-benzyloxy-2-fluorethylgruppe, eine 1-Methyl-1-phenoxyethylgruppe, eine 1-Methyl-1-methoxyethylgruppe, eine 1-Methyl-1-benzyloxyethylgruppe, eine 2,2,2-Trichlorethoxymethylgruppe, eine 1-[2-(Trimethylsilyl)ethoxy]ethylgruppe, eine Tetrahydropyranylgruppe, eine 3-Bromtetrahydropyranylgruppe, eine Tetrahydrothiopyranylgruppe, eine Tetrahydrofuranylgruppe, eine Tetrahydrothiofuranylgruppe, eine tert-Butylgruppe, eine Tritylgruppe, eine Benzylgruppe, eine 4-Methoxybenzylgruppe, eine 3,4-Dimethoxybenzylgruppe, eine 4-Nitrobenzylgruppe, eine 1,3-Benzodithiolan-2-ylgruppe, eine 2,2,2-Trichlorethylgruppe, eine 2-Phenyl-2-ethanon-1-ylgruppe, eine Cyclopropylmethylgruppe, -CH₂-O-Si-R⁵R⁶R⁷ oder -SiR⁵R⁶R⁷ bedeutet und R⁵, R⁶ und R⁷ jeweils unabhängig ein Wasserstoffatom, eine Alkylgruppe mit 1 bis 8 Kohlenstoffatomen, eine Alkoxygruppe mit 1 bis 8 Kohlenstoffatomen, eine Phenylgruppe oder eine Benzylgruppe bedeuten.

2. Das Verfahren nach Anspruch 1, wobei das Entfernen der Schutzgruppen in dem zweiten Schritt in einem Lösungsmittel erfolgt, das einen aliphatischen Kohlenwasserstoff enthält.

3. Das Verfahren nach Anspruch 2, wobei der Gehalt des aliphatischen Kohlenwasserstoffs in dem Lösungsmittel, das den aliphatischen Kohlenwasserstoff enthält, 30 Massen-% oder mehr und 100 Massen-% oder weniger beträgt.

4. Das Verfahren nach Anspruch 2, wobei der aliphatische Kohlenwasserstoff mindestens einer, ausgewählt aus der Gruppe bestehend aus Pentan, Hexan, Cyclohexan, Heptan und Octan, ist.

5. Das Verfahren nach Anspruch 1, wobei die Säure eine Brönsted-Säure ist.

6. Das Verfahren nach Anspruch 1, wobei die Säure Trifluoressigsäure oder Trichloressigsäure ist.

7. Das Verfahren nach Anspruch 1, wobei die Menge der Säure 0,1 Mol oder mehr und 3 Mol oder weniger pro 1 Mol der Verbindung, dargestellt durch Formel (2-A), beträgt.

8. Das Verfahren nach Anspruch 1, wobei das Verfahren weiter einen Schritt des Gewinnens der Verbindung dargestellt durch Formel (1-A), die als Verunreinigungen gebildet wurde, in dem zweiten Schritt beinhaltet und die in dem Schritt gewonnene Verbindung dargestellt durch Formel (1-A) in dem ersten Schritt wiederverwendet wird.

9. Das Verfahren nach Anspruch 1, wobei das Verfahren weiter einen Schritt des Gewinnens der Verbindung dargestellt durch Formel (2-A), die in dem zweiten Schritt nicht umgesetzt wurde, beinhaltet, und die in dem Schritt gewonnene Verbindung dargestellt durch Formel (2-A) in dem zweiten Schritt wiederverwendet wird.

10. Das Verfahren nach Anspruch 1, wobei der erste Schritt ein Schritt des Umsetzens der Verbindung dargestellt durch Formel (1-A) mit einer Verbindung dargestellt durch Formel (4-A) oder (4-B) ist, um die Verbindung dargestellt durch Formel (2-A) zu erhalten, 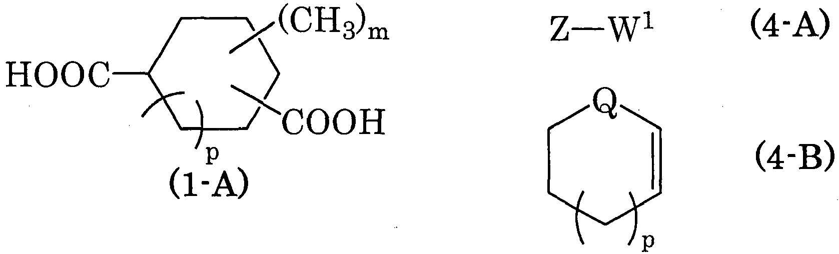 wobei Z, p und m wie vorstehend definiert sind, W¹ ein Halogenatom, eine Tosylgruppe oder eine Mesitylgruppe bedeutet, Q -O- oder -S- bedeutet und q 0 oder 1 bedeutet.

11. Das Verfahren nach Anspruch 1, wobei die durch Z dargestellte Gruppe eine Methylsulfanylmethylgruppe (-CH₂-SCH₃), eine Methoxymethylgruppe, eine Ethoxymethylgruppe, eine (2-Methoxyethoxy)methylgruppe, eine 1-Ethoxyethylgruppe, eine tert-Butyldimethylsilylgruppe, eine tert-Butyldiphenylsilylgruppe, eine Triisopropylsilylgruppe, eine Tetrahydropyranylgruppe oder eine Tetrahydrothiopyranylgruppe ist.

12. Das Verfahren nach Anspruch 1, wobei die Verbindung dargestellt durch Formel (1-A) eine Verbindung dargestellt durch Formel (1-B) ist, die Verbindung dargestellt durch Formel (2-A) eine Verbindung dargestellt durch Formel (2-B) ist, und die Verbindung dargestellt durch Formel (3-A) eine Verbindung dargestellt durch Formel (3-B) ist, 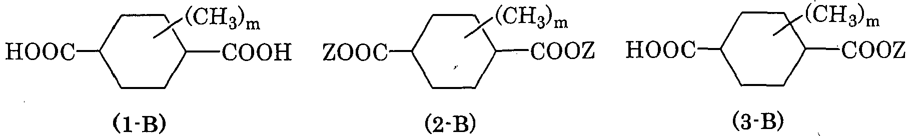 wobei m und Z wie vorstehend definiert sind.

13. Das Verfahren nach Anspruch 1, wobei m gleich 0 ist.

14. Das Verfahren nach Anspruch 1, wobei die Verbindung dargestellt durch Formel (3-A) ein trans-1,4-Cyclohexandicarbonsäuremonoester ist.

15. Ein Verfahren zur Herstellung einer polymerisierbaren Verbindung, umfassend Herstellen der Verbindung dargestellt durch Formel (3-A) nach dem Verfahren von Anspruch 1, und weiter umfassend einen dritten Schritt des Umsetzens der Verbindung dargestellt durch Formel (3-A) mit einer Verbindung dargestellt durch Formel (5), um eine Verbindung dargestellt durch Formel (6) zu erhalten, und einen vierten Schritt des Entfernens der durch Z dargestellten Schutzgruppe von der Verbindung dargestellt durch Formel (6), die in dem dritten Schritt erhalten wurde, um eine polymerisierbare Verbindung dargestellt durch Formel (7) zu erhalten, 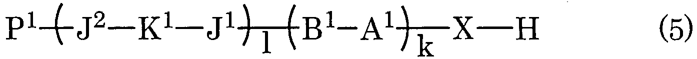 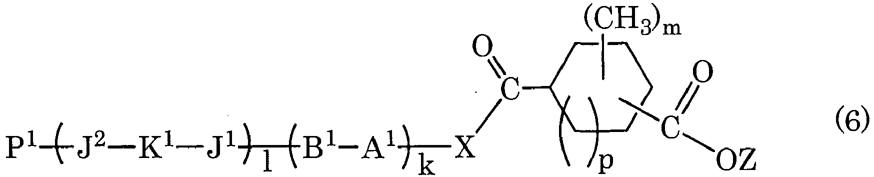 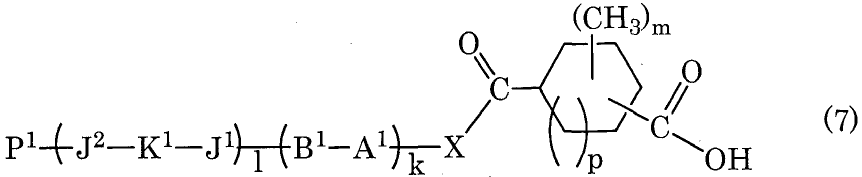 wobei m, Z und p wie vorstehend definiert sind, X -O-, -S- oder -N(R¹⁷)- bedeutet,
A¹ unabhängig bei jedem Auftreten eine zweiwertige alicyclische Kohlenwasserstoffgruppe mit 3 bis 10 Kohlenstoffatomen oder eine zweiwertige aromatische Kohlenwasserstoffgruppe mit 6 bis 20 Kohlenstoffatomen ist, ein in der alicyclischen Kohlenwasserstoffgruppe oder der aromatischen Kohlenwasserstoffgruppe enthaltenes Wasserstoffatom ersetzt sein kann durch ein Halogenatom, eine Alkylgruppe mit 1 bis 4 Kohlenstoffatomen und gegebenenfalls substituiert mit einem Halogenatom, eine Alkoxygruppe mit 1 bis 4 Kohlenstoffatomen und gegebenenfalls substituiert mit einem Halogenatom, eine Cyanogruppe oder eine Nitrogruppe, in der alicyclischen Kohlenwasserstoffgruppe enthaltenes -CH₂- ersetzt sein kann durch -O-, -S- oder -N(R¹⁷)-, in der alicyclischen Kohlenwasserstoffgruppe enthaltenes -CH(-)- ersetzt sein kann durch -N(-)-,
B¹ unabhängig bei jedem Auftreten -CR¹⁵R¹⁶-, -CH₂-CH₂-, -O-, -S-, -C(=O)-O-, -O-C(=O)-, -O-C(=O)-O-, -C(=S)-O-, -O-C(=S)-, -O-C(=S)-O-, -C(=O)-N(R¹⁷)-, -N(R¹⁷)-C(=O)-, -OCH₂-, -CH₂O-, -SCH₂-, -CH₂S-, -C(=S)-O-, -O-C(=S)-, -C(=O)-S-, -S-C(=O)-, -(C=S)-S-, -S-C(=S)-, -NH-C(=O)-O-, -O-C(=O)-NH-, -NH-C(=O)-NH-, -N(R¹⁷)-, -S(=O)-, -O-S(=O)₂-O-, -N=N- oder eine Einfachbindung ist,
R¹⁵ und R¹⁶ jeweils unabhängig ein Wasserstoffatom, ein Fluoratom oder eine Alkylgruppe mit 1 bis 4 Kohlenstoffatomen bedeuten, R¹⁷ ein Wasserstoffatom oder eine Alkylgruppe mit 1 bis 4 Kohlenstoffatomen bedeutet,
J¹ und J² jeweils unabhängig eine Alkandiylgruppe mit 1 bis 18 Kohlenstoffatomen bedeuten und ein in der Alkandiylgruppe enthaltenes Wasserstoffatom ersetzt sein kann durch eine Alkoxygruppe mit 1 bis 5 Kohlenstoffatomen oder ein Halogenatom,
K¹ unabhängig bei jedem Auftreten -O-, -C(=O)-O-, -O-C(=O)-, -C(=S)-O-, -O-C(=S)-, -C(=O)-S-, -S-C(=O)-, -(C=S)-S-, -S-C(=S)-, -NH-C(=O)-, -C(=O)-NH-, -NH-C(=O)-O-, -O-C(=O)-NH-, -NH-C(=O)-NH-, -O-C(=O)-O-, -N(R¹⁷)-, -S(=O)-, -O-S(=O)₂-O- oder eine Einfachbindung ist,
k und 1 jeweils unabhängig eine ganze Zahl von 1 bis 3 bedeuten und P¹ eine polymerisierbare Gruppe bedeutet.

16. Das Verfahren nach Anspruch 15, wobei P¹ eine Acryloyloxygruppe oder eine Methacryloyloxygruppe ist.

17. Das Verfahren nach Anspruch 15, wobei der vierte Schritt ein Schritt des Entfernens der durch Z dargestellten Schutzgruppe von der in dem dritten Schritt erhaltenen Verbindung dargestellt durch Formel (6) mit einer Säure ist.

18. Das Verfahren nach Anspruch 17, wobei die Säure mindestens eine, ausgewählt aus der Gruppe bestehend aus Ameisensäure, Essigsäure, Propionsäure, Chlorwasserstoffsäure, Bromwasserstoffsäure, Wasserstoffiodid, Salpetersäure, Schwefelsäure, p-Toluolsulfonsäure, Pyridinium-p-toluolsulfonat, Trifluoressigsäure, Trichloressigsäure und Methansulfonsäure, ist.

## Revendications

1. Procédé de préparation de monoesters d'acides cycloalcanedicarboxyliques qui est **caractérisé en ce qu'**il comprend : une première étape de protection de deux groupes carboxyle (-COOH) d'un composé représenté par la formule (1-A) avec un groupe représenté par Z pour obtenir un composé représenté par la formule (2-A), et une seconde étape de déprotection de l'un quelconque des groupes représentés par Z du composé représenté par la formule (2-A) obtenu dans la première étape avec un acide pour obtenir un composé représenté par la formule (3-A). 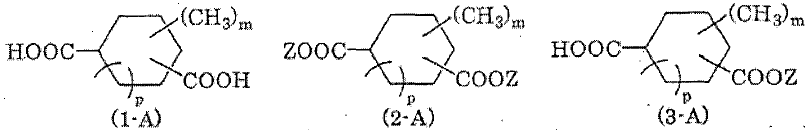 où m représente un nombre entier de 0 à 3, p représente 0 ou 1, Z représente un groupe méthylsulfanylméthyle (-CH₂-SCH₃), un groupe méthoxyméthyle, un groupe éthoxyméthyle, un groupe tert-butoxyméthyle, un groupe (4-pentényloxy)méthyle, un groupe (2-méthoxyéthoxy)méthyle, un groupe 1-éthoxyéthyle, un groupe benzyloxyméthyle, un groupe 4-méthoxybenzyloxyméthyle, un groupe 2-méthoxybenzyloxyméthyle, un groupe 4-nitrobenzyloxyméthyle, un groupe 1-méthyl-1-benzyloxy-2-fluoroéthyle, un groupe 1-méthyl-1-phénoxyéthyle, un groupe 1-méthyl-1-méthoxyéthyle, un groupe 1-méthyl-1-benzyloxyéthyle, un groupe 2,2,2-trichloroéthoxyméthyle, un groupe 1-[2-(triméthylsilyl)éthoxy]éthyle, un groupe tétrahydropyranyle, un groupe 3-bromotétrahydropyranyle, un groupe tétrahydrothiopyranyle, un groupe tétrahydrofuranyle, un groupe tétrahydrothiofuranyle, un groupe tert-butyle, un groupe trityle, un groupe benzyle, un groupe 4-méthoxybenzyle, un groupe 3,4-diméthoxybenzyle, un groupe 4-nitrobenzyle, un groupe 1,3-benzodithiolan-2-yle, un groupe 2,2,2-trichloroéthyle, un groupe 2-phényl-2-éthanon-1-yle, un groupe cyclopropylméthyle, -CH₂-O-Si-R⁵R⁶R⁷ ou -SiR⁵R⁶R⁷, et R⁵, R⁶ et R⁷ représentent chacun indépendamment un atome d'hydrogène, un groupe alkyle ayant de 1 à 8 atomes de carbone, un groupe alcoxy ayant de 1 à 8 atomes de carbone, un groupe phényle ou un groupe benzyle.

2. Procédé selon la revendication 1, dans lequel la déprotection dans la seconde étape est réalisée dans un solvant contenant un hydrocarbure aliphatique.

3. Procédé selon la revendication 2, dans lequel la teneur de l'hydrocarbure aliphatique dans le solvant contenant l'hydrocarbure aliphatique est un solvant contenant dans 30 % en masse ou plus et 100 % en masse ou moins.

4. Procédé selon la revendication 2, dans lequel l'hydrocarbure aliphatique est au moins un choisi dans le groupe constitué de pentane, hexane, cyclohexane, heptane et octane.

5. Procédé selon la revendication 1, dans lequel l'acide est un acide de Bronsted.

6. Procédé selon la revendication 1, dans lequel l'acide est l'acide trifluoroacétique ou l'acide trichloroacétique.

7. Procédé selon la revendication 1, dans lequel la quantité de l'acide est de 0,1 mole ou plus et de 3 moles ou moins pour 1 mole du composé représenté par la formule (2-A).

8. Procédé selon la revendication 1, dans lequel le procédé contient de plus une étape de récupération du composé représenté par la formule (1-A) qui a été produit comme impuretés dans la seconde étape, et le composé représenté par la formule (1-A) qui a été récupéré dans l'étape est réutilisé dans la première étape.

9. Procédé selon la revendication 1, dans lequel le procédé contient de plus une étape de récupération du composé représenté par la formule (2-A) qui n'a pas réagi dans la seconde étape, et le composé représenté par la formule (2-A) qui a été récupéré dans l'étape est réutilisé dans la seconde étape.

10. Procédé selon la revendication 1, dans lequel la première étape est une étape de réaction du composé représenté par la formule (1-A) avec un composé représenté par la formule (4-A) ou (4-B) pour obtenir le composé représenté par la formule (2-A), 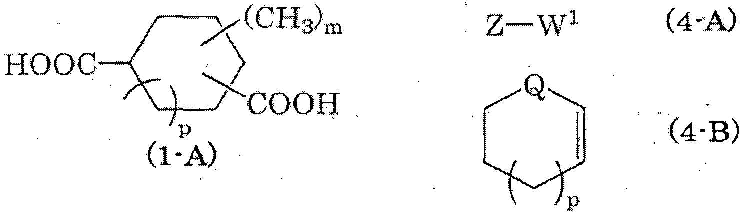 où Z, p et m sont identiques à ceux définis ci-dessus, W¹ représente un atome d'halogène, un groupe tosyle ou un groupe mésityle, Q représente -0- ou -S-, et q représente 0 ou 1.

11. Procédé selon la revendication 1, dans lequel le groupe représenté par Z est un groupe méthylsulfanylméthyle (-CH₂-SCH₃), un groupe méthoxyméthyle, un groupe éthoxyméthyle, un groupe (2-méthoxyéthoxy)méthyle, un groupe 1-éthoxyéthyle, un groupe tert-butyldiméthylsilyle, un groupe tert-butyldiphénylsilyle, un groupe triisopropylsilyle, un groupe tétrahydropyranyle ou un groupe tétrahydrothiopyranyle.

12. Procédé selon la revendication 1, dans lequel le composé représenté par la formule (1-A) est un composé représenté par la formule (1-B), le composé représenté par la formule (2-A) est un composé représenté par la formule (2-B), et le composé représenté par la formule (3-A) est un composé représenté par la formule (3-B). 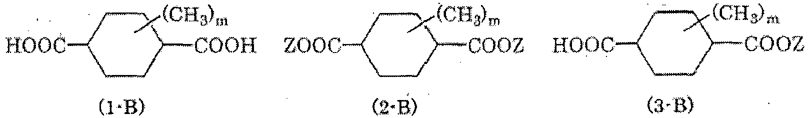 où n et Z sont identiques à ceux définis ci-dessus.

13. Procédé selon la revendication 1, dans lequel m est égal à 0.

14. Procédé selon la revendication 1, dans lequel le composé représenté par la formule (3-A) est un monoester d'acide trans-1,4-cyclohexanedicarboxylique.

15. Procédé de production d'un composé polymérisable comprenant la production du composé représenté par la formule (3-A) selon le procédé de la revendication 1, et comprenant de plus une troisième étape de réaction du composé représenté par la formule (3-A) avec un composé représenté par la formule (5) pour obtenir un composé représenté par la formule (6), et une quatrième étape de déprotection du groupe représenté par Z du composé représenté par la formule (6) qui a été obtenu dans la troisième étape pour obtenir un composé polymérisable représenté par la formule (7), 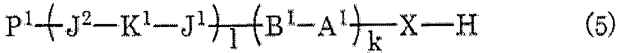 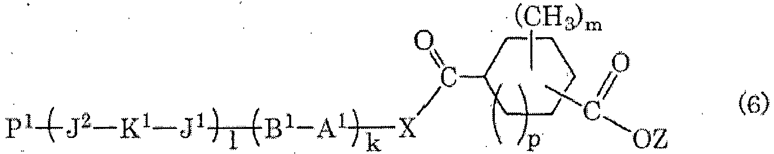 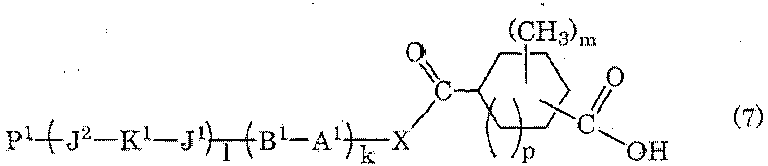 où m, Z et p sont identiques à ceux définis ci-dessus, X représente -O-, -S-, ou -N(R¹⁷)-,
A¹ est indépendamment dans chaque cas un groupe hydrocarboné alicyclique divalent ayant de 3 à 10 atomes de carbone ou un groupe hydrocarboné aromatique divalent ayant de 6 à 20 atomes de carbone, un atome d'hydrogène contenu dans le groupe hydrocarboné alicyclique ou le groupe hydrocarboné aromatique peut être remplacé par un atome d'halogène, un groupe alkyle ayant de 1 à 4 atomes de carbone et éventuellement substitué avec un atome d'halogène, un groupe alcoxy ayant de 1 à 4 atomes de carbone et éventuellement substitué avec un atome d'halogène, un groupe cyano ou un groupe nitro, -CH₂- contenu dans le groupe hydrocarboné alicyclique peut être remplacé par -O-, -S-ou -N(R¹⁷)-, -CH(-)- contenu dans le groupe hydrocarboné alicyclique peut être remplacé par -N(-)-,
B¹ est indépendamment dans chaque cas -CR¹⁵R¹⁶-, -CH₂-CH₂-, -O-, -S-, -C(=O)-O-, -O-C(=O)-, -O-C(=O)-O-, -C(=S)-O-, -O-C(=S), -O-C(=S)-O-, -C(=O)-N(R¹⁷)-, -N(R¹⁷)-C(=O)-, -OCH₂-, -CH₂O-, -SCH₂-, -CH₂S-, -C(=S)-O-, -O-C(=S)-, -C(=O)-S-, -S-C(=O)-, -(C=S)-S-, -S-C(=S)-, -NH-C(=O)-O-, -O-C(=O)-NH-, -NH-C(=O)-NH-, -N(R¹⁷)-,-S(=O)-, -O-S(=O)₂-O-, -N=N- ou une liaison simple,
R¹⁵ et R¹⁶ représentent chacun indépendamment un atome d'hydrogène, un atome de fluor ou un groupe alkyle ayant de 1 à 4 atomes de carbone, R¹⁷ représente un atome d'hydrogène ou un groupe alkyle ayant de 1 à 4 atomes de carbone,
J¹ et J² représentent chacun indépendamment un groupe alcanediyle ayant de 1 à 18 atomes de carbone, et un atome d'hydrogène contenu dans le groupe alcanediyle peut être remplacé par un groupe alcoxy ayant de 1 à 5 atomes de carbone ou un atome d'halogène,
K¹ est indépendamment dans chaque cas -0-, -C(=O)-O-, -O-C(=O)-, -C(=S)-O-, -O-C(=S)-, -C(=O)-S-, -S-C(=O)-, -(C=S)-S-, -S-C(=S)-, -NH-C(=O)-, -C(=O)-NH-, -NH-C(=O)-O-, -O-C(=O)-NH-, -NH-C(=O)-NH-, -O-C(=O)-O-, -N(R¹⁷)-, -S(=O)-, -O-S(=O)₂-O-, ou une liaison simple,
k et l représentent chacun indépendamment un nombre entier de 1 à 3, et
P¹ représente un groupe polymérisable.

16. Procédé selon la revendication 15, dans lequel P¹ est un groupe acryloyloxy ou un groupe méthacryloyloxy.

17. Procédé selon la revendication 15, dans lequel la quatrième étape est une étape de déprotection du groupe représenté par Z du composé représenté par la formule (6) qui a été obtenu dans la troisième étape avec un acide.

18. Procédé selon la revendication 17, dans lequel l'acide est au moins un choisi dans le groupe constitué d'acide formique, d'acide acétique, d'acide propionique, d'acide chlorhydrique, d'acide bromhydrique, d'iodure d'hydrogène, d'acide nitrique, d'acide sulfurique, d'acide p-toluènesulfonique, de p-toluènesulfonate de pyridinium, d'acide trifluoroacétique, d'acide trichloroacétique et d'acide méthanesulfonique.
